(19) Europäisches Patentamt
European Patent Office
Office européen des brevets

(11) EP 3 470 415 A1

(12) EUROPEAN PATENT APPLICATION
published in accordance with Art. 153(4) EPC

(43) Date of publication:
**17.04.2019 Bulletin 2019/16**

(21) Application number: **17802189.5**

(22) Date of filing: **24.05.2017**

(51) Int Cl.:
***C07D 487/04*** (2006.01) ***A61K 31/5025*** (2006.01)
***A61K 31/5377*** (2006.01) ***A61K 31/541*** (2006.01)

(86) International application number:
**PCT/CN2017/085765**

(87) International publication number:
**WO 2017/202343 (30.11.2017 Gazette 2017/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **24.05.2016 CN 201610353035**
**24.01.2017 CN 201710060243**

(71) Applicant: **Shanghai Institute of Materia Medica, Chinese Academy of Sciences**
**Shanghai 201203 (CN)**

(72) Inventors:
- **JIANG, Hualiang**
  **Pudong**
  **Shanghai 201203 (CN)**
- **LIU, Hong**
  **Pudong**
  **Shanghai 201203 (CN)**
- **GENG, Meiyu**
  **Pudong**
  **Shanghai 201203 (CN)**
- **ZHENG, Mingyue**
  **Pudong**
  **Shanghai 201203 (CN)**
- **AI, Jing**
  **Pudong**
  **Shanghai 201203 (CN)**
- **WANG, Yulan**
  **Pudong**
  **Shanghai 201203 (CN)**
- **WU, Xiaowei**
  **Pudong**
  **Shanghai 201203 (CN)**
- **LI, Shuangjie**
  **Pudong**
  **Shanghai 201203 (CN)**
- **PENG, Xia**
  **Pudong**
  **Shanghai 201203 (CN)**
- **LI, Chunpu**
  **Pudong**
  **Shanghai 201203 (CN)**
- **CHEN, Kaixian**
  **Pudong**
  **Shanghai 201203 (CN)**
- **WANG, Bao**
  **Shanghai 201203 (CN)**

(74) Representative: **Mewburn Ellis LLP**
**City Tower**
**40 Basinghall Street**
**London EC2V 5DE (GB)**

# (54) 5-MEMBERED HETEROCYCLE FUSED WITH [3,4-D]PYRIDAZINONE, AND MANUFACTURING METHOD, PHARMACEUTICAL COMPOSITION, AND APPLICATION THEREOF

(57) The present invention provides a compound comprising a 5-membered heterocycle fused with a pyridazinone, wherein the compound is used as an FGFR kinase inhibitor, and a manufacturing method and application thereof. The invention specifically provides a compound as represented by formula (I). Various radicals are as defined in the specification. The compound provided by the invention effectively inhibits an activity of an FGFR kinase, and can be used to manufacture a pharmaceutical product for treating a disease related to the activity of the FGFR kinase.

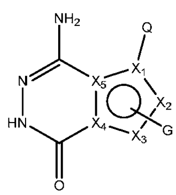

(I)

EP 3 470 415 A1

**Description**

## FIELD OF THE INVENTION

The present invention provides a class of five-membered heterocyclic [3,4-d]pyridazinone compounds, a preparation method thereof, a pharmaceutical combination and application thereof. The compound has FGFR kinase inhibitory activity and can be used in the treatment of various diseases related with the FGFR enzyme activity, the abnormal expression or activity of FGFR ligands, such as cancer, tumor, etc.

## BACKGROUND OF THE INVENTION

According to the statistics of World Health Organization (WHO), there are approximately 8.2 million people worldwide who die from cancer each year, which accounts for 13% of the total deaths. Due to the continue growing data, WHO expects that the global death from cancer would increase by 70% over the next 20 years (from WHO, fact sheet by Cancer, Globocan 2012).

The occurrence, evolution, spread of tumors and angiogenesis of tumor depend on various signal transduction pathways. Targeted therapy is a drug therapy that interferes with these signaling pathways to prevent cancer cell growth. Compared with traditional chemotherapy methods, targeted therapy can more specifically act on carcinogenic sites, inhibit tumor cell growth, and reduce damage to normal tissue cells. So far, the FDA has approved the marketing of 26 small molecule targeted drugs, for example, imatinib developed based on ABL tyrosine kinase, Afatinib based on EGFR, HER2/4 tyrosine kinase, and Trametinib based on MEK tyrosine kinase, etc. Targeted therapy has a significant impact on cancer treatment (Nature Reviews Clinical Oncology (2015) doi:10.1038/nrclinonc.2015.213).

The fibroblast growth factor receptors (FGFR) family is a receptor tyrosine protein kinases (RTKs) which include four receptor subtypes (FGFR1, FGFR2, FGFR3, and FGFR4). In the FGF signal transduction pathway, fibroblast growth factor binds to the FGFR receptor, induces FGFR dimerization, phosphorylates tyrosine at the end of cytoplasmic structure of FGFR, and activates downstream FRS2-Ras-MAPK, PLCγ, and PI3K- AKT/PKB signaling pathway. Under physiological conditions, the FGFR signaling pathway is involved in the regulation of processes such as embryonic development, cell proliferation and migration, and neovascularization (Cytokine & Growth Factor Reviews 16 (2005) 233-247). Studies have found that activation or high expression of FGFR mutations is closely related to the development of human tumors. Activation or overexpression of FGFR in cells leads to the persistence and overactivation of the FGFR signaling pathway, allowing cells to acquire carcinogenic functions such as over-proliferation and apoptosis escape. At the same time, the study found abnormal expression of FGFR in various cancer cells, such as breast cancer, lung cancer, ovarian cancer, gastric cancer, uterine tumor, glioblastoma, bladder cancer, liver cancer, solid tumor, etc. (Cancer Discovery. 2013; 3 (3): 264-279; Annals of Oncology. 2014; 25:552-563). Therefore, FGFR is recognized as an important target for the development of anti-tumor drugs and is one of the most popular targets for drug development.

Studies have shown that types of cancer involving FGF/FGFR include (but are not limited to): carcinoma (e.g., bladder cancer, breast cancer, cervical cancer, colorectal cancer, endometrial cancer, stomach cancer, head and neck cancer, kidney cancer, liver cancer, lung cancer, ovarian cancer, prostate cancer); hematopoietic malignancies (e.g., multiple myeloma, chronic lymphocytic lymphoma, adult T-cell leukemia, acute myeloid leukemia, non-Hodgkin's lymphoma, myeloproliferative neoplasms and Walden Trent's macroglobulinemia); and other neoplasms (eg, glioblastoma, melanoma, and rhabdomyosarcoma). In addition to its role in oncogenic neoplasms, FGFR activation is also implicated in bone and chondrocyte disorders, including but not limited to, achondroplasia and craniosynostosis syndrome.

There is a continuing need to develop new drugs for the treatment of cancer and other diseases, while the FGFR inhibitors of the present invention aims to help address this need.

## SUMMARY OF THE INVENTION

The present invention provides a novel and effective five-membered heterocyclic [3,4-d]pyridazinone FGFR small molecule inhibitor.

In the first aspect of the present invention, a compound of formula I, or a pharmaceutically acceptable salt thereof is provided,

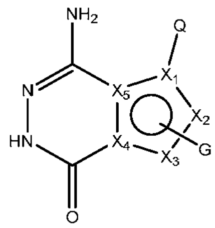

(I)

wherein,

$X_1$ is selected from CH, C, N; preferably CH or C;
$X_2$ and $X_3$ are each independently selected from CH, C, NH, N, O or S; preferably NH, N, CH or C;
$X_4$ and $X_5$ are C;
and $X_1$, $X_2$, $X_3$, $X_4$ and $X_5$ together form an aromatic five-membered ring;
Q is selected from: a substituted or unsubstituted naphthyl, substituted or unsubstituted 8-10 membered bicyclic heteroaryl;
or Q is selected from -L-A, wherein L is a substituted or unsubstituted C1-C4 alkylidene, substituted or unsubstituted C2-C4 alkenylene, C2-C4 alkynylene, C1-C4 alkylideneoxy, -(C1-C4 alkyl)-NH-, -CO-NH-, -NH-CO-; A is selected from a substituted or unsubstituted C6-C12 aryl, substituted or unsubstituted 5-8 membered cycloheteroaryl, substituted or unsubstituted 8-10 membered bicyclic heteroaryl;
G is 1-2 substituents, each of which is independently on $X_2$ and/or $X_3$ and selected from the group consisting of halogen, hydroxy, cyano, -L3-substituted or unsubstituted C6-C12 aryl, -L3-substituted or unsubstituted 5-12 membered heteroaryl, -L3-substituted or unsubstituted 3-12 membered heterocyclic group, -L3-substituted or unsubstituted C3-C8 cycloalkyl, $-(CH_2)_m$-L1 -R1, $-(CH_2)_m-N(R_2)(R_3)$, $-(CH_2)_m-C(=O)-N(R_2)(R_4)$;
wherein, the group G may optionally have 1-3 independent $-Lx_1-Lx_2-Lx_3-Lx_4-Lx_5$-M substituents; wherein $Lx_1$, $Lx_2$, $Lx_3$, $Lx_4$ and $Lx_5$ are each independently selected from the group consisting of: none, carbonyl (C=O), oxy (-O-), -C=S-, $-S(O)_2-$, $-CH_2-$, -CH=CH-, C3-C8 cycloalkylidene, -C≡C-, -NH-, $-N(R_5)-$; M is selected from the group consisting of H, -OH, halogen, cyano, $-N(R_2)(R_3)$, $-CH_3$, $-C(=O)CH_3$, C1-C6 alkoxy, 3-12 membered heterocyclyl, C3-C8 cycloalkyl, 5-12 membered heteroaryl, C3-C8 cycloalkenyl;
The $-Lx_1-Lx_2-Lx_3-Lx_4-Lx_5$-M substituent may be further substituted by one or more halogens, C1-C6 alkyl groups, C1-C6 alkoxy groups, cyano groups, hydroxyl groups, =O, $=CH_2$ (i.e., the methylene group replaces two hydrogen atoms on a group through a double bond), vinyl ($-CH=CH_2$), $-(CH_2)_kN(R_2)(R_3)$, -(C1-C6 alkyl)-$OR_2$, 3-12 membered heterocyclic groups or C3-C8 cycloalkyl groups;
k is selected from 0, 1, 2 or 3;
m is selected from 0, 1, 2, 3 or 4;
L1 is none, O, or -C(=O)O-;
L3 is none, or a substituted or unsubstituted C1-C4 alkylidene;
$R_1$, $R_3$ and $R_4$ are each independently selected from hydrogen, halogen, a substituted or unsubstituted C1-C6 alkyl, substituted or unsubstituted C3-C8 cycloalkyl, substituted or unsubstituted 3-12 membered heterocyclic group , -(C1-C6 alkyl)-N(C1-C6 alkyl)(C1-C6 alkyl), -(C1-C6 alkyl)-O-(C1-C6 alkyl), or substituted or unsubstituted phenyl;
$R_2$ is selected from hydrogen or a C1-C4 alkyl;
or $R_2$ and $R_3$ , $R_2$ and $R_4$ together with the attached nitrogen atom constitute a substituted or unsubstituted 4-7 membered heterocyclic ring containing 1 to 3 heteroatoms selected from N, O, S, and at least one hetero atom is N;
$R_5$ is selected from the group consisting of hydrogen, a C1-C4 alkyl, C3-C6 cycloalkyl, formyl, C1-C4 alkylcarbonyl, or C1-C4 alkoxycarbonyl;
the "substituted" means that one or more hydrogen atoms (preferably 1-5) on the group are substituted by groups selected from the group consisting of hydroxy, halogen, cyano, nitro, amino, carboxy, $-CH_2OH$, - $CONH_2$, a substituted or unsubstituted C1-C6 alkoxy, substituted or unsubstituted C1-C6 alkyl, substituted or unsubstituted C1-C6 alkylamino, C1-C4 alkylacyl, C1-C4 alkanesulfonyl, C1-C4 alkoxycarbonyl, C1-C4 alkanesulfonylamino, oxo (=O), $:CH_2$, C3-C6 cycloalkyl, 4-7 membered heterocyclyl, -NH(C1-C4 alkyl), -N(C1-C4 alkyl)(C1-C4 alkyl), pyrrolidinyl, piperidinyl, C3-C6 cycloalkylcarbonyl, phenyl, C2-C4 alkynyl, substituted or unsubstituted 5-10 heteroaryl; the substituents of the C1-C6 alkoxy, C1-C6 alkyl and the C1-C6 alkylamino are each independently 1 to 3 groups selected from the group consisting of: oxo, halogen, cyano, cyclopropyl, hydroxyl, amino, -N(C1-C4 alkyl)(C1-C4 alkyl); the substituent of the 5-10 membered heteroaryl is 1-3 groups selected from the group consisting of a C1-C6 alkyl, -C1-C4 alkylene-N(C1-C4 alkyl) (C1-C4 alkyl);
the C1-C6 alkoxy, C1-C6 alkyl and C1-C6 alkylamino include a straight-chain or branched-chain group;

while the compound is not any of the following structures:

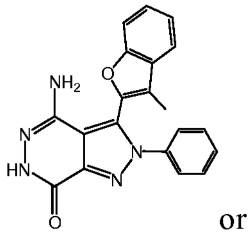 or 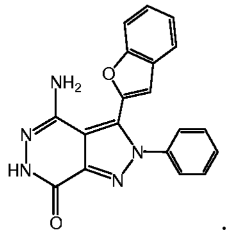 .

In another preferred embodiment, the C1-C6 alkyl is selectively substituted by cyano, hydroxy, -(C1-C4 alkyl)-O-(C1-C4 alkyl)-O-(C1-C4 alkyl), -O-CO-(C1-C4 alkyl), carbonyl (=O), C1-C4 alkoxycarbonyl, amino, $-CONH_2-$, -NH-CO-(C1-C4 alkyl), pyrrolidone, piperidine, morpholine, -NH(C1-C4 alkyl), -N(C1-C4 alkyl)(C1-C4 alkyl), C3-C6 cycloalkyl, or 1-3 halogens.

In another preferred embodiment, the C3-C6 cycloalkyl group is selectively substituted by 1 to 2 substituents each independently selected from a C1-C4 alkyl, hydroxyl, halogen, or amino.

In another preferred embodiment, the 4-7 membered heterocyclic group is substituted by 1 to 2 substituents independently selected from the group consisting of a C1-C4 alkyl, C1-C4 alkoxy, hydroxyl, amino, oxo, and $-CONH_2$; the phenyl is selectively substituted by 1 to 2 groups independently selected from the group consisting of fluorine, chlorine, bromine, cyano, trifluoromethyl, trifluoromethoxy, a C1-C4 alkyl, and C1-C4 alkoxy.

In another preferred embodiment, the substitutent refers to a group selected from the group consisting of hydroxyl, halogen, cyano, nitro, amino, carboxyl, $-CH_2OH$, $-CONH_2$, a substituted or unsubstituted C1-C6 alkyl, halogen substituted or unsubstituted C1-C4 alkoxy or C1-C4 alkylamino, C1-C4 alkanoyl, C1-C4sulfonyl, C1-C4 sulfonylamino, C2-C6 alkynyl, substituted or unsubstituted 5-10-membered heteroaryl; the C1-C6 alkyl substituent is independently substituted by one to three substituents selected from the group consisting of oxo, halogen, cyano, cyclopropyl, hydroxy, amino, -N(C1-C6 alkyl) (C1-C6 alkyl); the substituent on the 5-10 membered heteroaryl is selected from the group consisting of a C1-C6 alkyl, -(C1-C6 alkyl)-N(C1-C6 alkyl)(C1-C6 alkyl);

In another preferred embodiment, G is not an unsubstituted phenyl.

In another preferred embodiment, when $X_2$ is N, then G is not an unsubstituted phenyl.

In another preferred embodiment, when G is on $X_2$ and Q is

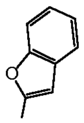 or 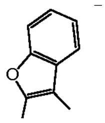 ,

then G is not an unsubstituted phenyl.

In another preferred embodiment, at least one of $Lx_1$, $Lx_2$, $Lx_3$, $Lx_4$, and $Lx_5$ is -NH-.

In another preferred embodiment, at least one of $Lx_1$, $Lx_2$, $Lx_3$, $Lx_4$, and $Lx_5$ is -CH=CH-.

In another preferred embodiment, at least one of $Lx_1$, $Lx_2$, $Lx_3$, $Lx_4$, and $Lx_5$ is -C≡C-.

In another preferred embodiment, Q is a substituted or unsubstituted group selected from the group consisting of: naphthyl, benzo 5-6 membered monocyclic heteroaryl, 5-6 membered monocyclic heteroaryl, and 5-6 membered monocyclic heteroaryl;

or Q is selected from -L-A, wherein L is an unsubstituted or halogenated C1-C4 alkylidene, unsubstituted or halogenated C2-C4 alkenylene, C2-C4 alkynylene, C1-C4 alkylideneoxy, -(C1-C4 alkyl)-NH-, -CO-NH-, -NH-CO-; A is selected from a substituted or unsubstituted C6-C12 aryl, substituted or unsubstituted 5-6 membered monocyclic heteroaryl;

In another preferred embodiment, the Q is selected from the group consisting of a substituted or unsubstituted group: naphthalene, indole, indazole, isoindole, benzimidazole, benzofuran, benzothiophene, benzothiazole, quinoline, isoquinoline, benzopyrimidine and benzopyran;

In another preferred embodiment, the L is selected from the group consisting of an unsubstituted or halogenated C1-C3 straight or branched alkylidene, unsubstituted or halogenated C2-C4 straight or branched alkenyl, unsubstituted or halogenated C2-C4 straight or branched alkynyl, -CO-NH-, -NH-CO-.

In another preferred embodiment, the A is a substituted or unsubstituted group selected from the group consisting of: pyrrolyl, furyl, pyrazolyl, oxazolyl, isoxazolyl, imidazolyl, phenyl, pyridyl, pyrimidinyl, pyridazinyl, pyrazinyl;

In another preferred embodiment, the G is 1-2 substituents independently selected from the group consisting of halogen, hydroxy, cyano, -L3-substituted or unsubstituted C6-C10 aryl, -L3-substituted or unsubstituted 5-10 membered heteroaryl, -L3-substituted or unsubstituted 4-7 membered heterocyclic group, -L3-substituted or unsubstituted C3-C6 cycloalkyl, $-(CH_2)_m$-L1 -R1, $-(CH_2)_m-N(R_2)(R_3)$, $-(CH_2)_m-C(=O)-N(R_2)(R_4)$;
wherein L3 is none, a substituted or unsubstituted methylidene, substituted or unsubstituted ethylidene;
and the group G may optionally have 1-3 independent $-Lx_1-Lx_2-Lx_3-Lx_4-Lx_5-M$ substituents; wherein $Lx_1$, $Lx_2$, $Lx_3$, $Lx_4$ and $Lx_5$ are each independently selected from the group consisting of: none, carbonyl (C=O), oxy (-O-), -C=S-, $-S(O)_2-$, $-CH_2-$, -CH=CH-, C3-C8 cycloalkylidene, ethynylene (-C≡C-), -NH-, $-N(R_5)-$;
M is selected from the group consisting of H, -OH, halogen, cyano, $-N(R_2)(R_3)$, $-CH_3$, $-C(=O)CH_3$, C1-C4 alkoxy, 4-7 membered heterocyclyl, C3-C6 cycloalkyl, 5-10 membered heteroaryl, and C3-C6 cycloalkenyl;
the $-Lx_1-Lx_2-Lx_3-Lx_4-Lx_5-M$ substituent may be further substituted by one or more halogens, C1-C6 alkyl groups, cyano groups, hydroxy, oxygen atoms (=O), $=CH_2$, $-(CH_2)_kN(R_2)(R_3)$, -(C1-C6 alkyl)-$OR_2$, 3-12 membered heterocyclic groups or C3-C6 cycloalkyl;
$R_1$, $R_3$ and $R_4$ are each independently selected from hydrogen, halogen, a substituted or unsubstituted C1-C4 alkyl, substituted or unsubstituted C3-C6 cycloalkyl, substituted or unsubstituted 4-7 membered heterocyclic group, or substituted or unsubstituted phenyl;
or $R_2$ and $R_3$ , $R_2$ and $R_4$ together with the attached nitrogen atom constitute a substituted or unsubstituted 4-7 saturated membered heterocyclic ring containing 1 to 3 hetero atoms selected from N, O, S, and at least one hetero atom is N;
$R_5$ is selected from the group consisting of hydrogen, C1-C4 alkyl, C3-C6 cycloalkyl, formyl, C1-C4 alkylcarbonyl, C1-C4 alkoxycarbonyl.

In another preferred embodiment, $X_1$ is C, and both of $X_2$ and $X_3$ are N; or $X_1$ is C, $X_2$ is CH and $X_3$ is N; or $X_1$ is C, $X_2$ is C and $X_3$ is N.

In another preferred embodiment, each of $X_1$, $X_2$, $X_3$, $X_4$, $X_5$, Q, G, $-Lx_1-Lx_2-Lx_3-Lx_4-Lx_5-M$, A, m, L1, L3, $R_1$, $R_2$, $R_3$, $R_4$ is independently the corresponding group of the compounds in the examples.

In another preferred embodiment, the compound is selected from the compounds A1-A92 in the table.

In the second aspect of the present invention, a preparation method for compound (I) of the first aspect of the present invention is provided, which comprises the following steps:

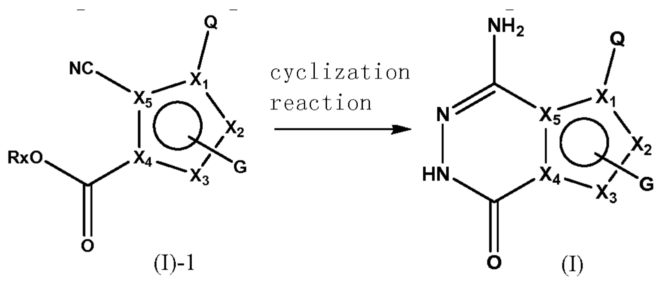


(I)-1 (I)

wherein $X_1$, $X_2$, $X_3$, $X_4$, $X_5$, Q and G have the same definitions as the corresponding description above; Rx is selected from C1-C6 alkyl;

The compound of formula (I)-1 is cyclized with hydrazine hydrate to give a compound of formula (I).

In another preferred embodiment, Rx is methyl or ethyl.

In the third aspect of the present invention, the use of compound of the first aspect of the present invention or the pharmaceutically acceptable salt thereof is provided for:

(a) preparation of a medicine for treating diseases associated with FGFR kinase activity or expression amount;
(b) preparation of FGFR kinase targeting inhibitor;
(c) *in vitro* non-therapeutic inhibition of FGFR kinase activity;
(d) *in vitro* non-therapeutic inhibition of tumor cell proliferation; and / or
(e) treatment of disease associated with FGFR kinase activity or expression amount.

In another preferred embodiment, the disease associated with FGFR activity or expression amount is selected from the group consisting of carcinoma, hematopoietic malignant disease, other neoplasms, bone and chondrocyte disorders, hypophosphatemia, fibrotic diseases, psoriasis, keloid, bullous skin disease, atherosclerosis, restenosis, glomerular membrane cell proliferative disorder, glomerular lesion, diabetic nephropathy, nephropathy and benign prostatic hyperplasia, eye disease, and craniosynostosis; preferably is a carcinoma selected from the group consisting of: bladder cancer, breast cancer, cervical cancer, colorectal cancer, endometrial cancer, stomach cancer, head and neck cancer, kidney cancer, liver cancer, lung cancer, ovarian cancer, prostate cancer, esophageal cancer, gallbladder cancer, pancreatic cancer, thyroid cancer, skin cancer, leukemia, multiple myeloma, chronic lymphocytic lymphoma, adult T-cell leukemia, B-cell lymphoma, acute myeloid leukemia, Hodgkin's lymphoma or non-Hodgkin's lymphoma, Waldenstrom's macroglobulinemia, hairy cell lymphoma, Bojit's lymphoma, glioblastoma, melanoma, and rhabdomyosarcoma.

In another preferred embodiment, the FGFR kinase is selected from the following group: FGFR1, FGFR2, FGFR3, or combinations thereof; and / or

In another preferred embodiment, the tumor cells are gastric cancer cell strain, lung cancer cell strain, leukemia cell strain, bladder cancer cell strain, and liver cancer cell strain.

In another preferred embodiment, the disease associated with FGFR kinase activity or expression is selected from the group consisting of bladder cancer, breast cancer, cervical cancer, colorectal cancer, endometrial cancer, stomach cancer, head and neck cancer, kidney cancer, liver cancer, lung cancer, ovarian cancer, prostate cancer, multiple myeloma, chronic lymphocytic lymphoma, adult T-cell leukemia, acute myeloid leukemia, non-Hodgkin's lymphoma, myeloproliferative neoplasms and Walden Trent's macroglobulinemia.

In another preferred embodiment, the compound of the present invention can be used to prevent or inhibit metastasis of general tumors.

In the fourth aspect of the present invention, a pharmaceutical composition is provided, comprising: (i) effective amount of formula I compound, or a pharmaceutically acceptable salt thereof, and (ii) a pharmaceutically acceptable carrier.

In another preferred embodiment, the effective amount means a therapeutically or inhibitory effective amount, preferably from 0.01 to 99.9%.

In another preferred embodiment, the pharmaceutical composition is used to inhibit the FGFR kinase activity.

In another preferred embodiment, the pharmaceutical composition is used to treat diseases associated with FGFR kinase activity or expression amount.

In the fifth aspect of the present invention, a method for inhibiting FGFR kinase activity is provided, comprising steps: administering an inhibitory effective amount of formula I compound of the first aspect of the invention or a pharmaceutically acceptable salt thereof to a subject in need thereof, or administering an inhibitory effective amount of pharmaceutical composition of the fourth aspect of the invention to a subject in need thereof.

In another preferred embodiment, the inhibition is *in vitro* or *in vivo* inhibition.

It should be understood that, in the present invention, each of the technical features specifically described above and below (such as those in the Examples) can be combined with each other, thereby constituting new or preferred technical solutions which need not be specified again herein..

## DESCRIPTION OF THE DRAWINGS

Figure 1. inhibitory effects of compound A26 on the growth of a transplanted tumor in human lung cancer NCI-H1581 nude mice;

Figure 2. The influence of A26 on the body weight of human lung cancer NCI-H tumor-bearing mice;

Figure 3. Growth diagram of a transplanted tumor of human lung cancer NCI-H1581 nude mice by Somcl-15-290.

## EMBODIMENTS FOR CARRYING OUT THE INVENTION

Based on a long-term and intensive research, the inventors have prepared a compound having the structure of formula I, and have found it having FGFR kinase inhibitory activity. And the compound has excellent inhibitory activities against a series of FGFR kinases at very low concentrations (as low as ≤ 100 nmol / L), thus can be used for the treatment of diseases associated with FGFR kinase activity or expression such as tumors. The present invention is completed on this basis.

### Terms

As used herein, the term "C1-C6 alkyl" refers to a linear or branched alkyl with 1 to 6 carbon atoms, such as methyl, ethyl, propyl, isopropyl, butyl, isobutyl, sec-butyl, tert-butyl, n-amyl, iso-amyl, n-hexyl and iso-hexyl, or the like. The definition "C1-C6 alkyl" includes "C1-C4 alkyl".

The term "C1-C6 alkylene" refers to a group formed by C1-C6 alkyl losing one hydrogen atom, such as $-CH_2-$, $-CH_2-CH_2-$, $-CH_2-CH_2-CH_2-$ and $-CH_2-CH(CH_3)-CH_2-$, or the like. The definition "C1-C6 alkylene" includes "C1-C4 alkylene". The term "C2-C6 alkenyl" refers to a straight or branched chain group having 1-3 double bonds and 2 to 6 carbon atoms, such as ethenyl, propenyl, isopropenyl, 1-butenyl, 2-butenyl, 2-methyl-1-propenyl, 1,3-butadienyl and 1,3,5-hexanetrienyl, or the like. The definition of "C2-C6 alkylene" includes "C2-C4 alkenyl".

The term "C2-C6 alkenylene" refers to a group formed by C2-C6 alkenyl losing one hydrogen atom, such as $-CH=CH-$ and $-CH_2CH=CH-$, or the like. The definition of "C2-C6 alkenylene" includes "C2-C4 alkenylene".

The term "C2-C6 alkynyl" refers to a straight or branched chain group having 1-3 triple bonds and 2-6 carbon atoms, such as ethynyl, propynyl, 1-butynyl, 2-butyne, 1-pentynyl, 2-pentynyl, 3-methyl-1-butynyl, 1-hexyne, 1,3-hexadiynyl, and 3-hexynyl, or the like. The definition "C2-C6 alkylene" includes "C2-C4 alkynyl".

The term "C2-C6 alkynylene" refers to a group formed by C2-C6 alkynyl losing one hydrogen atom, such as -C≡C- and -C≡C-, or the like. The definition of "C2-C6 alkynylene" includes "C2-C4 alkynylene".

The term "C6-C12 aryl" refers to a monocyclic or fused bicyclic ring having 6 to 12 carbon atoms which having a conjugated $\pi$-electron system, such as phenyl and naphthyl, or the like. The definition of "C6-C12 aryl" includes "C6-C10 aryl".

The term "C6-C12 arylidene" refers to a group formed by C6-C12 aryl losing one hydrogen atom, including monocyclic or bicyclic arylidene, such as phenylidene, naphthylidene, or the like. The definition of "C6-C12 arylidene" includes "C6-C10 arylidene".

The term "5-12 membered heteroaryl" refers to an unsaturated cyclic substituient with a 5-12 membered monocyclic or fused polycyclic ring and one or more heteroatoms selected from O, S, N or P on the ring system, preferably a 5-10 membered monocyclic or fused bicyclic ring having 1 to 5 hetero atoms selected from O, S, N or P on the ring system, further preferably a 5-8 membered monocyclic heteroaryl or 8-10 membered bicyclic heteroaryl, most preferably selected from the group consisting of 5-6 membered monocyclic heteroaryl, benzo 5-6 membered monocyclic heteroaryl, 5-6 membered monocyclic heteroaryl fused 5-6 membered monocyclic heteroaryl, such as pyridyl, thienyl, furyl, pyrrolyl, thiazolyl, imidazolyl, benzofuranyl, indolyl, indazolyl, isoindazolyl, benzimidazolyl, benzothiophene, benzothiazolyl, quinolyl, isoquinolyl, benzopyrimidinyl and benzopyranyl group, or the like.

Term "C3-C8 cycloalkyl" refers to a saturated cycloalkyl with 3-8 carbon atoms, such as cyclopropyl, cyclobutyl, cyclopentyl, cycloheptyl, or the like. The definition "C3-C8 cycloalkyl" includes "C3-C6 cycloalkyl".

Term "C3-C8 cycloalkylidene" refers to a group formed by a C3-C8 cycloalkyl by losing one hydrogen, such as cyclopropylidene, cyclobutylidene, cyclopentylidene, cycloheptylidene, or the like. The definition "C3-C8 cycloalkylidene" includes "C3-C6 cycloalkylidene".

Term "C3-C8 cycloalkenyl" refers to a carbocyclic group having 3-8 carbon atoms and 1-3 double bonds which does not have a fully conjugated $\pi$-electron system, such as cyclopropylene, cyclobutene, cyclopentylene, cycloheptylene, or the like. The definition "C3-C8 cycloalkenyl" includes "C3-C6 cycloalkenyl".

The term "3-12 membered heterocyclic group" refers to a saturated cyclic substituient with 3 to 12 membered monocyclic or fused bicyclic ring having one or more (preferably 1 to 5) heteroatoms selected from O, S, N or P on the ring system, such as piperidinyl, pyrrolidinyl, piperazinyl, tetrahydrofuranyl, morpholinyl, or the like. The definition "3-12 membered heterocyclic group" includes "4-7 membered heterocyclic group".

The term "halogen" means fluoro, chloro, bromo or iodo; preferably fluoro, chloro or bromo.

The term "C1-C6 alkoxy" refers to a straight or branched chain alkyl group having 1 to 6 carbon atoms, such as methoxy, ethoxy, propoxy, isopropoxy, butoxy, iso-butoxy, sec-butoxy, and tert-butoxy, or the like. The definition "C1-C6 alkoxy" includes "C1-C4 alkoxy".

The term "C1-C6 alkylideneoxy" refers to groups formed by a C1-C6 alkyoxy losing one hydrogen atom, such as $-OCH_2-$, $-OCH_2-CH_2-$, $-OCH(CH_3)-$, $-OCH_2-CH_2-CH_2-$, $-OCH_2-CH(CH_3)-$, and $-OCH_2-CH(CH_3)-CH_2-$, or the like. The definition "C1-C6 alkylideneoxy" includes "C1-C4 alkylideneoxy".

In the present invention, the term "comprising", "comprise(s)" or "including" means that the various components can be used together in the mixture or composition of the present invention. Therefore, terms "mainly comprise..." and "comprise..." are within the term "comprise".

In the present invention, the term "pharmaceutically acceptable" component refers to substances which are suitable for applying to humans and / or animals without undue harmful side reactions (such as toxicity, stimulation or allergy), that is, substances of reasonable benefit / risk ratio.

In the present invention, the term "effective amount" refers to an amount in which the therapeutic agents can treat, relieve or prevent the targeted disease, or exhibit detectable treatment or prevention effects. The exact effective amount for a subject will depend on the size and health condition of the subject, the nature and extent of the disorder, and the therapeutic agent and / or therapeutic agent combination selected for administration. Therefore, it is useless to specify an accurate effective amount in advance. However, for a given situation, the effective amount may be determined by routine experimentation, which can be determined by clinicians.

In the present invention, unless otherwise indicated, the term, "substituted" means that one or more hydrogen atoms on the group are substituted with substituents selected from the following group: halogen, an unsubstituted or halogenated C1-C6 alkyl, unsubstituted or halogenated C2-C6 acyl group, unsubstituted or halogenated C1-C6 alkylhydroxy.

Unless otherwise indicated, all compounds in the invention are intended to include all possible optical isomers, such as single chiral compounds, or mixtures of various chiral compounds (i.e., racemates). In compounds of the present invention, each chiral carbon atom may optionally be in R configuration or S configuration, or the mixture of R configuration and S configuration.

As used herein, the term "compound of the invention" refers to formula I compound. The term also comprises the crystal forms, pharmaceutically acceptable salts, hydrates or solvates of compound of formula I.

As used herein, the term "pharmaceutically acceptable salt" refers to a salt suitable for use as a medicament formed by the compound of the present invention with an acid or base. The pharmaceutically acceptable salts include inorganic and organic salts. Preferred salts are salts formed by the compounds of the present invention and acid. Suitable salt-forming acids include, but are not limited to: inorganic acids such as hydrochloric acid, hydrobromic acid, hydrofluoric acid, sulfuric acid, nitric acid, phosphoric acid; organic acids such as formic acid, acetic acid, propionic acid, oxalic acid, malonic acid, succinic acid, fumaric acid, maleic acid, lactic acid, malic acid, tartaric acid, citric acid, picric acid, methanesulfonic acid, toluenesulfonic acid, benzenesulfonic acid and the like; and acidic amino acids such as aspartic acid, glutamic acid.

**Compound of Formula I**

The present invention provides a compound of formula I:

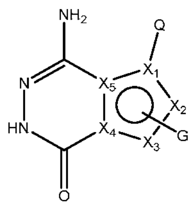

Formula (I)

Meanwhile, the chiral carbon atom in the compound of formula (I) is R type and / or S type.

The compound of the formula (I) and enantiomers, diastereomers, racemates and mixtures thereof or pharmaceutically acceptable salts thereof according to the invention are preferably selected from one of the following compounds:

| No. | Chemical Name | Structure |
|---|---|---|
| A1 | 4-amino-3-(3-methyl-1-benzothiophen-2-yl)-2-phenyl-2,6-dihydro-7H-pyrazol o[3,4-d]pyridazine-7-one | 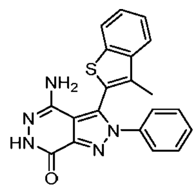 |
| A2 | 4-amino-3-(naphthalen-2-yl)-2-phenyl-2,6-dihydro-7H-pyrazolo [3,4-d]pyridazin-7-one | 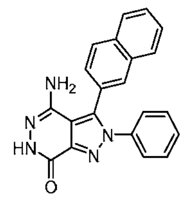 |
| A3 | 4-amino-3-(1,3-dimethyl-1H-indol-2-yl)-2-phenyl-2,6-dihydro-7H-pyrazolo[3, 4-d]pyridazin-7-one | 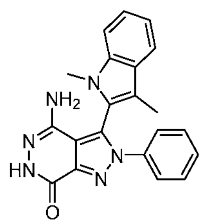 |

(continued)

| No. | Chemical Name | Structure |
|---|---|---|
| A4 | 4-amino-3-(3,5-dimethyl-1-benzofuran-2-yl)-2-phenyl-2,6-dihydro-7H-pyrazolo[3,4-d]pyridazine-7-one | 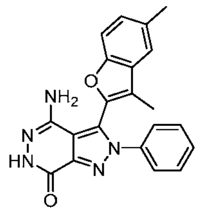 |
| A5 | 4-amino-2-phenyl-3-(quinolin-3-yl)-2,6-dihydro-7H-pyrazolo[3,4-d]pyridazin-7-one | 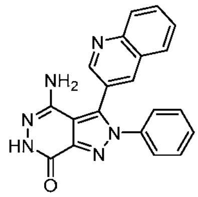 |
| A6 | 4-amino-3-(6-chloro-3-methyl-1-benzofuran-2-yl)-2-phenyl-2,6-dihydro-7H-pyrazolo[3,4-d]pyridazin-7-one | 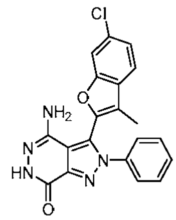 |
| A7 | 4-amino-3-(5-chloro-3-methyl-1-benzofuran-2-yl)-2-phenyl-2,6-dihydro-7H-pyrazolo[3,4-d]pyridazin-7-one | 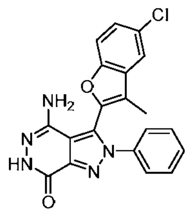 |
| A8 | 4-amino-3-(6-fluoro-3-methyl-1-benzofuran-2-yl)-2-phenyl-2,6-dihydro-7H-py razolo [3,4-d]pyridazin-7-one | 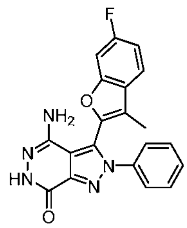 |
| A9 | 4-amino-3-(3,5-dimethyl-1-benzofuran-2-yl)-2-(4-{[ (dimethylamino)methyl]a mino}phenyl)-2,6-dihydro-7H-pyrazolo [3,4-d]pyridazin-7-one | 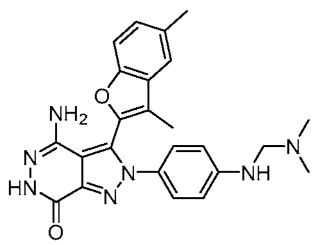 |
| A10 | 4-amino-3-(3,5-dimethyl-1-benzofuran-2-yl)-2-(1-propionylpiperidin-4-yl)-2,6-dihydro-7H-pyrazolo[3,4-d]pyridazin-7-one | 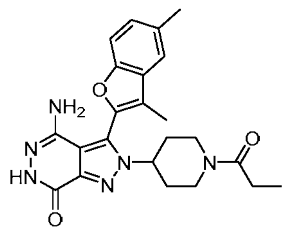 |

(continued)

| No. | Chemical Name | Structure |
|---|---|---|
| A11 | 4-amino-2-(4-{[2-(dimethylamino)ethy l](methyl)amino}phenyl)-3-(3,5-dimeth yl-1-benzofuran-2-yl)-2,6-dihydro-7H-pyrazolo [3,4-d]pyridazin-7-one | 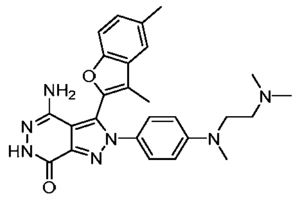 |
| A12 | 4-amino-3-(3,5-dimethyl-1-benzofuran-2-yl)-2-[4-(morpholin-4-yl) phenyl]-2,6-dihydro-7H-pyrazolo[3,4-d]pyridazin-7-one | 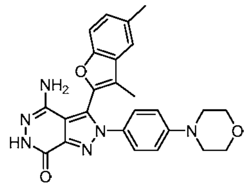 |
| A13 | 4-amino-2-(4-chlorophenyl)-3-(3,5-dim ethyl-1-benzofuran-2-yl)-2,6-dihydro-7H-pyrazolo[3,4-d]pyridazin-7-one | 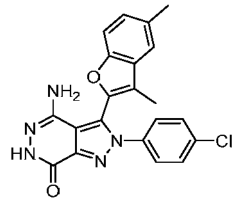 |
| A14 | 4-amino-3-(3,5-dimethyl-1-benzofuran-2-yl)-2-{4-[2-(pyrrolidin-1-yl)ethoxy]phenyl}-2,6-dihydro-7H-pyrazolo[3,4-d]pyridazin-7-one | 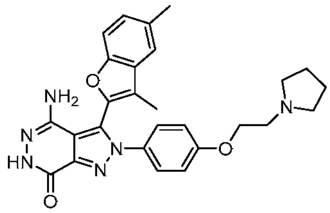 |
| A15 | 4-amino-2-{4-[2-(dimethylamino)ethoxy]phenyl}-3-(3,5-dimethyl-1-benzofuran-2-yl)-2,6-dihydro-7H-pyrazolo[3,4-d] pyridazin-7-one | 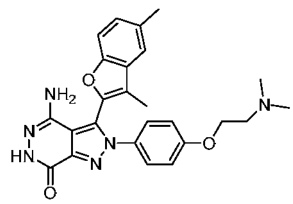 |
| A16 | 4-amino-2-[4-(4-cyclopropylpiperazin-1-yl)phenyl]-3-(3,5-dimethyl-1-benzofuran-2-yl)-2,6-dihydro-7H-pyrazolo[3, 4-d] pyridazin-7-one | 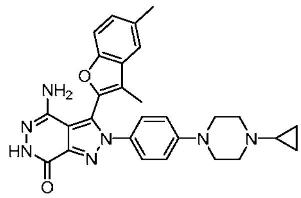 |
| A17 | 4-amino-2-{4-[3-(dimethylamino)azeti din-1-yl]phenyl}-3-(3,5-dimethyl-1-benzofuran-2-yl)-2,6-dihydro-7H-pyrazol o[3,4-d] pyridazin-7-one | 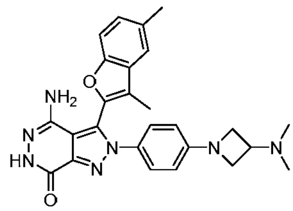 |

(continued)

| No. | Chemical Name | Structure |
|---|---|---|
| A18 | 4-amino-3-(3,5-dimethyl-1-benzofuran-2-yl)-2-[4-(4-ethylpiperazin-1-yl)phenyl]-2,6 -dihydro-7H-pyrazolo[3,4-d] pyridazin-7-one | 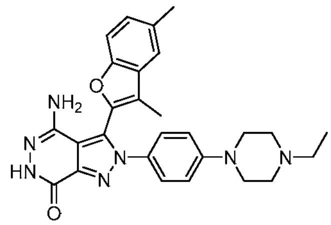 |
| A19 | 4-amino-2-(4-{[3-(dimethylamino)prop yl](methyl)amino}phenyl)-3-(3,5-dimet hyl-1-benzofuran-2-yl-2,6-dihydro-7H-pyrazolo[3,4-d]pyridazin-7-one | 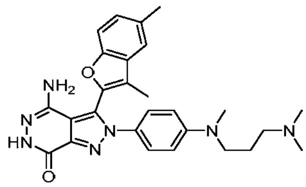 |
| A20 | 4-amino-3-(3,5-dimethyl-1-benzofuran-2-yl)-2-(4-{[(1-methylpiperidin-4-yl)methyl]amino}phenyl)-2,6-dihydro-7H-p yrazolo[3,4-d]pyridazin-7-one | 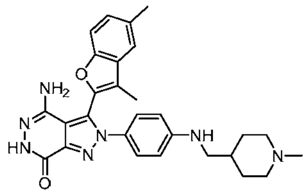 |
| A21 | 4-amino-3-(3,5-dimethyl-1-benzofuran-2-yl)-2-{4-[4-(methoxymethyl)piperidin-1-yl]phenyl}-2,6-dihydro-7H-pyrazol o[3,4-d]pyridazin-7-one | 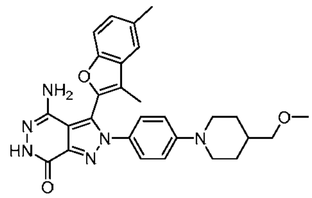 |
| A22 | 4-amino-2-{4-[3-(diethylamino)pyrrolidin-1-yl]phenyl}-3-(3,5-dimethyl-1-benzofuran-2-yl)-2,6-dihydro-7H-pyrazolo[3,4-d] pyrida zin-7-one | 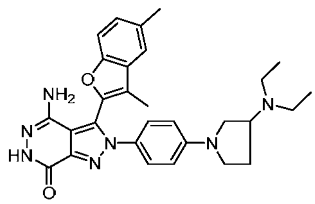 |
| A23 | 4-amino-3-(5-chloro-3-methyl-1-benzofuran-2-yl)-2-(1-ethylpiperidin-4-yl)-2, 6-dihydro-7H-pyrazolo[3,4-d]pyridazin -7-one | 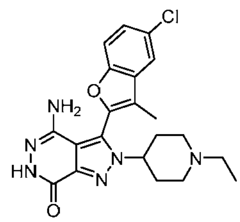 |
| A24 | 4-amino-3-(3,5-dimethyl-1-benzofuran-2-yl)-2-(1-ethylpiperidin-4-yl)-2,6-dihydro-7H-pyrazolo[3,4-d]pyridazin-7-one | 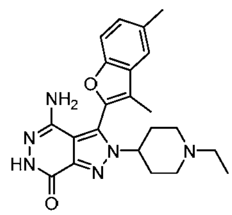 |

(continued)

| No. | Chemical Name | Structure |
|---|---|---|
| A25 | N-{4-[4-amino-3-(5-chloro-3-methyl-1-benzofuran-2-yl)-7-carbonyl-6,7-dihydro-2H-pyrazolo[3,4-d]pyridazin-2-yl]p henyl} prop-2-enamide | 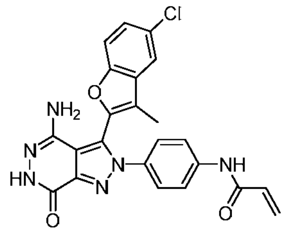 |
| A26 | (2E)-N-{4-[4-amino-3-(5-chloro-3-met hyl-1-benzofuran-2-yl)-7-carbonyl-6,7-dihydro-2H-pyrazolo[3,4-d]pyridazin-2-yl]phenyl}-4-(dimethylamino)but-2-enamide | 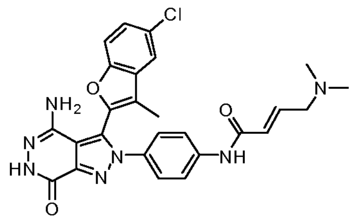 |
| A27 | (2E)-N-{4-[4-amino-3-(3,5-dimethyl-1-benzofuran-2-yl)-7-carbonyl-6,7-dihydro-2H-pyrazolo[3,4-d]pyridazin-2-yl]ph enyl}-4-(azetidin-1-yl)but-2-enamide | 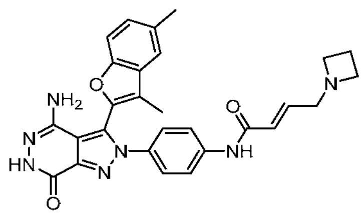 |
| A28 | (2E)-N-{4-[4-amino-3-(3,5-dimethyl-1-benzofuran-2-yl)-7-carbonyl-6,7-dihydro-2H-pyrazolo[3,4-d]pyridazin-2-yl]ph enyl}-4-(morpholin-4-yl)but-2-enamide | 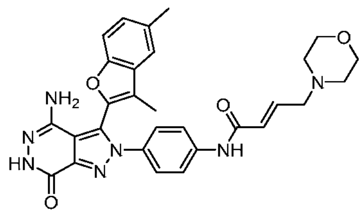 |
| A29 | 2-(1-acryloylpiperidin-4-yl)-4-amino-3-(3,5-dimethyl-1-benzofuran-2-yl)-2,6-dihydro-7H-pyrazolo [3,4-d]pyridazin-7-one | 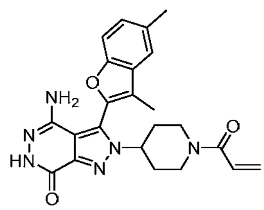 |
| A30 | 4-amino-2-{1-[(2E)-4-(dimethylamino) but-2-enoyl]piperidin-4-yl}-3-(3,5-dim ethyl-1-benzofuran-2-yl)-2,6-dihydro-7H-pyrazolo[3,4-d]pyridazin-7-one | 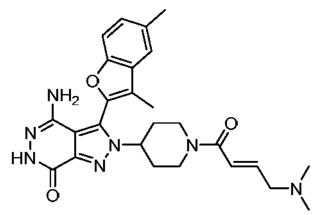 |
| A31 | 4-amino-2-{1-[(2E)-4-(azetidin-1-yl)bu t-2-enoyl]piperidin-4-yl}-3-(3,5-dimeth yl-1-benzofuran-2-yl)-2,6-dihydro-7H-py razolo[3,4-d]pyridazin-7-one | 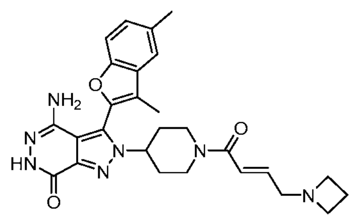 |

(continued)

| No. | Chemical Name | Structure |
|---|---|---|
| A32 | N-(4-[4-amino-3-(3,5-dimethyl-1-benzofu ran-2-yl)-7-carbonyl-6,7-dihydro-2H-pyra zolo[3,4-d]pyridazin-2-yl]-2-{ [(dimethyla mino)ethyl](methyl)amino}phenyl)prop-2-enamide | 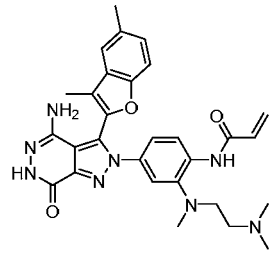 |
| A33 | N-{4-[4-amino-3-(3,5-dimethyl-1-benzofuran-2-yl)-7-carbonyl-6,7-dihydro-2H-pyrazolo[3,4-d]pyridazin-2-yl]cyclohexyl}prop-2-enamide | 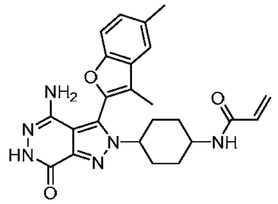 |
| A34 | 4-amino-2-(1-{(2E)-4-[cyclopropyl(me thyl)amino]but-2-enoyl} piperidin-4-yl)-3-(3,5-dimethyl-1-benzofuran-2-yl)-2,6-dihydro-7H-pyrazolo[3,4-d]pyridazin-7-one | 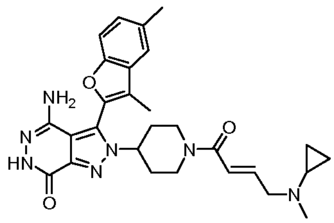 |
| A35 | N-{4-[4-amino-3-(3,5-dimethyl-1-benzofuran-2-yl)-7-carbonyl-6,7-dihydro-2H-pyrazolo[3,4-d]pyridazin-2-yl]cyclo hexyl}-N,N-dimethylglycinamide | 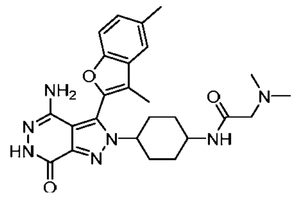 |
| A36 | 4-amino-2-{1-[2-(dimethylamino)ethyl]pi peridin-4-yl}-3-(3,5-dimethylbenzofuran-2-yl)-2H-pyrazolo[3,4-d]pyridazin-7(6H)-one | 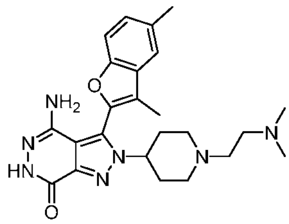 |
| A37 | 4-amino-3-(3,5-dimethyl-1-benzofuran-2-yl)-2-[(1-ethylpiperidin-4-yl)methyl]-2,6-dihydrogen-7H-pyrazolo[3,4-d]pyr idazin-7-one | 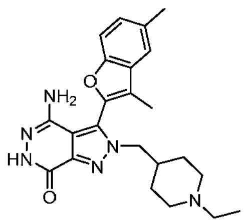 |

(continued)

| No. | Chemical Name | Structure |
|---|---|---|
| A38 | 2-[(1-acryloylpiperidin-4-yl) methyl]-4-amino-3-(3,5-dimethyl-1-benzofuran-2-yl)-2,6-dihydro-7H-pyrazolo [3,4-d]pyridazin-7-one | 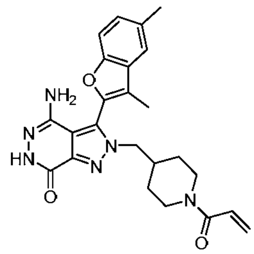 |
| A39 | 2-[(1-acryloylpiperidin-4-yl)methyl]-4-amino-3-(3,5-dimethyl-1-benzofuran-2-yl)-2,6-dihydro-7H-pyrazolo[3,4-d]pyridazin-7-one | 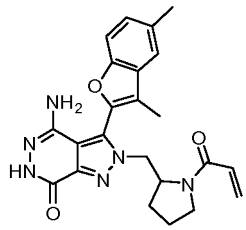 |
| A40 | 2-(1-acryloylpiperidin-3-yl)-4-amino-3-(3,5-dimethyl-1-benzofuran-2-yl)-2,6-dihydro-7H-pyrazolo[3,4-d]pyridazin-7-one | 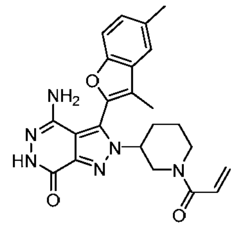 |
| A41 | 4-amino-2-{1-[2-(dimethylamino)ethyl]piperidin-4-yl}-3-(3,5-dimethylbenzofuran-2-yl)-2H-pyrazolo[3,4-d]pyridazine-7 (6H)-one | 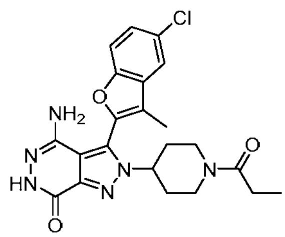 |
| A42 | 4-amino-3-(3,5-dimethyl-1-benzofuran-2-yl)-2-(tetrahydro-2H-pyran-4-yl)-2,6-dihydro-7H-pyrazolo[3,4-d]pyridazin-7-one | 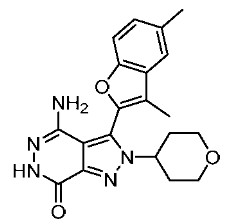 |
| A43 | 2-[(1-acryloylpiperidin-3-yl)methyl]-4-amino-3-(3,5-dimethyl-1-benzofuran-2--(3,5-dimethyl-yl)-2,6-dihydro-7H-pyrazolo[3,4-d] pyridazin-7-one | 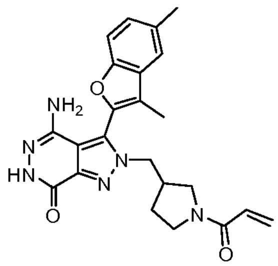 |

(continued)

| No. | Chemical Name | Structure |
|---|---|---|
| A44 | 4-amino-3-(3,5-dimethyl-1-benzofuran-2-yl)-2-{4-[(5-carbonylpyrrolidin-3-yl) amino]phenyl}-2 ,6-dihydro-7H-pyrazolo [3,4-d]pyridazin-7-one | 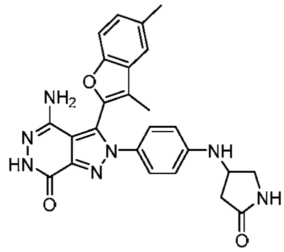 |
| A45 | 2-[(1-acryloylazetidin-3-yl)methyl]-4-amino-3-(3,5-dimethyl-1-benzofuran-2-yl)-2,6-dihydro-7H-pyrazolo[3,4-d]pyridazin-7-one | 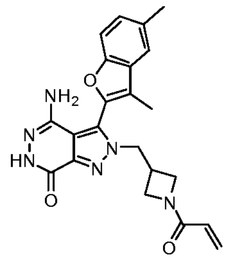 |
| A46 | 4-amino-3-(5-chloro-3-methyl-1-benzo furan-2-yl)-2-(4-{3-[2-(dimethylamino)ethyl]azetidine-1-yl}phenyl)-2,6-dihydro-7H-pyrazolo[ 3,4-d]pyridazin-7-one | 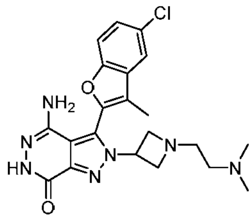 |
| A47 | 4-amino-3-(3,5-dimethyl-1-benzofuran-2-yl)-2-{1-[(4-methylpiperazin-1-yl)carbonyl]pyrrolidine-3-yl}-2,6-dihydro-7H-pyrazolo[3,4-d]pyridazin-7-one | 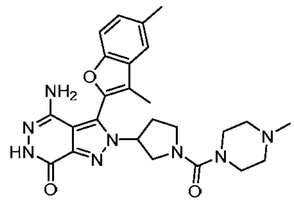 |
| A48 | 4-amino-3-(3,5-dimethyl-1-benzofuran-2-yl)-2-{1-[(1,1-dihydroxythiomorpholin-4-yl)carbonyl]pyrrolidin-3-yl}-2,6-dihydro-7H-pyrazolo[3,4-d]pyridazin-7-one | 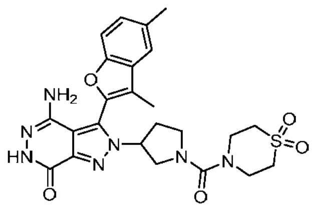 |
| A49 | 4-amino-2-{1-[(dimethylamino)acetyl]pyrrolidin-3-yl}-3-(3,5-dimethyl-1-benzofuran-2-yl)-2,6-dihydro-7H-pyrazolo [3,4-d] pyridazin-7-one | 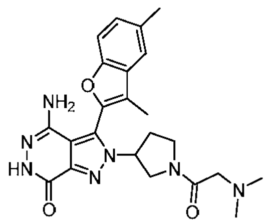 |

(continued)

| No. | Chemical Name | Structure |
|---|---|---|
| A50 | 4-amino-2-{[N-cyclohexyl-(2E)-4-metho xybut-2-enamide]-4-yl}-3-(3,5-dimethyl-1-benzofuran-2-yl)-2,6-dihydro-7H-pyraz olo[3,4-d]pyridazin-7-one | 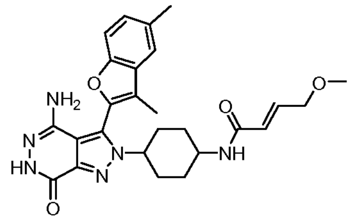 |
| A51 | 2-(1-acryloylpyrrolidin-3-yl)-4-amino-3-(3,5-dimethyl-1-benzofuran-2-yl)-2,6-dihydro-7H-pyrazolo[3,4-d]pyridazin-7-one | 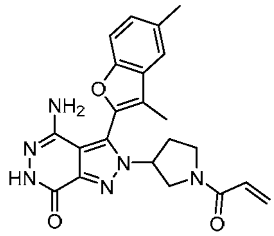 |
| A52 | 4-amino-3-(3,5-dimethyl-1-benzofuran-2-yl)-2-[1-(2-fluoroacryloyl)pyrrolidin-3-yl]-2,6-dihydro-7H-pyrazolo[3,4-d] pyridazin-7-one | 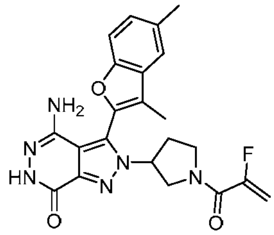 |
| A53 | 4-amino-3-(3,5-dimethyl-1-benzofuran-2-yl)-2-{1-[2-(pyrrolidin-1-ylmethyl)acryloyl]pyrrolidine-3-yl}-2,6-dihydro-7H-pyrazolo[3,4-d] pyridazin-7-one | 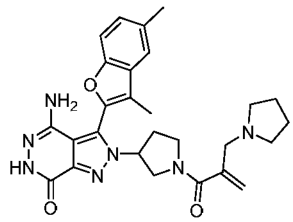 |
| A54 | 4-amino-3-(3,5-dimethyl-1-benzofuran-2-yl)-2-[1-(2-fluoroacryloyl)piperidin-4-yl]-2,6-dihydro-7H-pyrazolo[3,4-d] pyridazin-7-one | 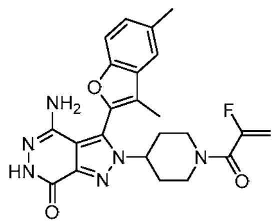 |
| A55 | 2-({4-[4-amino-3-(3,5-dimethyl-1-benzofuran-2-yl)-7-carbonyl-6,7-dihydro-2H-pyrazolo[3,4-d]pyridazin-2-yl]piperi din-1-yl} carbonyl)prop-2-enenitrile | 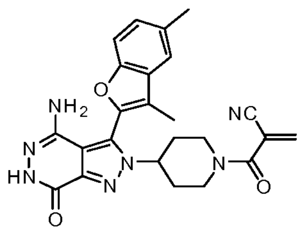 |

(continued)

| No. | Chemical Name | Structure |
|---|---|---|
| A56 | 4-amino-3-(5-chloro-3-methyl-1-benzofuran-2-yl)-2-{1-[(2E)-4-(cyclopropylamino)but-2-enoyl]piperidin-4-yl}-2,6-d ihydro-7H-pyrazolo[3,4-d]pyridazin-7-one | 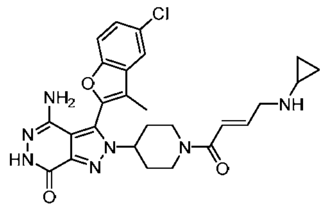 |
| A57 | 4-amino-3-(5-chloro-3-methyl-1-benzofuran-2-yl)-2-{1-[(2E)-4-(pyrrolidin-1-yl)butyl-2-enoyl]piperidin-4-yl}-2,6-dihydro-7H-pyrazolo[3,4-d]pyridazin-7-one | 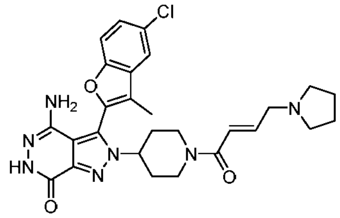 |
| A58 | N-{4-[4-Amino-3-(5-chloro-3-methyl-1-benzofuran-2-yl)-7-carbonyl-6,7-dihydro-2H-pyrazolo[3,4-d]pyridazin-2-yl]cy clohexyl}-2-fluoroprop-2-enamide | 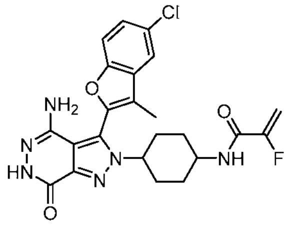 |
| A59 | N-{4-[4-amino-3-(3,5-dimethyl-1-benzofuran-2-yl)-7-carbonyl-6,7-dihydro-2H-pyrazolo[3,4-d]pyridazin-2-yl]cyclo hexyl}-2-cyanoprop-2-enamide | 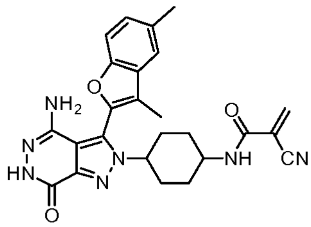 |
| A60 | (2E)-N-{4-[4-amino-3-(3,5-dimethyl-1-benzofuran-2-yl)-7-carbonyl-6,7-dihydro-2H-pyrazolo[3,4-d]pyridazin-2-yl] cyclohexyl}-4-(4-methylpiperazin-1-yl)but-2-enamide | 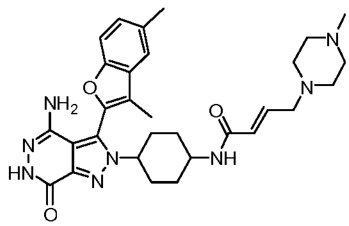 |
| A61 | (2Z)-N-{4-[4-Amino-3-(5-chloro-3-met hyl-1-benzofuran-2-yl)-7-carbonyl-6,7-dihydro-2H-pyrazolo[3,4-d]pyridazin-2-yl] cyclohexyl}-4-(dimethylamino)-2-fluorobut-2-enamide | 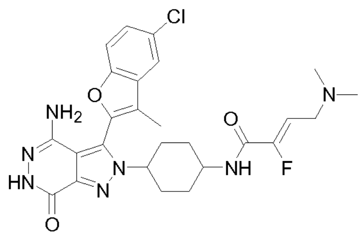 |
| A62 | 4-amino-3-(5-chloro-3-methyl-1-benzofuran-2-yl)-2-[1-(prop-2-ynyl)piperidin-4-yl]-2,6-dihydro-7H-pyrazolo[3,4-d]pyridazin-7-one | 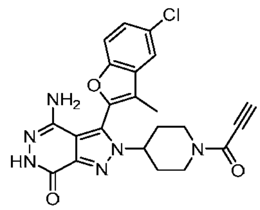 |

(continued)

| No. | Chemical Name | Structure |
|---|---|---|
| A63 | 4-amino-3-(3,5-dimethyl-1-benzofuran-2-yl)-2-[1-(4-hydroxybut-2-ynyl)pyrrolidin-3-yl]-2,6-dihydro-7H-pyrazolo[3,4-d]pyridazin-7-one | 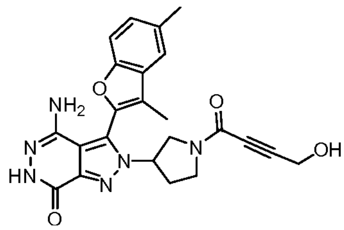 |
| A64 | N-{4-[4-Amino-3-(5-chloro-3-methyl-1-benzofuran-2-yl)-7-carbonyl-6,7-dihydro-2H-pyrazolo[3,4-d]pyridazin-2-yl]cy clohexyl}but-2-ynylamide | 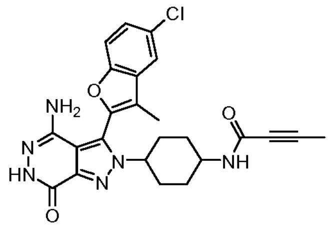 |
| A65 | 4-amino-3-(3,5-dimethyl-1-benzofuran-2-yl)-2-(1-{(2E)-4-[(3R)-3-fluoropyrrolidine-1-yl]but-2-enoyl}piperidin-4-yl)-2,6-dihydro-7H-pyrazolo[3,4-d]pyridazin-7-one | 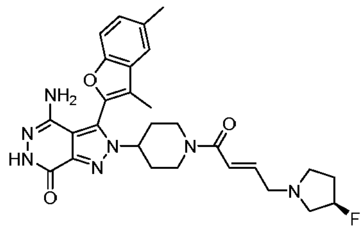 |
| A66 | 4-amino-3-(3,5-dimethyl-1-benzofuran-2-yl)-2-{1-[(2E)-4-(4-hydroxypiperidin-1-yl)-but-2-enoyl]piperidin-4-yl}-2,6-dihydro-7H-pyrazolo[3,4-d]pyridazin-7-one | 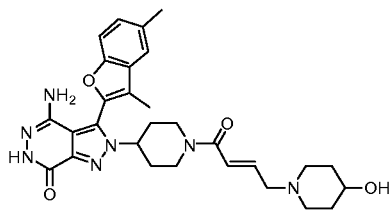 |
| A67 | 1-(1-acryloylpiperidin-4-yl)-4-amino-3-(3,5-dimethyl-1-benzofuran-2-yl)-1,6-dihydro-7H-pyrrolo[2,3-d]pyridazin-7-one | 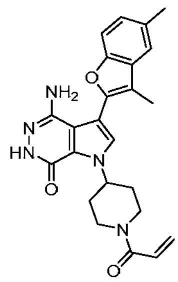 |
| A68 | 1-[(1-acryloylpiperidin-4-yl)methyl]-4-amino-3-(3,5-dimethyl-1-benzofuran-2-yl)-1,6-dihydrogen-7H-pyrrolo[2,3-d]pyridazin-7-one | 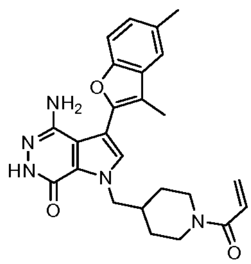 |

(continued)

| No. | Chemical Name | Structure |
|---|---|---|
| A69 | 1-[(3S)-1-acryloylpyrrolidin-3-yl]-4-amino-3-(3,5-dimethyl-1-benzofuran-2-yl)-1,6-dihydrogen-7H-pyrrolo[2,3-d]pyridazin-7-one | 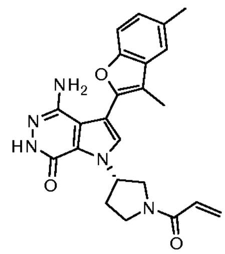 |
| A70 | (2E)-N-{4-[4-Amino-3-(1-methyl-1H-benzimidazol-2-yl)-7-carbonyl-6,7-dihydro-2H-pyrazolo[3,4-d]pyridazin-2-yl] phenyl}-4-(dimethylamino)but-2-enamide | 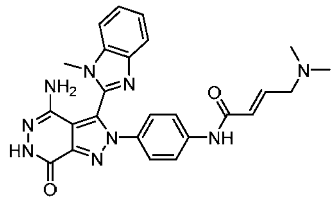 |
| A71 | (2E)-N-{4-[4-Amino-3-(1,3-dimethyl-1H-indol-2-yl)-7-carbonyl-6,7-dihydro-2H-pyrazolo[3,4-d]pyridazin-2-yl]pheny l}-4-(dimethylamino)but-2-enamide | 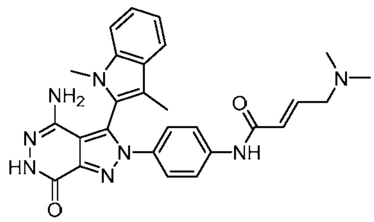 |
| A72 | (2E)-N-{4-[4-Amino-3-(1,3-benzothiazol-2-yl)-7-carbonyl-6,7-dihydro-2H-pyrazolo[3,4-d]pyridazin-2-yl]phenyl}-4-( dimethylamino)but-2-enamide | 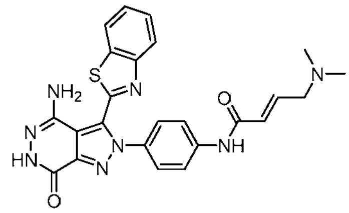 |
| A73 | (2E)-N-{4-[4-Amino-3-(3,5-dimethyl-1-benzofuran-2-yl)-7-carbonyl-6,7-dihydro-1H-pyrrolo[2,3-d]pyridazin-1-yl]cyclohexyl}-4-(azetidin-1-yl)but-2-enamide | 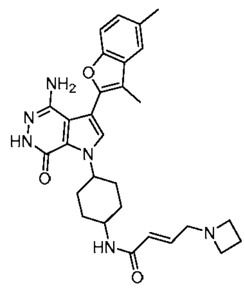 |
| A74 | 2-(1-acryloylpiperidin-4-yl)-4-amino-3-[(3,5-dimethoxyphenyl) ethynyl]-2,6-di hydro-7H-pyrazolo[3,4-d]pyridazin-7-o ne | 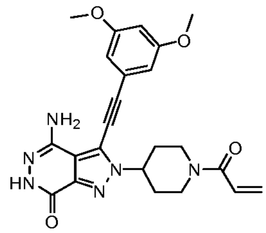 |

(continued)

| No. | Chemical Name | Structure |
|---|---|---|
| A75 | 4-amino-2-[1-(cyclopropylcarbonyl)piperidin-4-yl]-3-(3,5-dimethyl-1-benzofuran-2-yl)-2,6-dihydrogen-7H-pyrazolo[ 3,4-d]pyridazin-7-one | 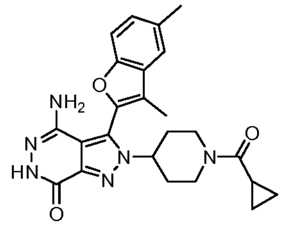 |
| A76 | N-{4-[4-Amino-3-(3,5-dimethyl-1-benzofuran-2-yl)-7-carbonyl-6,7-dihydro-2H-pyrazolo[3,4-d]pyridazin-2-yl]cyclo hexyl}-2-cyanoacetamide | 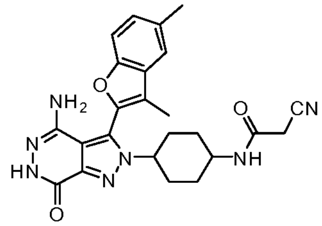 |
| A77 | 2-(1-acryloylpiperidin-4-yl)-4-amino-3-[(3,5-diethoxyphenyl) ethynyl]-2,6-dihydro-7H-pyrazolo[3,4-d]pyridazin-7-one | 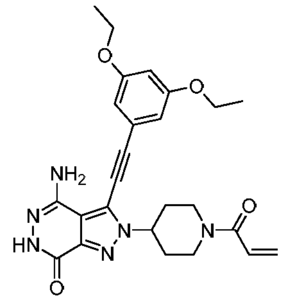 |
| A78 | N-{4-[4-Amino-3-[(3,5-dimethoxyphenyl)ethynyl]-7-carbonyl-6,7-dihydro-2H-pyrazolo [3,4-d]pyridazin-2-yl]phenyl} prop-2-enamide | 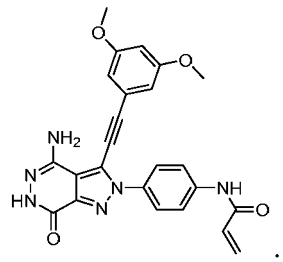 |
| A79 | 2-(1-acryloylpiperidin-3-yl)-4-amino-3-(5-methoxy-3-methylbenzofuran-2-yl)-2,6-dihydro-7H-pyrazolo[3,4-d]pyrida zin-7-one | 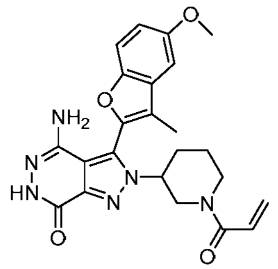 |
| A80 | 2-(1-acryloylpiperidin-4-yl)-4-amino-3-(5-fluoro-3-methylbenzofuran-2-yl)-2,6-dihydro-7H-pyrazolo[3,4-d]pyridazin -7-one | 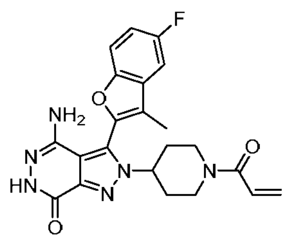 |

(continued)

| No. | Chemical Name | Structure |
|---|---|---|
| A81 | 2-(1-propylpiperidin-4-yl)-4-amino-3-(3-methyl-5-(trifluoromethyl) benzofuran-2-yl)-2,6-dihydro-7H-pyrazolo[3,4-d] pyridazin-7-one | 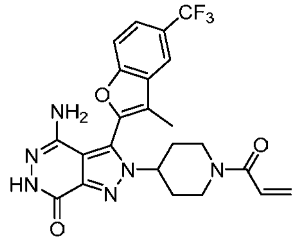 |
| A82 | 2-(1-acryloylpiperidin-4-yl)-4-amino-3-(3,6-dimethylbenzofuran-2-yl)-2,6-dihydro-7H-pyrazole[3,4-d]pyridazin-7-on e | 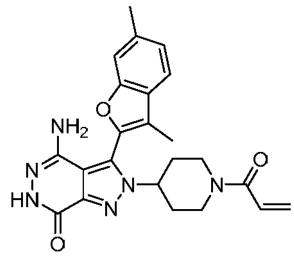 |
| A83 | 2-(1-acryloylpiperidin-4-yl)-4-amino-3-(6-fluoro-3-methylbenzofuran-2-yl)-2,6-dihydro-7H-pyrazole[3,4-d]pyridazin e-7-one | 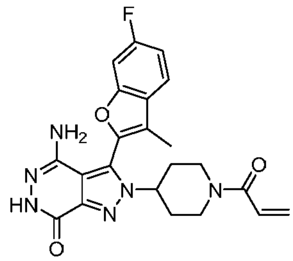 |
| A84 | 2-(1-acryloylpiperidin-4-yl)-4-amino-3-(6-chloro-3-methylbenzofuran-2-yl)-2,6-dihydro-7H-pyrazolo [3,4-d]pyridazin e-7-one | 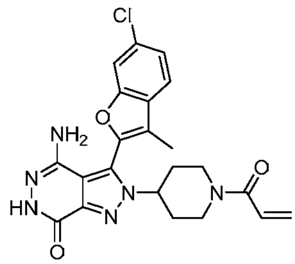 |
| A85 | 2-(1-acryloylpiperidin-4-yl)-4-amino-3-(3-ethyl-5-methylbenzofuran-2-yl)-2,6-dihydro-7H-pyrazolo[3,4-d]pyridazin-7-one | 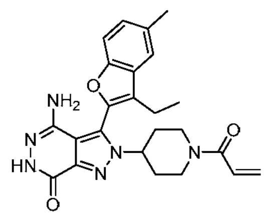 |
| A86 | 2-(1-acryloylpiperidin-4-yl)-4-amino-3-(5-chloro-3-methylbenzofuran-2-yl)-2,6-dihydro-7H-pyrazolo[3,4-d]pyridazin-7-one | 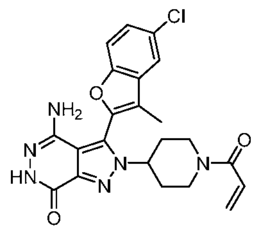 |

(continued)

| No. | Chemical Name | Structure |
|---|---|---|
| A87 | 2-(1-acryloylpiperidin-3-yl)-4-amino-3-(5-chloro-3-methylbenzofuran-2-yl)-2,6-dihydro-7H-pyrazolo[3,4-d]pyridazin e-7-one | 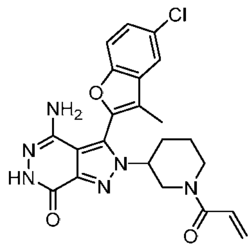 |
| A88 | N-(4-(4-amino-3-(3,5-dimethylbenzofuran-2-yl)-7-carbonyl-6,7-dihydro-2H-pyrazolo[3,4-d]pyridazin-2-yl)cyclohexy l) acryloylamide | 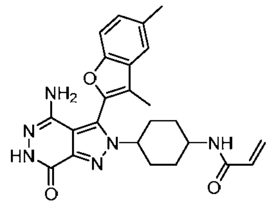 |
| A89 | (E)-2-(1-but-2-enoylpiperidin-3-yl)-4-amino-3-(5-methyl-3-methylbenzofuran-2-yl)-2,6-dihydro-7H-pyrazolo[3,4-d]pyridazin-7-one | 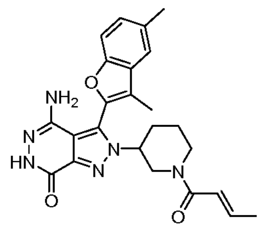 |
| A90 | (E)-2-(1-pent-2-enoylpiperidin-3-yl)-4-amino-3-(5-methyl-3-methylbenzofuran-2-yl)-2,6-dihydro-7H-pyrazolo[3,4-d] pyridazin-7-one | 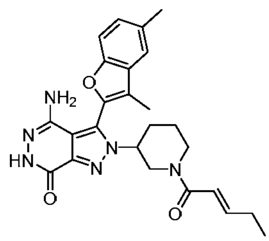 |
| A91 | 4-amino-3-(5-methyl-3-methyl-1-benzo furan-2-yl)-2-{1-[(2E)-4-(N,N-dimethyl)butyl-2-enoyl]piperidin-3-yl}-2,6-dihydro-7H-pyrazolo[3,4-d]pyridazin-7-one | 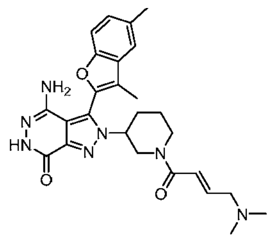 |
| A92 | 4-amino-3-(5-methyl-3-methyl-1-benzofuran-2-yl)-2-{1-[(2E)-4-(pyrrolidin-1-yl)butyl-2-enoyl]piperidin-3-yl}-2,6-di hydro-7H-pyrazolo[3,4-d]pyridazin-7-one | 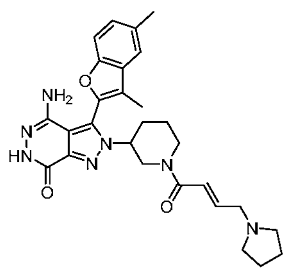 |

The present invention also provides a process for the preparation of the compound of formula (I). The materials and reagents used in the present invention are commercially available unless otherwise specified.

In the second aspect of the present invention, a method for preparing a five-membered heterocyclic [3,4-d]pyridazinone compound represented by the formula (I), an enantiomer thereof, a diastereomer thereof, a racemate or

the mixtures thereof, a pharmaceutically acceptable salt thereof, a hydrate or a solvate thereof is provided,

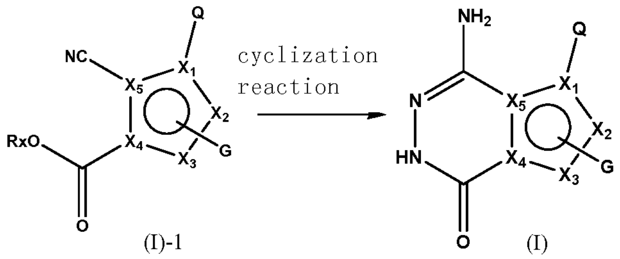


(I)-1 (I)

Wherein, the $X_1$, $X_2$, $X_3$, $X_4$, $X_5$, Q and G are the same as described above;
Rx is selected from C1-C6 alkyl, preferably methyl or ethyl;

The compound of the formula (I)-1 is cyclized with hydrazine hydrate to give a compound of the formula (I); preferably, the reaction between the compound of the formula (I)-1 and hydrazine hydrate can be carried out in a suitable solvent selected from the group consisting of polar solvents, preferably one or more of methanol, ethanol, isopropanol, acetonitrile and dioxane; the temperature of the reaction is selected from room temperature to 100 °C, and the reaction time is selected from 10 minutes to 24 hours;

According to the demand, the compound of the formula (I) thus obtained can be further converted by a substitution reaction, a reduction reaction, a condensation reaction or the like. For example, when G is a -L3-halogen-substituted C6-C12 aryl group, it may form a novel compound of the formula (I) with a compound such as amine, alcohol or 3-12 membered heterocyclic group containing a nitrogen atom through substitution reaction; when G is a -L3-nitro-substituted C6-C12 aryl group, an amino group can be obtained by a reduction reaction, and if necessary, it can further form a novel compound of the formula (I) with a carboxylic acid through condensation reactiono; or, an amino group substituted by acid halide to form a novel compound of the formula (I); when G is a -L3-substituted or unsubstituted nitrogen-containing 3-12 membered heterocyclic group or -L3-amino substituted C3-C8 cycloalkyl group, it can form a new compound of formula (I) with an acid halide or a halogenated product through substitution reaction;

The compound of formula (I)-1 can be prepared by the following reaction route:

(1) When all of $X_1$, $X_4$, and $X_5$ are C, and both of $X_2$ and $X_3$ are N,

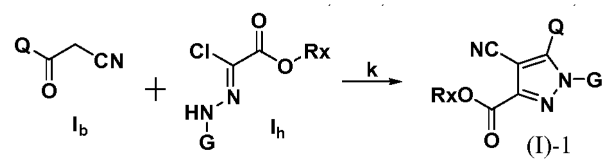


$I_b$ $I_h$ (I)-1

The compound Ib is dissolved in a solvent, and a base is added thereto. After stirring for 5 to 10 minutes, the compound $I_h$ is added, and after argon gas protection, the reaction is stirred at room temperature to obtain a compound (I)-1. The solvent is anhydrous ethanol or dimethyl chloride, and the base is sodium ethoxide or triethylamine;

Compound $I_b$ can be prepared by the following method:

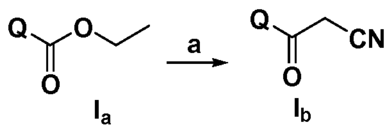


$I_a$ $I_b$

The compound $I_a$ is dissolved in dry tetrahydrofuran, sodium hydrogen and acetonitrile are added at room temperature, and stirred at 50 °C to obtain a compound $I_b$;

Compound $I_h$ can be prepared by the following method:

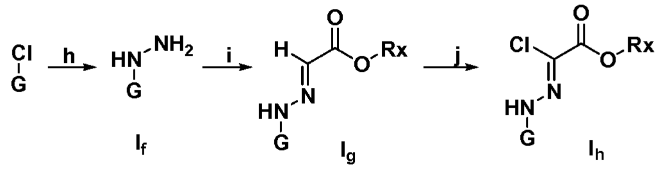

$I_f$ $I_g$ $I_h$

Step h: adding compound G-Cl to dichloromethane, adding hydrazine hydrate solution at room temperature, and stirring the reaction to obtain compound If;
Step i: Dissolving If in a solvent, adding sodium carbonate, adding a glyoxylate solution in toluene after stirring for 5 min, and then stirring the reaction at 50 °C to obtain a compound $I_g$, wherein the solvent is 1,4-dioxane and water;
Step j: Dissolving the compound $I_g$ in ethyl acetate, adding N-chlorosuccinimide, and stirring the reaction at room temperature to obtain the product $I_h$;
Compound $I_h$ can also be prepared by the following method:

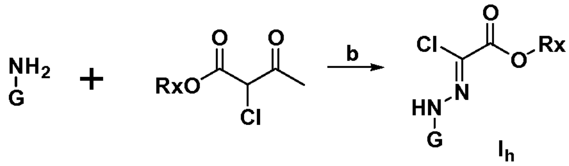

$I_h$

The compound G-$NH_2$ is suspended in a solvent, an aqueous solution of sodium nitrite is added thereto under ice bath. After reacted for half an hour, sodium acetate and 2-chloroacetoacetate in ethanol is further added, and the reaction is stirred to obtain compound $I_h$, while the solvent is water and concentrated hydrochloric acid;

(2) When all of $X_1$, $X_4$ and $X_5$ are C, $X_2$ is CH, and $X_3$ is N,

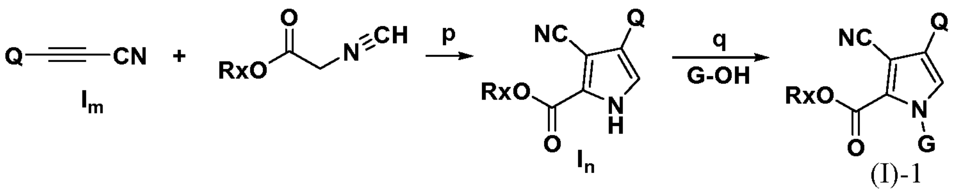

$I_m$ $I_n$ (I)-1

Step p: Dissolving compound $I_m$ and isocyanurate in 1,4-dioxane, adding a catalytic amount of 1,3-bis(diphenylphosphino)propane under argon atmosphere, and stirring at 110 °C to obtain a compound $I_n$;
Step q: Dissolving the compound $I_n$ and G-OH in tetrahydrofuran, adding triphenylphosphine, and adding diethyl azodicarboxylate in toluene dropwise under a nitrogen atmosphere, and stirring the reaction at room temperature to obtain a compound (I)-1;
Compound $I_m$ can be prepared by the following method:

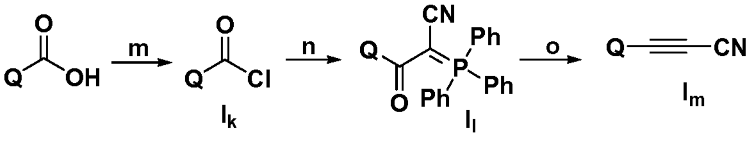

$I_k$ $I_l$ $I_m$

Step m: Dissolving Q-COOH in dry dichloromethane, adding oxalyl chloride and a catalytic amount of DMF at room temperature, and stirring at room temperature to obtain compound $I_{k;}$
Step n: adding cyanomethyltriphenylphosphonium chloride and triethylamine to dichloromethane, addinga compound $I_k$ solution in dichloromethane at room temperature, and stirring the reaction to obtain compound $I_l$;
Step o: Placing the compound li in a microwave tube, and stirring the reaction at 110 °C to obtain compound $I_m$;

**FGFR activity inhibitor and applications thereof**

The compounds of the invention can inhibit activities of one or more FGFR enzymes. For example, a compound of the invention can be used to inhibit the activity of the enzyme in a cell or an individual or patient in need of inhibition of FGFR enzyme by administering an inhibitory amount of a compound of the invention to the cell, subject or patient.

In some embodiments, the compounds of the invention are inhibitors of one or more of FGFR1, FGFR2, FGFR3, and FGFR4, for example, having inhibitory effects to all of the above enzymatic activities, or selectively inhibiting one or more FGFR enzymes. The above selectivity may be such that the IC50 value of inhibition is 2, 3, 5, 10, 50 or 100 times and above.

As FGFR inhibitors, the compounds of the invention are useful in the treatment of various diseases associated with FGFR enzyme activity, abnormal expression or activity of FGFR ligands. The abnormal proliferation disease associated with FGFR activity or expression amount includes, but is not limited to, the following cancer or tumor diseases: mammalian cancer or tumor (such as duct or lobular tumor), respiratory tumor (small cell lung cancer, non-small cell lung cancer, small cell/non-small cell cancer, bronchial carcinoma, bronchial adenoma, pleural pulmonary blastoma), brain tumors (eg brain stem and hypothalamic tumor, astrocytoma, malignant glioma, medulloblastoma, ependymoma, melanotic neuro-ectodermal tumor, pineal tumor), digestive organ tumor (esophageal cancer, gastric cancer, gallbladder cancer, small intestine cancer, colon cancer, rectal cancer, anal cancer), liver tumor (especially hepatocellular carcinoma , cholangiocarcinoma, mixed liver cancer), head and neck cancer (laryngocarcinoma, pharyngolaryngeal cancer, nasopharyngeal, oropharyngeal cancer, eye cancer, oral cancer), skin cancer (squamous cell carcinoma, Kaposi's sarcoma, Malignant melanoma, Merkel cell carcinoma, non-melanoma skin cancer), soft tissue tumors (especially soft tissue sarcoma, osteosarcoma, malignant fibrous histiocytoma, lymphosarcoma, rhabdomyosarcoma), ocular tumors (especially melanoma, uveal melanoma, retinoblastoma), endocrine and exocrine gland tumors (such as thyroid and parathyroid tumors, pancreatic and salivary gland tumors), urinary tract tumors (bladder cancer, penile tumor, kidney cancer, renal pelvis and ureteral tumors), genital tumors (female endometrial cancer, cervical cancer, ovarian cancer, vaginal cancer, vulvar cancer, uterine cancer, and male prostate cancer and testicular cancer), as well as metastatic tumors of these tumors.

Other exemplary cancers include proliferative blood diseases such as lymphoma and leukemia in solid form and blood cells, myeloproliferative diseases such as acute myeloid leukemia, acute lymphocytic leukemia, chronic myeloid leukemia, chronic myeloid leukemia, hairy cell leukemia and AIDS-associated lymphoma, Hodgkin's lymphoma, non-Hodgkin's lymphoma, cutaneous T-cell lymphoma, Burkitt's lymphoma, and central nervous system lymphoma.

Other cancers that can be treated with the compounds of the invention include ocular tumors, glioblastoma, melanoma, rhabdomyosarcoma, lymphoma, and osteosarcoma.

In addition to carcinogenic neoplasms, the compounds of the invention are also useful in the treatment of skeletal and chondrocyte disorders including, but not limited to, achondroplasia, rib dysplasia, dwarfism, thanatophoric dysplasia (TD), Bill-History Twenson Skin Roundabout Syndrome, Pfeiffer Syndrome and Craniosynostosis syndrome.

The compounds of the invention are also useful in the treatment of hypophosphatemia including, for example, X-linked hypophosphatemic rickets, autosomal recessive hypophosphatemia rickets, autosomal dominant hypophosphatemia rickets, and tumor-induced osteomalacia.

The compounds of the invention may additionally be useful in the treatment of fibrotic diseases, such as diseases symptoms or conditions characterized by fibrosis. Exemplary fibrotic diseases include cirrhosis, glomerulonephritis, pulmonary fibrosis, systemic fibrosis, rheumatoid arthritis, and wound healing.

The compounds of the invention are also useful in the treatment of psoriasis, plaque, bullous skin disease, atherosclerosis, restenosis, mesangial cell proliferative disorders, glomerular lesions, diabetic nephropathy, nephropathy and benign prostatic hyperplasia.

The compounds of the invention are also useful in the treatment of a variety of ocular diseases including, for example, age-related macular degeneration, dry macular degeneration, ischemic retinal vein occlusion, diabetic macular edema, diabetic retinopathy, and retinopathy of prematurity.

The compounds of the invention are also useful for preventing or inhibiting the metastasis of typical tumors.

The compounds of the invention may also be used in combination therapy, i.e. with one or more other agents or methods of treatment, such as in combination with antiviral agents, chemotherapeutic agents or other anticancer agents, immunopotentiators, immunosuppressive agents, radioactivity, anti-tumor and antiviral vaccines, interleukin therapy and/or tyrosine kinase inhibitors for the treatment of FGFR related diseases, disorders or conditions. The agent may be combined with a compound of the invention in a single dosage form, or the agent may act as a nucleoside and nucleotide reverse transcriptase inhibitor, a non-nucleoside reverse transcriptase inhibitor, a protease inhibitor, and other antiviral drugs.

**Compared with the prior art, the main advantages of the present invention includes:**

1. The present inventors have found that the five-membered heterocyclic [3,4-d]pyridazinone compound has FGFR kinase inhibitory activity. The five-membered heterocyclic [3,4-d]pyridazinone compounds reported in the present invention provide more options for the treatment of various diseases associated with FGFR activity, abnormal expression or activity of FGFR ligands, particularly cancer and other diseases associated with abnormal proliferation.
2. The compounds of the present invention exhibit comparable or even better therapeutic effects to existing FGFR inhibitors in zoological experiments.

The present invention will be further illustrated below with reference to the specific examples. It should be understood that these examples are only to illustrate the invention but not to limit the scope of the invention. The experimental methods with no specific conditions described in the following examples are generally performed under the conventional conditions, or according to the manufacturer's instructions. In the examples, the percentage of yield of the chemical experiment is calculated by molar ratio, and the percentage of the reagent solution of the pharmacological experiment is calculated by volume.
The starting materials used in the present invention are commercially available without being specifically described.
Some of the abbreviations used in the reaction schemes and examples are defined as follows:

| | |
|---|---|
| DMF | dimethylformamide |
| THF | Tetrahydrofuran |
| NCS | Chlorosuccinimide |
| TEA | Triethylamine |
| TLC | Thin layer chromatography |
| $Pd_2(dba)_3$ | Tris(dibenzylideneacetone)dipalladium |
| BINAP | (±)-2,2'-bis-(diphenylphosphino)-1,1'-binaphthyl |
| BOC | Tert-butoxycarbonyl |
| DCM | Dichloromethane |
| DMSO | Dimethyl sulfoxide |
| MW | microwave |

**Example 1 Preparation of**

**4-amino-3-(3-methyl-1-benzothiophen-2-yl)-2-phenyl-2,6-dihydro-7H-pyrazolo[3,4-d]py ridazine-7-one (A1)**

Reaction formula 1.1

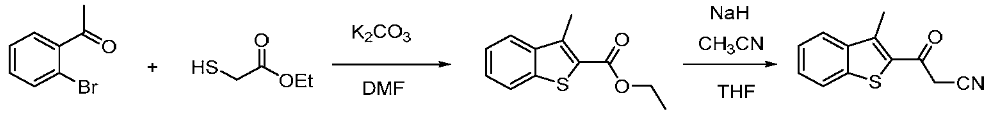


**Step** 1: Preparation of ethyl 3-methyl-1-benzothiophene-2-carboxylate (A1a)

2-Bromoacetophenone (2 g, 10.05 mmol) was dissolved in 20 mL of DMF, and potassium carbonate (2.08 g, 15.07 mmol) was added, then ethyl thloylyllate (1.2 mL, 11.05 mmol) was added at room temperature, and stirred at 100 °C overnight under the protection of nitrogen. After TLC monitored that the reaction was completed, the reaction solution was cooled to room temperature, 50 mL of water was added, and ethyl acetate (3×50 mL) was added for extraction. The combined organic layer was washed with water (3×50 mL) and saturated NaCl solution (50 mL), dried

over anhydrous $Na_2SO_4$ and filtered. The solvent was removed under reduced pressure, and the residue was isolated and purified by flash column chromatography (petroleum ether / ethyl acetate = 15/1, v / v) to give the product 1.2g, yield 54.50%. MS (ESI, m/z): 221(M +H)$^+$.

**Step** 2: Preparation of 3-(3-methyl-1-benzothiophen-2-yl)-3-carbonylpropionitrile (A1b)

A1a (1g, 4.54 mmol) was dissolved in 15 mL of dry THF, and then sodium hydrogen (236 mg, 5.9 mmol) was added and followed by acetonitrile (472 μl, 9.08 mmol) and stirred at 60 °C overnight under nitrogen protection. The solvent was evaporated under reduced pressure, and 15 mL of ice water was added, and then the mixture was acidified to pH 4-5 with 1N HCl solution to precipitate pale yellow solids, and filtered to give 800 mg of solid, which was dried under reduced pressure and used in the next step reaction without purification. MS (ESI, m/z): 216(M +H)$^+$.

Reaction formula 1.2

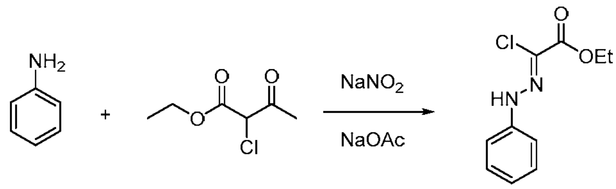


**Step** 3: Preparation of ethyl (2Z)-chloro(2-phenylhydrazine) acetate (A1c)

Aniline (8 g, 83.33 mmol) was added to a mixed solvent of 30 mL of water/HCl = 5/1, and an aqueous solution of sodium nitrite (6.32 g, 91.66 mmol) was added dropwise thereto under ice-cooling, and stirred for half an hour. Then sodium acetate (7.52 g, 91.66 mmol) and 25 mL of ethyl 2-chloroacetoacetate (15.1 g, 91.66 mmol) in ethanol were added. After TLC monitored that the reaction was completed, 40 mL of water was added, and ethyl acetate (3×80 mL) was added for extraction. The combined organic layer was washed with water (3×50 mL) and saturated NaCl solution (50 mL), dried over anhydrous $Na_2SO_4$, and filtered. The solvent was removed under reduced pressure, and the residue was isolated and purified by flash column chromatography (petroleum ether / ethyl acetate = 20/1, v / v) to give the product 12.5g, yield 66.2%. MS (ESI, m/z): 227(M +H)$^+$.

Reaction formula 1.3

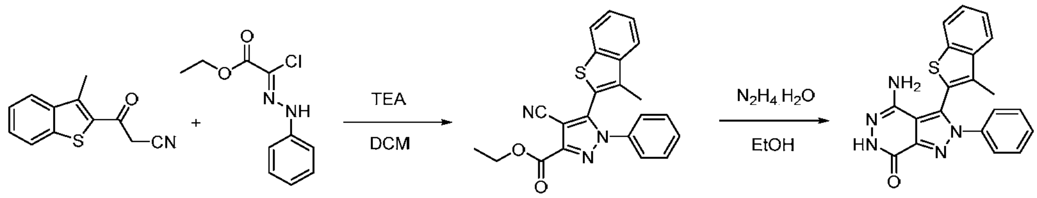


**Step** 4: Preparation of ethyl 4-cyano-5-(3-methyl-1-benzothiophen-2-yl)-1-phenyl-1H-pyrazole-3-carboxylate (A1d)

A1b (700 mg, 3.25 mmol) was added to 10 mL of dry DCM, then TEA (1.32 g, 13.01 mmol) was added. After stirring for 10 min, A1c (884 mg, 3.9 mmol) was added and reacted at room temperature. After TLC monitored that the reaction was completed, 20 mL of water was added, and ethyl acetate (3×30 mL) was added for extraction. The combined organic layer was washed with water (3×20 mL) and saturated NaCl solution (50 mL), dried over anhydrous $Na_2SO_4$, and filtered. The solvent was removed under reduced pressure, and the residue was isolated and purified by flash column chromatography (petroleum ether / ethyl acetate = 4/1, v / v) to give 680 mg of the mixture product of tautomer, yield 51.9%. MS (ESI, m/z): 388(M +H)$^+$. $^1$H NMR(400 MHz, DMSO-$d_6$) δ 7.84 - 7.78 (m, 1H), 7.76 - 7.73 (m, 1H), 7.48 - 7.43 (m, 2H), 7.40 - 7.34 (m, 5H), 4.54(q, J = 7.1 Hz, 2H, major), 4.12 (q, J = 7.1 Hz, 2H, minor), 2.26 (s, 3H, major), 2.05 (s, 2H, minor), 1.48 (t, J = 7.1 Hz, 3H, major), 1.26 (t, J = 7.1 Hz, 3H, minor).

**Step 5:** Preparation of A1

The above mixture of A1d (600 mg, 1.55 mmol) was dissolved in 5 mL of anhydrous ethanol, and then hydrazine hydrate (543 μl, 10.84 mmol) was added and refluxed at 90 °C overnight. After TLC monitored that the reaction was completed, the solvent was evaporated under reduced pressure and purified by flash column chromatography (dichloromethane/methanol = 15/1, v/v) to provide 400 mg of product (yield: 69.2%). MS (ESI, m/z): 374(M +H)$^+$. $^1$H NMR (400 MHz, DMSO-$d_6$) δ 11.44 (s, 1H), 8.00 (m, 1H), 7.85 - 7.79 (m, 1H), 7.44 - 7.35 (m, 7H), 5.00 (s, 2H), 2.12 (s, 3H).

**Example 2 Preparation of 4-amino-3-(naphthalen-2-yl)-2-phenyl-2,6-dihydro-7H-pyrazolo[3,4-d]pyridazin-7-one (A2)**

Compound A2 was prepared by the same method as in Example 1, except that ethyl 3-methyl-1-benzothiophene-2-carboxylate was replaced by ethyl 2-naphthoate. The yield of the last step was 49.5%.
MS (ESI, m/z): 354(M +H)$^+$.

**Example 3 Preparation of 4-amino-3-(1,3-dimethyl-1H-indol-2-yl)-2-phenyl-2,6-dihydro-7H-pyrazolo[3,4-d]pyridazin-7-one (A3)**

Reaction formula 3.1

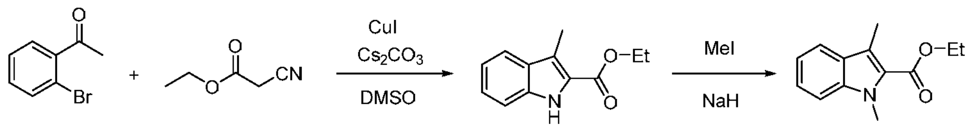


**Step 1:** Preparation of ethyl 3-methyl-1H-indole-2-carboxylate

2-bromoacetophenone (3g, 15.07mmol), CuI (287mg, 1.51mmol) and cesium carbonate (9.82g, 30.14mmol) were added to 40mL of DMSO, and ethyl cyanoacetate was slowly added at 50 °C under protect of nitrogen. After the reaction was completed, 40 mL of water was added, and ethyl acetate (3×50 mL) was added for extraction. The combined organic layer was washed with water (3×30 mL) and saturated NaCl solution (50 mL), dried over anhydrous $Na_2SO_4$ and filtered. The solvent was dried under reduced pressure, and the residue was isolated and purified by flash column chromatography (petroleum ether / ethyl acetate = 4/1, v / v) to afford the subject product 1.5 g, yield 49%.MS (ESI, m/z): 204(M +H)$^+$.

**Step 2:** Preparation of ethyl 1,3-dimethyl-1H-indole-2-carboxylate

The product of the above step (1.3 g, 6.4 mmol) was dissolved in 10 mL of dry DMF, sodium hydrogen (184 mg, 7.68 mmol) was added, and after stirred for 2 h, iodomethane (448 μl, 7.68 mmol) was added and the reaction was continued until complete. 40 mL of water and ethyl acetate (3×50 mL) was added for extraction. The combined organic layer was washed with water (3×30 mL) and saturated NaCl solution (50 mL), dried over anhydrous $Na_2SO_4$ and filtered. The solvent was removed under reduced pressure, and the residue was isolated and purified by flash column chromatography (petroleum ether / ethyl acetate = 6/1, v / v) to afford the subject product 1.25 g, yield 89.9%. MS (ESI, m/z): 218(M +H)$^+$.

**Step 3:** Preparation of A3

Compound A3 was prepared by the same method as in Example 1, except that ethyl 3-methyl-1-benzothiophene-2-carboxylate was replaced by ethyl 1,3-dimethyl-1H-indole-2-carboxylate. The yield of the last step was 32%. MS (ESI, m/z): 371(M +H)$^+$.

**Example 4 Preparation of 4-amino-3-(3,5-dimethyl-1-benzofuran-2-yl)-2-phenyl-2,6-dihydro-7H-pyrazolo[3,4-d]pyridazine-7-one (A4)**

Reaction formula 4.1

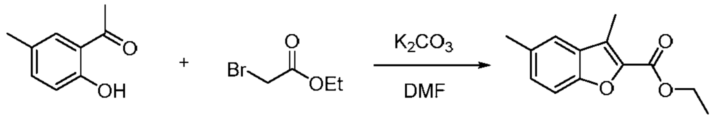


**Step 1:** Preparation of ethyl 3,5-dimethyl-1-benzofuran-2-carboxylate (A4a)

2-Hydroxy-5-methylacetophenone (10 g, 65.26 mmol) was dissolved in 70 mL of dry DMF, anhydrous potassium carbonate (13.53 g, 97.88 mmol) and ethyl bromoacetate (8.72 mL, 78.31mmol) were added, and stirred at 100 °C overnight. After TLC monitored that the reaction was completed, 80 mL of water was added and ethyl acetate (3×100 mL) was added for extraction. The combined organic layer was washed with water (3×80 mL) and saturated NaCl solution (80 mL), dried over anhydrous $Na_2SO_4$ and filtered. The solvent was removed under reduced pressure, and the residue was isolated and purified by flash column chromatography (petroleum ether / ethyl acetate = 10/1, v / v) to afford product 7.5g, yield 52.7%. MS (ESI, m/z): 219(M +H)$^+$.

**Step 2:** Preparation of A4

Compound A4 was prepared by the same method as in Example 1, except that ethyl 3-methyl-1-benzothiophene-2-carboxylate was replaced by ethyl 3,5-dimethyl-1-benzofuran-2-carboxylate. The yield of the last step was 40%. $^1$H NMR (500 MHz, DMSO-$d_6$) δ 11.49 (s, 1H), 7.49 - 7.41 (m, 7H), 7.27 - 7.15 (m, 1H), 5.19 (s, 2H), 2.40 (s, 3H), 1.87 (s, 3H).

**Example 5 Preparation of 4-amino-2-phenyl-3-(quinolin-3-yl)-2,6-dihydro-7H-pyrazolo[3,4-d]pyridazin-7-one (A5)**

Compound A5 was prepared by the same method as in Example 1, except that ethyl 3-methyl-1-benzothiophene-2-carboxylate was replaced by ethyl 3-quinolinecarboxylate. The yield of the last step was 53%. MS (ESI, m/z): 355(M +H)$^+$.

**Example 6 Preparation of 4-amino-3-(6-chloro-3-methyl-1-benzofuran-2-yl)-2-phenyl-2,6-dihydro-7H-pyrazolo[3,4 -d]pyridazin-7-one (A6)**

Compound A6 was prepared by the same method as in Example 1, except that ethyl 3-methyl-1-benzothiophene-2-carboxylate was replaced by ethyl 6-chloro-3-methyl-1-benzofuran-2-carboxylate. The yield of the last step was 32%. $^1$H NMR (400 MHz, DMSO-$d_6$) δ 11.50 (s, 1H), 7.78 (d, J = 1.4 Hz, 1H), 7.67 (d, J = 8.4 Hz, 1H), 7.48 - 7.40 (m, 5H), 7.37 (dd, J = 8.4, 1.4 Hz, 1H), 5.25 (s, 2H), 1.92 (s, 3H).

**Example 7 Preparation of 4-amino-3-(5-chloro-3-methyl-1-benzofuran-2-yl)-2-phenyl-2,6-dihydro-7H-pyrazolo[3,4 -d]pyridazin-7-one (A7)**

Compound A7 was prepared by the same method as in Example 1, except that ethyl 3-methyl-1-benzothiophene-2-carboxylate was replaced by ethyl 5-chloro-3-methyl-1-benzofuran-2-carboxylate. The yield of the last step was 37%. $^1$H NMR (400 MHz, DMSO-$d_6$) δ 11.50 (s, 1H), 7.78 (d, J = 2.1 Hz, 1H), 7.61 (d, J = 8.8 Hz, 1H), 7.49 - 7.38 (m, 6H), 5.24 (s, 2H), 1.92 (s, 3H).

**Example 8 Preparation of 4-amino-3-(6-chloro-3-methyl-1-benzofuran-2-yl)-2-phenyl-2,6-dihydro-7H-pyrazolo[3,4 -d]pyridazin-7-one (A8)**

Compound A8 was prepared by the same method as in Example 1, except that ethyl 3-methyl-1-benzothiophene-2-carboxylate was replaced by ethyl 6-fluoro -3-methyl-1-benzofuran-2-carboxylate. The yield of the last step was 39%. $^1$H NMR (400 MHz, DMSO-$d_6$) δ 11.49 (s, 1H), 7.75 - 7.65 (m, 1H), 7.56 (dd, J = 9.2, 2.2 Hz, 1H), 7.49 - 7.41 (m, 5H), 7.25 - 7.17 (m, 1H), 5.23 (s, 2H), 1.92 (s, 3H).

**Example 9 Preparation of 4-amino-3-(3,5-dimethyl-1-benzofuran-2-yl)-2-(4-{[(dimethylamino)methyl]amino}phen yl)-2,6 -dihydro-7H-pyrazolo[3,4-d]pyridazin-7-one (A9)**

**Step 1:** Preparation of 4-amino-2-(4-bromophenyl)-3-(3,5-dimethyl-1-benzofuran-2-yl)-2,6-dihydro-7H-pyrazolo[3, 4-d]pyridazin-7-one

The compound was prepared by the same method as in Example 1, except that ethyl 3-methyl-1-benzothiophene-2-carboxylate was replaced by ethyl 3,5-dimethyl-1-benzofuran-2-carboxylate. The yield of the last step was 41%. MS (ESI, m/z): 451(M +H)$^+$

Reaction formula 9.1

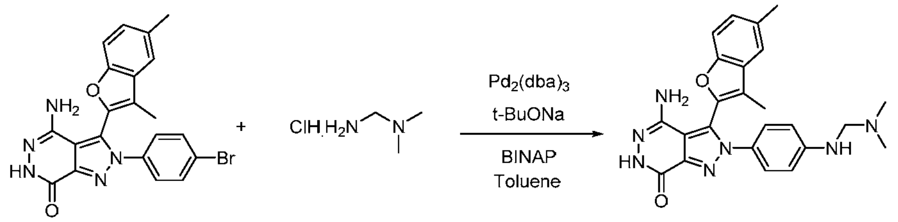


**Step 2:** Preparation of A9

The product of the previous step (200 mg, 0.44 mmol) was dissolved in 5 mL of toluene, and N,N-dimethyldiamine hydrochloride (147 mg, 1.33 mmol), $Pd_2(dba)_3$ (81 mg, 0.09mmol), BINAP (55 mg, 0.09 mmol) and sodium tert-butoxide (128 mg, 1.33 mmol) were added, and stirred at 100 °C overnight under argon atmosphere. After TLC monitored that the reaction was finished, 20 mL of water was added and ethyl acetate (3×30 mL) was added for extraction. The combined organic layer was washed successively with water (3×30 mL) and 40 mL of saturated NaCl solution, dried over anhydrous $Na_2SO_4$ ands filtered. The solvent was removed under reduced pressure. The residue was isolated and purified by flash column chromatography (dichloromethane/methanol = 15/1, v/v) to afford 86mg of the product, yield 43.6%. MS (ESI, m/z): 509(M +H)$^+$.

**Example 10 Preparation of 4-amino-3-(3,5-dimethyl-1-benzofuran-2-yl)-2-(1-propionylpiperidin-4-yl)-2,6-dihydro-7 H-pyrazolo[3,4-d]pyridazin-7-one (A10)**

The compound was prepared by the same method as in Example 29, except that propionyl chloride was replaced by acryloyl chloride. The yield of the last step was 42%. MS (ESI, m/z): 435(M +H)$^+$.

**Example 11 Preparation of 4-amino-2-(4-{[2-(dimethylamino)ethyl](methyl)amino}phenyl)-3-(3,5-dimethyl-1-benzo furan-2-yl)-2,6-dihydro-7H-pyrazolo[3,4-d]pyridazin-7-one (A11)**

Compound A11 was prepared by the same method as in Example 9, except that N,N-dimethylmethylenediamine hydrochloride was replaced by N,N,N-trimethylethylenediamine. The yield of the last step was 42%. MS (ESI, m/z): 472(M +H)$^+$.

**Example 12 Preparation of 4-amino-3-(3,5-dimethyl-1-benzofuran-2-yl)-2-[4-(morpholin-4-yl)phenyl]-2,6-dihydro-7 H-pyrazolo[3,4-d]pyridazin-7-one (A12)**

Compound A12 was prepared by the same method as in Example 9, except that N,N,N'-trimethylethylenediamine was replaced by morpholine. The yield of the last step was 34%. MS (ESI, m/z): 457(M +H)$^+$.

**Example 13 Preparation of 4-amino-2-(4-chlorophenyl)-3-(3,5-dimethyl-1-benzofuran-2-yl)-2,6-dihydro-7H-pyrazol o[3,4-d]pyridazine-7-one (A13)**

Compound A13 prepared by the same method as in Example 1, except that aniline was replaced by p-chloroaniline. The yield of the last step was 40%. $^1H$ NMR (500 MHz, DMSO-$d_6$) $\delta$ 11.50 (s, 1H), 7.59 - 7.48 (m, 3H), 7.42 (s, 1H), 7.30 (d, J = 7.5 Hz, 2H), 7.07 (d, J = 8.0Hz, 1H), 5.16 (s, 2H), 2.46 (s, 3H), 1.89 (s, 3H).

**Example 14 Preparation of 4-amino-3-(3,5-dimethyl-1-benzofuran-2-yl)-2-{4-[2-(pyrrolidin-1-yl)ethoxy]phenyl}-2,6 -dihydro-7H-pyrazolo[3,4-d]pyridazin-7-one (A14)**

Compound A14 was prepared by the same method as in Example 9, except that N,N-dimethylmethylenediamine hydrochloride was replaced by N-(2-hydroxyethyl)-pyrrolidine. The yield of the last step was 14%. MS (ESI, m/z): 485(M +H)$^+$.

**Example 15 Preparation of 4-amino-2-{4-[2-(dimethylamino)ethoxy]phenyl}-3-(3,5-dimethyl-1-benzofuran-2-yl)-2,6 -dihydro-7H-pyrazolo[3,4-d]pyridazin-7-one (A15)**

Compound A15 was prepared by the same method as in Example 9, except that N,N-dimethylmethylenediamine hydrochloride was replaced by N,N-dimethylethanolamine. The yield of the last step was 16%. MS (ESI, m/z): 459(M +H)$^+$.

**Example 16 Preparation of 4-amino-2-[4-(4-cyclopropylpiperazin-1-yl)phenyl]-3-(3,5-dimethyl-1-benzofuran-2-yl)-2 ,6-dihydro-7H-pyrazolo[3,4-d]pyridazin-7-one (A16)**

Compound A16 was prepared by the same method as in Example 9, except that N,N-dimethylmethyldiamine hydrochloride was replaced by 1-cyclopropylpiperazine. MS (ESI, m/z): 496(M +H)$^+$.

**Example 17 Preparation of 4-amino-2-{4-[3-(dimethylamino)azetidin-1-yl]phenyl}-3-(3,5-dimethyl-1-benzofuran-2-yl)-2,6-dihydro-7H-pyrazolo[3,4-d]pyridazin-7-one (A17)**

Compound A16 was prepared by the same method as in Example 9, except that N,N-dimethyldiamine hydrochloride was replaced by 3-(dimethylamino)azetidine dihydrochloride. The yield of the last step was 27%. MS (ESI, m/z): 470(M +H)$^+$.

**Example 18 Preparation of 4-amino-3-(3,5-dimethyl-1-benzofuran-2-yl)-2-[4-(4-ethylpiperazin-1-yl)phenyl]-2,6 -dihydro-7H-pyrazolo[3,4-d]pyridazin-7-one (A18)**

Compound A18 was prepared by the same method as in Example 9, except that N,N-dimethylmethyldiamine hydrochloride was replaced by N-ethylpiperazine. MS (ESI, m/z): 484(M +H)$^+$.

**Example 19 Preparation of 4-amino-2-(4-{[3-(dimethylamino)propyl](methyl)amino}phenyl)-3-(3,5-dimethyl-1-benz ofuran-2-yl)-2,6-dihydro-7H-pyrazolo[3,4-d]pyridazin-7-one (A19)**

Compound A19 was prepared by the same method as in Example 9, except that N,N-dimethylmethylenediamine hydrochloride was replaced by N,N,N'-trimethyl-1,3-propane diamine. The yield of the last step was 33%. MS (ESI, m/z): 486(M +H)$^+$.

**Example 20 Preparation of 4-amino-3-(3,5-dimethyl-1-benzofuran-2-yl)-2-(4-{[(1-methylpiperidin-4-yl)methyl]amin o}phenyl)-2,6-dihydro-7H-pyrazolo[3,4-d]pyridazin-7-one (A20)**

Compound A20 was prepared by the same method as in Example 9, except that N,N-dimethylmethylenediamine hydrochloride was replaced by (1-methyl-4-piperidine-)methylamine. The yield of the last step was 35%. MS (ESI, m/z): 498(M +H)$^+$.

**Example 21 Preparation of 4-amino-3-(3,5-dimethyl-1-benzofuran-2-yl)-2-{4-[4-(methoxymethyl)piperidin-1-yl]phe nyl}-2,6-dihydro-7H-pyrazolo[3,4-d]pyridazin-7-one (A21)**

Compound A21 was prepared by the same method as in Example 9, except that N,N-dimethylmethylenediamine hydrochloride was replaced by 4-(methoxymethyl)piperidine. The yield of the last step was 26%. MS (ESI, m/z): 499(M +H)$^+$.

**Example 22 Preparation of 4-amino-2-{4-[3-(diethylamino)pyrrolidin-1-yl]phenyl}-3-(3,5-dimethyl-1-benzofuran-2-yl) -2,6-dihydro-7H-pyrazolo[3,4-d]pyridazin-7-one (A22)**

Compound A22 was prepared by the same method as in Example 9, except that N,N-dimethylmethylenediamine hydrochloride was replaced by 3-(diethylamino)pyrrolidine. The yield of the last step was 34%. MS (ESI, m/z): 512(M +H)$^+$.

**Example 23 Preparation of 4-amino-3-(5-chloro-3-methyl-1-benzofuran-2-yl)-2-(1-ethylpiperidin-4-yl)-2,6-dihydro-7H-pyrazolo[3,4-d]pyridazin-7-one (A23)**

Reaction formula 23.1

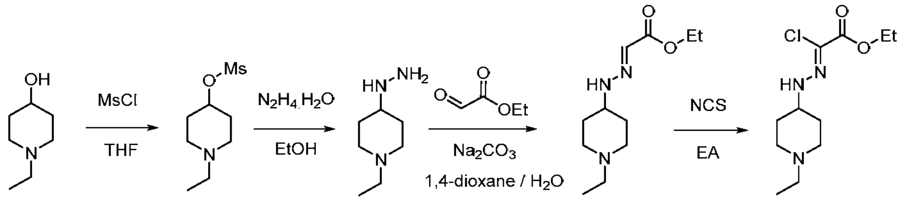


**Step 1:** Preparation of 1-ethylpiperidin-4-ylmethanesulfonate (A23a)

N-Ethyl-4-hydroxypiperidine (3 g, 23.22 mmol) was dissolved in 20 mL of dry THF, TEA was added, and methanesulfonyl chloride (1.98 mL, 25.54 mmol) was slowly added dropwise under ice-cooling, and stirred at room temperature until the reaction was completed. 40 mL of water was added and ethyl acetate (3×50 mL) was used for extraction. The combined organic layer was washed successively with water (3×30 mL) and saturated NaCl solution (50 mL), dried over anhydrous $Na_2SO_4$ and filtered. The solvent was removed under reduced pressure, and the residue was isolated and purified by flash column chromatography (petroleum ether / ethyl acetate = 4/1, v / v) to give the product 4.0g, yield 81.2%. MS (ESI, m/z): 208(M +H)$^+$.

**Step 2:** Preparation of ethyl (2E)-[2-(1-ethylpiperidin-4-yl)hydrazono]acetate (A23b)

A23a (3 g, 14.47 mmol) was dissolved in 15 mL of absolute ethanol, and hydrazine hydrate (1.45 mL, 28.95 mmol) was added, and the reaction was stirred with heating at 50 °C. After the reaction was completed, the solvent was concentrated under reduced pressure, and was used for the next step reaction without purification. The concentrated residue was dissolved in 20 mL of mixed solvent (1,4-dioxane/water = 5/1), and ethyl glyoxylate in toluene (8.6 mL, 41.89 mmol) and sodium carbonate (4.44 g, 41.89 mmol) were added, the reaction was stirred with heating at 50 °C. 40 mL of water was added and ethyl acetate (3×50 mL) was added for extraction. The combined organic layer was washed successively with water (3×30 mL) and saturated NaCl solution (50 mL), dried over anhydrous $Na_2SO_4$ and filtered. The solvent was removed under reduced pressure, and the residue was isolated and purified by flash column chromatography (petroleum ether / ethyl acetate = 6/1, v/v) to give 3.7g product. MS (ESI, m/z): 228(M +H)$^+$.

**Step 3:** Preparation of ethyl (2Z)-chloro[2-(1-ethylpiperidin-4-yl)hydrazono]acetate (A23c)

A23b (3 g, 13.2 mmol) was dissolved in 15 mL of ethyl acetate, NCS (2.47 g, 18.48 mmol) was added, and the reaction was stirred with heating at 50 °C. 40 mL of water was added and ethyl acetate (3×50 mL) was added for extraction. The combined organic layer was washed successively with water (3×30 mL) and saturated NaCl solution (50 mL), dried over anhydrous $Na_2SO_4$ and filtered. The solvent was removed under reduced pressure, and the residue was isolated and purified by flash column chromatography (petroleum ether / ethyl acetate = 6/1, v/v) to give the product 2.9g, yield 84.0%. MS (ESI, m/z): 262(M +H)$^+$.

**Step 4:** 4-amino-3-(5-chloro-3-methyl-1-benzofuran-2-yl)-2-(1-ethylpiperidin-4-yl)-2,6-dihydro-7H-pyrazolo[3,4-d]pyridazin-7-one (A23)

Compound A23 was prepared by the same method as in Example 7, except that ethyl (2Z)-chloro(2-phenylhydrazono]acetate was replaced by ethyl (2Z)-chloro[2-(1-ethylpiperidin-4-yl)hydrazono]acetate. The yield of the last step was 31%. MS (ESI, m/z): 427(M +H)$^+$.

**Example 24 Preparation of 4-amino-3-(3,5-dimethyl-1-benzofuran-2-yl)-2-(1-ethylpiperidin-4-yl)-2,6-dihydro-7H-p yrazolo[3,4-d]pyridazin-7-one (A24)**

Compound A24 was prepared by the same method as in Example 23, except that ethyl 5-chloro-3-methyl-1-benzofuran-2-carboxylate with ethyl 3,5-dimethyl-1-benzofuran-2-carboxylate. The yield of the last step was 37%. $^1$H NMR (400 MHz, DMSO-$d_6$) δ 11.49 (s, 1H), 7.60 (d, J = 1.5 Hz, 1H), 7.55 (d, J = 7.5 Hz, 1H), 7.42 - 7.35 (m, 1H), 5.21

(s, 2H), 3.87 - 3.79 (m, 1H), 3.24 (t, J = 7.1 Hz, 2H), 2.56 - 2.51 (m, 4H), 1.90 (s, 3H), 1.83 -1.76 (m, 4H), 1.18 (q, J = 7.1 Hz, 3H).

**Example 25 Preparation of N-{4-[4-amino-3-(5-chloro-3-methyl-1-benzofuran-2-yl)-7-carbonyl-6,7-dihydro-2H-pyr azolo[3,4-d]pyridazin-2-yl]phenyl}prop-2-enamide (A25)**

Reaction formula 25.1

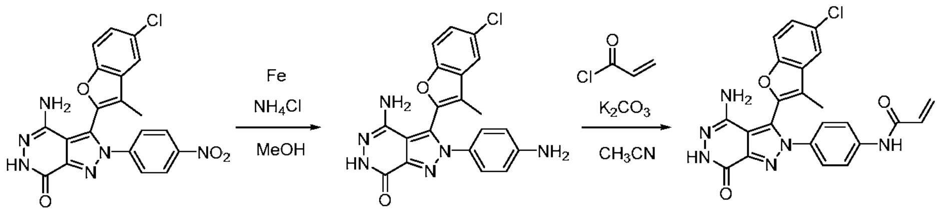


**Step 1:**

4-amino-3-(5-chloro-3-methyl-l-benzofuran-2-yl)-2-(4-nitrophenyl)-2,6-dihydro-7H-pyr azolo[3,4-d Pyridazine-7-one was prepared by the same method as in Example 7, except that aniline was replaced by p-nitroaniline, yield 33%. $^1$H NMR (400 MHz, DMSO-$d_6$) δ 11.55 (s, 1H), 8.35 - 8.28 (m, 2H), 7.76 - 7.69 (m, 2H), 7.52 - 7.44 (m, 2H), 7.25 (dd, J = 8.5, 1.3 Hz, 1H), 5.27 (s, 2H), 1.97 (s, 3H).

**Step 2:**

The product of the above step (1 g, 2.29 mmol) was dissolved in 10 mL of methanol, then ammonium chloride (367 mg, 6.87 mmol) and iron powder (384 mg, 6.87 mmol) were added and stirred at 70 °C until the reaction was completed. The solvent was directly dried under reduced pressure to give pale yellow solids which were used for the next step without purification.

1 g of the product obtained above was dissolved in 10 mL of acetonitrile, potassium carbonate (679 mg, 4.92 mmol) was added, and acryloyl chloride (404 μL, 4.92 mmol) was added thereto by stirring. The mixture was stirred at room temperature until the reaction was completed. 30 mL of water was added and ethyl acetate (3×40 mL) was added for extraction. The combined organic layer was washed successively with water (3×30 mL) and saturated NaCl solution (50 mL), dried over anhydrous $Na_2SO_4$ and filtered. The solvent was dried under reduced pressure, and the residue was isolated and purified by flash column chromatography (dichloromethane/methanol = 10/1, v/v) to give 200 mg product (compound A25). $^1$H NMR (400 MHz, DMSO--$d_6$) δ 11.50 (s, 1H), 10.43 (s, 1H), 7.82 - 7.69 (m, 3H), 7.64 (d, J = 8.8 Hz, 1H), 7.47 - 7.35 (m, 3H), 6.43 (dd, J = 17.0, 10.1 Hz, 1H), 6.26 (d, J = 17.0 Hz, 1H), 5.78 (d, J = 10.1 Hz, 1H), 5.23 (s, 2H), 1.95 (s, 3H).

**Example 26 Preparation of (2E)-N-{4-[4-amino-3-(5-chloro-3-methyl-1-benzofuran-2-yl)-7-carbonyl-6,7-dihydro-2H - pyrazolo[3,4-d]pyridazin-2-yl]phenyl}-4-(dimethylamino)but-2-enamide (A26)**

Compound A26 was prepared by the same method as in example 25, except that acryloyl chloride was replaced by (E)-4-(dimethylamino)but-2-enoyl chloride. MS (ESI, m/z): 518(M +H)$^+$.

**Example 27 Preparation of (2E)-N-{4-[4-amino-3-(3,5-dimethyl-1-benzofuran-2-yl)-7-carbonyl-6,7-dihydro-2H-pyra zolo[3,4-d]pyridazin-2-yl]phenyl}-4-(azetidin-1-yl)but-2-enamide (A27)**

Compound A27 was prepared by the same method as in example 25, except that ethyl 5-chloro-3-methyl-1-benzofuran-2-carboxylate was replaced by ethyl 3,5-dimethyl-1-benzofuran-2-carboxylate, and acryloyl chloride was replaced by (E)-4-(azetidin-1-yl)but-2-enoyl chloride. MS (ESI, m/z): 510(M +H)$^+$.

**Example 28 Preparation of (2E)-N-{4-[4-amino-3-(3,5-dimethyl-1-benzofuran-2-yl)-7-carbonyl-6,7-dihydro-2H-pyra zolo[3,4-d]pyridazin-2-yl]phenyl}-4-(morpholin-4-yl)but-2-enamide (A28)**

Compound A28 was prepared by the same method as in example 27, except that (E)-4-(azetidin-1-yl)but-2-enoyl chloride was replaced by (E)-4-(morpholin-4-yl)but-2-enoyl chloride. MS (ESI, m/z): 540(M +H)$^+$.

**Example 29 Preparation of 2-(1-acryloylpiperidin-4-yl)-4-amino-3-(3,5-dimethyl-1-benzofuran-2-yl)-2,6-dihydro-7H -pyrazolo[3,4-d]pyridazin-7-one (A29)**

Compound A29 was prepared by the same method as in Example 36, except that 2-chloro-N,N-dimethylethylamine was replaced by acryloyl chloride. MS (ESI, m/z): 433(M +H)$^+$.

**Example 30 Preparation of 4-amino-2-{1-[(2E)-4-(dimethylamino)but-2-enoyl]piperidin-4-yl}-3-(3,5-dimethyl-1-ben zofuran-2-yl)-2,6-dihydro-7H-pyrazolo[3,4-d]pyridazin-7-one (A30)**

Compound A30 was prepared by the same method as in example 29, except that acryloyl chloride was replaced by (E)-4-(dimethylamino)but-2-enoyl chloride. MS (ESI, m/z): 490(M +H)$^+$.

**Example 31 Preparation of 4-amino-2-{1-[(2E)-4-(azetidin-1-yl)but-2-enoyl]piperidin-4-yl}-3-(3,5-dimethyl -1-benzofuran-2-yl)-2,6-dihydro-7H-pyrazolo[3,4-d]pyridazin-7-one (A31)**

Compound A31 was prepared by the same method as in Example 29, except that acryloyl chloride was replaced by (E)-4-(azetidin-1-yl)but-2-enoyl chloride. MS (ESI, m/z): 502(M +H)$^+$.

**Example 32 Preparation of N-(4-[4-amino-3-(3,5-dimethyl-1-benzofuran-2-yl)-7-carbonyl-6,7-dihydro-2H-pyrazolo[ 3,4-d]pyridazin-2-yl]-2-{[(dimethylamino)ethyl](methyl)amino}phenyl)prop-2-enamide (A33)**

Compound A32 was prepared by the same method as in Example 25, except that ethyl 5-chloro-3-methyl-1-benzofuran-2-carboxylate with ethyl 3,5-dimethyl-1-benzofuran-2-carboxylate, and p-aminonitrobenzene was replaced by 2-(N,N',N'-trimethylethylenediamine)-4-aminonitrobenzene. MS (ESI, m/z): 541(M +H)$^+$

**Example 33 Preparation of N-{4-[4-amino-3-(3,5-dimethyl-1-benzofuran-2-yl)-7-carbonyl-6,7-dihydro-2H-pyrazolo[ 3,4-d]pyridazin-2-yl]cyclohexyl}prop-2-enamide (A33)**

Compound A33 was prepared by the same method as in Example 32, except that 2-(N,N',N'-trimethylethylenediamine)-4-aminonitrobenzene was replaced by 4-nitrocyclohexylamine. MS (ESI, m/z): 447(M +H)$^+$.

**Example 34 Preparation of 4-amino-2-(1-{(2E)-4-[cyclopropyl(methyl)amino]but-2-enoyl}piperidin-4-yl)-3-(3,5-dim ethyl-1-benzofuran-2-yl)-2,6-dihydro-7H-pyrazolo[3,4-d]pyridazin-7-one (A34)**

Compound A34 was prepared by the same method as in example 29, except that acryloyl chloride was replaced with 4-(N-methyl-N-cyclopropyl)aminobut-2-enoyl chloride. MS (ESI, m/z): 516(M +H)$^+$.

**Example 35 Preparation of N-{4-[4-amino-3-(3,5-dimethyl-1-benzofuran-2-yl)-7-carbonyl-6,7-dihydro-2H-pyrazolo[ 3,4-d]pyridazin-2-yl]cyclohexyl}-N,N-dimethylglycinamide (A35)**

Compound A35 was prepared by the same method as in example 33, except that acryloyl chloride was replaced by dimethylaminoacetyl chloride. MS (ESI, m/z): 478(M +H)$^+$.

**Example 36 Preparation of 4-amino-2-{1-[2-(dimethylamino)ethyl]piperidin-4-yl}-3-(3,5-dimethylbenzofuran-2-yl)-2H-pyridyl Oxazo[3,4-d]pyridazine-7(6H)-one (A36)**

**Step 1:** Preparation of 3-(3,5-dimethylbenzofuran-2-yl)-3-carbonylpropionitrile (A36a) A36a was prepared by the same method as Example A4.

Reaction formula 36.1

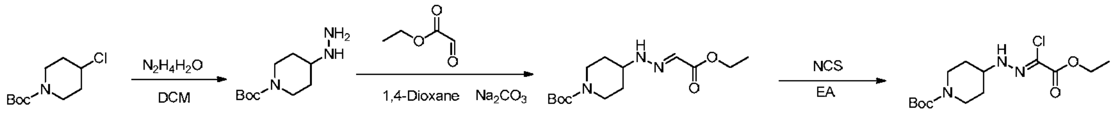


**Step 2:** Preparation of tert-butyl 4-hydrazino-piperidine-1-carboxylate (A36b)

The compound 1-BOC-4-chloropiperidine (2 g, 9.10 mmol) was added to DCM, and solution of hydrazine hydrate (0.7 mL, 14 mmol) was added at room temperature. After TLC monitored that the reaction was completed, the mixture was directly evaporated to dryness to obtain 1.8 g compound, yield 93%, and was used in the next step without purification. MS (ESI, m/z): 215(M +H)$^+$

**Step 3:** Preparation of (E)-tert-butyl 4-(2-(2-ethoxy-2-carbonylethylidene)hydrazino)piperidine-1-carboxylate (A36c)

A36b (2g, 9.29mmol) was dissolved in a 1:1 solution of 1,4-dioxane and water, and sodium carbonate (1g, 10.22mmol) was added. After stirred for 5 min, ethyl glyoxylate in toluene (2.21 mL, 10.22 mmol) was added, and the reaction was stirred at 50 °C. After TLC monitored that the reaction has been completed, the product was separated by flash column to give 2g compound, yield 65%. MS (ESI, m/z): 299(M +H)$^+$.

**Step 4:** Preparation of (Z)-tert-butyl 4-(2-(1-chloro2-ethoxy-2-carbonylethylidene)hydrazino)piperidine-1-carboxylate (A36d)

Compound A36c (2g, 6.68mmol) was dissolved in ethyl acetate, NCS (1.78, 13.36mmol) was added, and the reaction was stirred at room temperature. After TLC monitored that the reaction was completed, 50 mL of water was added and ethyl acetate (3×50 mL) was added for extraction. The combined organic layer was washed with water (3×50 mL) and saturated NaCl solution (50 mL), dried over anhydrous $Na_2SO_4$ and filtered. The solvent was removed under reduced pressure to directly give the product 2g, yield 76%.
MS (ESI, m/z): 333(M +H)$^+$.

Reaction formula 36.2

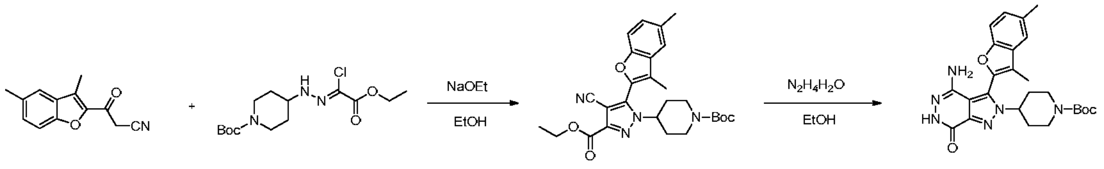


**Step 5:** Preparation of tert-butyl 4-(4-cyano-5-(3,5-dimethylbenzofuran-2-yl)-3-(carbethoxy <ethoxycarbonyl>)-1H-pyrazol-1 -yl)piperidine-1 -carboxylate (A36e)

Under argon atmosphere, A36a (700 mg, 3.25 mmol) was added to 10 mL of anhydrous EtOH, NaOEt (0.42 g, 6.6 mmol) was added, and stirred for 10 min, and then A36d (1.1 g, 3.28 mmol) was added to react under room temperature. After TLC monitored that the reaction was completed, 20 mL of water was added, and ethyl acetate (3×30 mL) was added for extraction. The combined organic layer was washed with water (3×20 mL) and saturated NaCl solution (50 mL), dried over anhydrous $Na_2SO_4$ and filtered. The solvent was removed under reduced pressure. The residue was isolated and purified by flash column chromatography (petroleum ether / ethyl acetate = 4/1, v / v) to give 680 mg of the mixture product of tautomer, yield 50.9%. MS(ESI, m/z): 492(M +H)$^+$.

**Step 6:** Preparation of tert-butyl 4-(4-amino-3-(3,5-dimethylbenzofuran-2-yl)-7-carbonyl-6,7-dihydro-2H-pyrazole[3,4-d]pyri dazin-2-yl)piperidine-1-carboxylate(A36f)

The above mixture of A36e (600 mg, 1.55 mmol) was dissolved in 5 mL of anhydrous ethanol, and then hydrazine hydrate (543 μl, 10.84 mmol) was added and refluxed at 90 °C overnight. After TLC monitored that the reaction was completed, the solvent was evaporated under reduced pressure and the residue was purified by flash column chromatography (dichloromethane/methanol = 15/1, v/v) to provide 400 mg of product (yield: 69.2%). MS (ESI, m/z): 478(M +H)$^+$.

Reaction formula 36.3

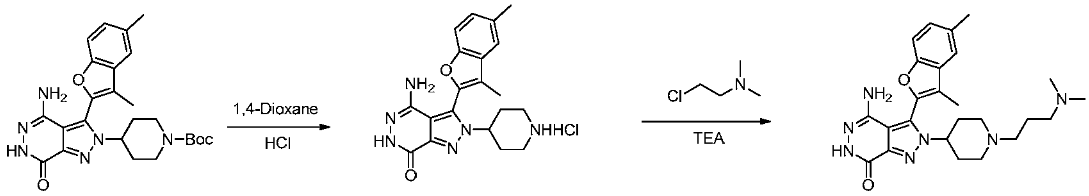


**Step 7:** Preparation of 4-amino-3-(3,5-dimethylbenzofuran-2-yl)-2-(piperidin-4-yl)-2H-pyrazolo[3,4-d]pyridazine-7 (6H)-one hydrochloride (A36g)

The above A36f (500 mg, 1.0 mmol) was added to 20 mL of 4N hydrochloric acid in 1,4-dioxane, and stirred at room temperature for 3 hours, TCL monitored that the reaction was completed, and the mixture was directly concentrated to dryness under reduced pressure to give 0.4 g product, yield 98%. MS (ESI, m/z): 435(M +H)$^+$.

**Step** 8: Preparation of 4-amino-2-(1-(2-(2-dimethylamino)ethyl)piperidin-4-yl)-3-(3,5-dimethylbenzofuran-2-yl)-2 H-pyrazolo[3,4-d]pyridazin-7(6H)-one (A36)

The above A36g (0.5 g, 1.32 mmol) was added to 20 mL of DCM, 0.36 mm of TEA was added, and stirred at room temperature for 10 min. 2-chloro-N,N-dimethylethylamine (142 mg, 1.32 mmol) was added to the reaction mixture, and stirred at room temperature for 2 hours. After TLC monitored that the reaction was completed, 20 mL of water was added and DCM (3×30 mL) was added for extraction. The combined organic layer was washed with water (3×20 mL) and saturated NaCl solution (50 mL), dried over anhydrous $Na_2SO_4$ and filtered. The solvent was removed under reduced pressure. The residue was isolated and purified by flash column chromatography (dichloromethane / ethyl acetate = 4/1, v / v) to obtain the product 300mg, yield 52.2%. MS (ESI, m/z): 435(M +H)$^+$.

**Example 37 Preparation of 4-amino-3-(3,5-dimethyl-1-benzofuran-2-yl)-2-[(1-ethylpiperidin-4-yl)methyl]-2,6-dihyd ro-7H-pyrazolo[3,4-d]pyridazin-7-one (A37)**

Compound 37 was prepared by the same method as in Example 36, except that 1-BOC-4-chloropiperidine was replaced by 4-(chloromethyl)-1-ethylpiperidine. The yield of the last step was 60.7%. MS (ESI, m/z): 421(M +H)$^+$.

**Example 38 Preparation of 2-[(1-acryloylpiperidin-4-yl)methyl]-4-amino-3-(3,5-dimethyl-1-benzofuran-2-yl)-2,6-dih ydro-7H-pyrazolo[3,4-d]pyridazin-7-one (A29)**

Compound 38 was prepared by the same method as in Example 36, except that 1-BOC-4-chloropiperidine was replaced by 1-BOC-4-(chloromethyl)piperidine and 2-chloro-N,N-dimethylethylamine was replaced by acryloyl chloride. The yield of the last step was 40.7%. MS (ESI, m/z): 447(M +H)$^+$.

**Example 39 Preparation of 2-[(1-acryloylpiperidin-4-yl)methyl]-4-amino-3-(3,5-dimethyl-1-benzofuran-2-yl)-2,6-dih ydro-7H-pyrazolo[3,4-d]pyridazin-7-one (A29)**

Compound 39 was prepared by the same method as in Example 36, except that 1-BOC-4-chloropiperidine was replaced by 1-BOC-2-(chloromethyl)pyrrolidine and 2-chloro-N,N-dimethylethylamine was replaced by acryloyl chloride. MS (ESI, m/z): 433(M +H)$^+$

**Example 40 Preparation of 2-(1-acryloylpiperidin-3-yl)-4-amino-3-(3,5-dimethyl-1-benzofuran-2-yl)-2,6-dihydro-7H -pyrazolo[3,4-d]pyridazin-7-one (A40)**

Compound was prepared by the same method as in Example 36, except that 1-BOC-4-chloropiperidine was replaced by 1-BOC-3-chloropyrrolidine and 2-chloro-N,N-dimethylethylamine was replaced by acryloyl chloride. MS (ESI, m/z): 433(M +H)$^+$.

**Example 41 Preparation of 4-amino-3-(5-chloro-3-methyl-1-benzofuran-2-yl)-2-(1-propionylpiperidin-4-yl)-2,6-dihy dro-7H-pyrazolo[3,4-d]pyridazin-7-one (A41)**

Compound A41 was prepared by the same method as in Example 36, except that 2-chloro-N,N-dimethylethylamine was replaced by propionyl chloride, and ethyl 3,5-dimethyl-1-benzothiophene-2-carboxylate was replaced by 5-chloro-3-methyl-1-benzofuran-2-carboxylate. MS (ESI, m/z): 455(M +H)$^+$.

**Example 42 Preparation of 4-amino-3-(3,5-dimethyl-1-benzofuran-2-yl)-2-(tetrahydrofuran-2H-pyran-4-yl)-2,6-dih ydro-7H-pyrazolo[3,4-d]pyridazin-7-one (A42)**

Compound was prepared by the same method as in example 4, except that aniline was replaced by 4-aminotetrahydro-2H-pyran. MS (ESI, m/z): 380(M +H)$^+$.

**Example 43 Preparation of 2-[(1-acryloylpiperidin-3-yl)methyl]-4-amino-3-(3,5-dimethyl-1-benzofuran-2-yl)-2,6-dih ydro-7H-pyrazolo[3,4-d]pyridazin-7-one (A43)**

Compound was prepared by the same method as in Example 36, except that 1-BOC-4-chloropiperidine was replaced by 1-BOC-3-(chloromethyl)pyrrolidine and 2-chloro-N,N-dimethylethylamine was replaced by acryloyl chloride. MS (ESI, m/z): 433(M +H)$^+$.

**Example 44 Preparation of 4-amino-3-(3,5-dimethyl-1-benzofuran-2-yl)-2-{4-[(5-carbonylpyrrolidin-3-yl)amino]phe nyl}-2,6-dihydro-7H-pyrazolo[3,4-d]pyridazin-7-one (A14)**

The compound was prepared by the same method as in Example 1, except that aniline was replaced with 4-((4-aminophenyl)amino)pyrrolidin-2-one and 3-(3-methyl-1-benzothiophen-2-yl)-3-carbonylpropionitrile was replaced by 3-(3,5-dimethylbenzofuran-2-yl)-3-carbonylpropionitrile. MS (ESI, m/z): 470(M +H)$^+$.

**Example 45 Preparation of 2-[(1-acryloylazetidine-3-yl)methyl]-4-amino-3-(3,5-dimethyl-1-benzofuran-2-yl)-2,6-dih ydro-7H-pyrazolo[3,4-d]pyridazin-7-one (A45)**

Compound was prepared by the same method as in Example 29, except that 1-BOC-4-chloropiperidine was replaced by1-BOC-3-(chloromethyl)azetidine. MS (ESI, m/z): 419(M +H)$^+$.

**Example 46 Preparation of 4-amino-3-(5-chloro-3-methyl-1-benzofuran-2-yl)-2-{3-[2-(dimethyl)ethyl]azetidine-1-yl 1-2,6-dihydro-7H-pyrazolo[3,4-d]pyridazin-7-one (A23)**

Compound was prepared by the same method as in example 36, except that ethyl 3,5-dimethyl-1-benzofuran-2-carboxylate was replaced by ethyl 5-chloro-3-methyl-1-benzofuran-2-carboxylate, and 1-BOC-4-chloropiperidine was replaced by 1-BOC-3-chloroazetidine. MS (ESI, m/z): 441(M +H)$^+$.

**Example** 47 **Preparation of 4-amino-3-(3,5-dimethyl-1-benzofuran-2-yl)-2-{1-[(4-methylpiperazin-1-yl)carbonyl] pyr rolidin-3-yl}-2,6-dihydro-7H-pyrazolo[3,4-d]pyridazin-7-one (A47)**

Compound was prepared by the same method as in Example 36, except that 0 was replaced by 1-BOC-3-chloropyrrolidine and 2-chloro-N,N-dimethylethylamine was replaced by 4-methyl-piperazine-1-carbonyl chloride. MS (ESI, m/z): 491(M +H)$^+$.

**Example 48 Preparation of 4-amino-3-(3,5-dimethyl-1-benzofuran-2-yl)-2-{1-[(1,1-dioxythiomorpholin-4-yl)carbony l]pyrrolidin-3-yl}-2,6-dihydro-7H-pyrazolo[3,4-d]pyridazin-7-one (A48)**

The compound was prepared by the same method as in Example 47, except that 4-methylpiperazine-1-carbonyl chloride was replaced with 1,1-dithiomorpholine-4-carbonyl chloride. The yield of the last step was 61.5%. MS (ESI, m/z): 526(M +H)$^+$.

**Example 49 Preparation of 4-amino-2-{1-[(dimethylamino)acetyl]pyrrolidin-3-yl}-3-(3,5-dimethyl-1-benzofuran-2-y l)-2,6-dihydro-7H-pyrazolo[3,4-d]pyridazin-7-one (A49)**

Compound was prepared by the same method as in example 47, except that 4-methylpiperazine-1-carbonyl chloride was replaced by dimethylaminoacetyl chloride. MS (ESI, m/z): 450(M +H)$^+$.

**Example 50 Preparation of 4-amino-2-{[N-cyclohexyl-(2E)-4-methoxybut-2-enamide]-4-yl}-3-(3,5-dimethyl-1-benzof uran-2-yl)-2,6-dihydro-7H-pyrazolo[3,4-d]pyridazin-7-one (A50)**

The compound was prepared by the same method as in Example 33, except that acryloyl chloride was replaced by (2E)-4-methoxybut-2-enoyl chloride. The yield of the last step was 65.5%. MS (ESI, m/z): 491(M +H)$^+$.

**Example 51 Preparation of 2-(1-acryloylpyrrolidin-3-yl)-4-amino-3-(3,5-dimethyl-1-benzofuran-2-yl)-2,6-dihydro-7 H-pyrazolo[3,4-d]pyridazin-7-one (A51)**

The compound was prepared by the same method as in Example 47, except that 4-methylpiperazine-1-carbonyl chloride was replaced by acryloyl chloride. The yield of the last step was 42.5%. MS (ESI, m/z): 419(M +H)$^+$.

**Example 52 Preparation of 4-amino-3-(3,5-dimethyl-1-benzofuran-2-yl)-2-[1-(2-fluoroacryloyl)pyrrolidin-3-yl]-2,6-dihydro-7H-pyrazolo [3,4-d]pyridazin-7-one (A52)**

The compound was prepared by the same method as in Example 47, except that 4-methylpiperazine-1-carbonyl chloride was replaced by 2-fluoroacryloyl chloride. The yield of the last step was 41.5%. MS (ESI, m/z): 437(M +H)$^+$.

**Example 53 Preparation of 4-amino-3-(3,5-dimethyl-1-benzofuran-2-yl)-2-{1- [2-(pyrrolidin-1-ylmethyl)acryloyl] pyr rolidin-3-yl}-2,6-dihydro-7H-pyrazolo[3,4-d]pyridazin-7-one (A53)**

The compound was prepared by the same method as in Example 47, except that 4-methylpiperazine-1-carbonyl chloride was replaced by 2-(pyrrolidin-1-ylmethyl)acryloyl chloride. The yield of the last step was 34.5%. MS (ESI, m/z): 502(M +H)$^+$.

**Example 54 Preparation of 4-amino-3-(3,5-dimethyl-1-benzofuran-2-yl)-2-[1-(2-fluoroacryloyl)piperidin-4-yl]-2,6-di hydro-7H-pyrazolo[3,4-d]pyridazin-7-one (A54)**

The compound was prepared by the same method as in Example 36, except that 2-chloro-N,N-dimethylethylamine was replaced by 2-fluoroprop-2-enoyl chloride. The yield of the last step was 68.5%. MS (ESI, m/z): 451(M +H)$^+$.

**Example 55 Preparation of 2-({4-[4-amino-3-(3,5-dimethyl-1-benzofuran-2-yl)-7-carbonyl-6,7-dihydro-2H-pyrazolo [3,4-d]pyridazin-2-yl]piperidin-1-yl}carbonyl)prop-2-enenitrile (A55)**

Compound A29 was prepared by the same method as in Example 36, except that2-chloro-N,N-dimethylethylamine was replaced by 2-cyanoprop-2-enoyl chloride. The yield of the last step was 23.5%. MS (ESI, m/z): 458(M +H)$^+$.

**Example 56 Preparation of 4-amino-3-(5-chloro-3-methyl-1-benzofuran-2-yl)-2-{1-[(2E)-4-(cyclopropylamino)butyl-2-enoyl]piperidin-4-yl}-2,6-dihydro-7H-pyrazolo[3,4-d]pyridazin-7-one (A56)**

Compound was prepared by the same method as in Example 36, except that 2-chloro-N,N-dimethylethylamine was replaced by (E)-4-(cyclopropylamino)but-2-enoyl chloride, and ethyl 3,5-dimethyl-1-benzofuran-2-carboxylate was replaced with ethyl 5-chloro-3-methyl-1-benzofuran-2-carboxylate. The yield of the last step was 43.5%. MS (ESI, m/z): 522(M +H)$^+$.

**Example 57 Preparation of 4-amino-3-(5-chloro-3-methyl-1-benzofuran-2-yl)-2-{1-[(2E)-4-(pyrrolidin-1-yl)butyl-2-e noyl]piperidin-4-yl}-2,6-dihydro-7H-pyrazolo[3,4-d]pyridazin-7-one** (A57)

Compound was prepared by the same method as in Example 36, except that 2-chloro-N,N-dimethylethylamine was replaced by (E)-4-(pyrrolidin-1-yl)but-2-enoyl chloride, and ethyl 3,5-dimethyl-1-benzothiophene-2-carboxylate was replaced by 5-chloro-3-methyl-1-benzofuran-2-carboxylate. MS (ESI, m/z): 536(M +H)$^+$.

**Example 58 Preparation of N-{4-[4-amino-3-(5-chloro-3-methyl-1-benzofuran-2-yl)-7-carbonyl-6,7-dihydro-2H-pyr azolo[3,4-d]pyridazin-2-yl]cyclohexyl}-2-fluoroprop-2-enamide** (A58)

Compound was prepared by the same method as in Example 33, except that 2-fluoroprop-2-enoyl chloride was replaced by acryloyl chloride, and ethyl 3,5-methyl-1-benzothiophene-2-carboxylate was replaced by ethyl 5-chloro-3-methyl-1-benzofuran-2-carboxylate. The yield of the last step was 34.7%. MS (ESI, m/z): 485(M +H)$^+$.

**Example 59 Preparation of N-{4-[4-amino-3-(3,5-dimethyl-1-benzofuran-2-yl)-7-carbonyl-6,7-dihydro-2H-pyrazolo[ 3,4-d]pyridazin-2-ylcyclohexyl}-2-cyanoprop-2-enamide (A59)**

The compound was prepared by the same method as in Example 33, except that acryloyl chloride was replaced by 2-cyanoprop-2-yl chloride. The yield of the last step was 61.5%. MS (ESI, m/z): 472(M +H)$^+$.

**Example 60 Preparation of (2E)-N-{4-[4-amino-3-(3,5-dimethyl-1-benzofuran-2-yl)-7-carbonyl-6,7-dihydro-2H-pyra zolo[3,4-d]pyridazin-2-yl]cyclohexyl}-4-(4-methylpiperazin-1-yl)but-2-enamide** (A60)

The compound was prepared by the same method as in Example 33, except that acryloyl chloride was replaced by (2E)-4-(4-methylpiperazin-1-yl)but-2-enoyl chloride. The yield of the last step was 61.5%. MS (ESI, m/z): 559(M +H)$^+$.

**Example 61 Preparation of (2Z)-N-{4-[4-amino-3-(5-chloro-3-methyl-1-benzofuran-2-yl)-7-carbonyl-6,7-dihydro-2H - pyrazolo[3,4-d]pyridazin-2-yl]cyclohexyl}-4-(dimethylamino)-2-fluorobut-2-enamide (A61)**

Compound was prepared by the same method as in Example 33, except that acryloyl chloride was replaced by (Z)-4-(dimethylamino)-2-fluorobut-2-enoyl chloride, and ethyl 3,5-methyl-1-benzothiophene-2-carboxylate was replaced by ethyl 5-chloro-3-methyl-1-benzofuran-2-carboxylate. The yield of the last step was 67.5%. MS (ESI, m/z): 542(M +H)$^+$.

**Example 62 Preparation of 4-amino-3-(5-chloro-3-methyl-1-benzofuran-2-yl)-2-[1-(prop-2-ynyl)piperidin-4-yl]-2, 6-dihydro-7H-pyrazolo [3,4-d]pyridazin-7-one (A62)**

Compound was prepared by the same method as in Example 36, except that 2-chloro-N,N-dimethylethylamine was replaced by propynyl chloride, and ethyl 3,5-dimethyl-1-benzofuran-2-carboxylate was replaced with ethyl 5-chloro-3-methyl-1-benzofuran-2-carboxylate. The yield of the last step was 74.5%. MS (ESI, m/z): 451(M+H)$^+$.

**Example 63 Preparation of 4-amino-3-(3,5-dimethyl-1-benzofuran-2-yl)-2-[1-(4-hydroxybut-2-ynyl)pyrrolidin-3-yl]-2,6-dihydro-7H-pyrazolo[3,4-d]pyridazin-7-one (A63)**

The compound was prepared by the same method as in Example 47 that 4-methylpiperazine-1-carbonyl chloride was replaced by 4-hydroxybutan-2-ynyl chloride. The yield of the last step was 67.5%. MS (ESI, m/z): 447(M +H)$^+$.

**Example 64 Preparation of N-{4-[4-amino-3-(5-chloro-3-methyl-1-benzofuran-2-yl)-7-carbonyl-6,7-dihydro-2H-pyr azolo[3,4-d]pyridazin-2-yl]cyclohexyl}but-2-ynylamide (A64)**

Compound was prepared by the same method as in Example 33, except that acryloyl chloride was replaced by but-2-ynyl chloride, and ethyl 3,5-methyl-1-benzothiophene-2-carboxylate was replaced by ethyl 5-chloro-3-methyl-1-benzofuran-2-carboxylate. The yield of the last step was 65.5%. MS (ESI, m/z): 479(M +H)$^+$.

**Example 65 Preparation of 4-amino-3-(3,5-dimethyl-1-benzofuran-2-yl)-2-(1-{(2E)-4-[(3R)-3-fluoropyrrolidine-1-yl] but-2-enoyl}piperidin-4-yl)-2,6-dihydro-7H-pyrazolo[3,4-d]pyridazin-7-one (A66)**

Compound was prepared by the same method as in Example 36, except that 2-chloro-N,N-dimethylethylamine was replaced by (R,E)-4-(3-fluoropyrrolidin-1-yl)but-2-enoyl chloride. The yield of the last step was 61.5%. MS (ESI, m/z): 534(M +H)$^+$.

**Example 66 Preparation of 4-amino-3-(3,5-dimethyl-1-benzofuran-2-yl)-2-{1-[(2E)-4-(4-hydroxypiperidin-1-yl)-but-2-enoyl]piperidin-4-yl}-2,6-dihydro-7H-pyrazolo[3,4-d]pyridazin-7-one (A66)**

The compound was prepared by the same method as in Example 36, except that 2-chloro-N,N-dimethylethylamine was replaced by (2E)-4-(4-hydroxypiperidin-1-yl)but-2-enoyl chloride. The yield of the last step was 54.5%. MS (ESI, m/z): 546(M +H)$^+$.

**Example 67 Preparation of 1-(1-acryloylpiperidin-4-yl)-4-amino-3-(3,5-dimethyl-1-benzofuran-2-yl)-1,3a,6,7a-tetra hydro-7H- pyrazolo[2,3-d]pyridazin-7-one (A67)**

Reaction formula 67.1

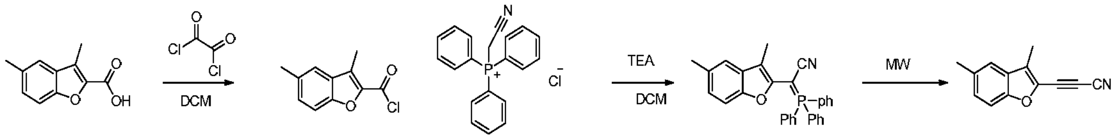


**Step 1**

3,5-Dimethylbenzofuran-2-carboxylic acid (6 g, 31.55 mmol) was dissolved in dry dichloromethane, and oxalyl chloride (4 g, 31.55 mmol) and catalytic amount of DMF were added at room temperature, and stirred under room temperature. After TLC monitored that the reaction was completed, the product was directly spin dried to give the product 3,5-dimethylbenzofuran-2-acylchloride (6.5 g, yield 98%). MS (ESI, m/z): 208$(M +H)^+$

**Step 2**

The cyanomethyltriphenylphosphonium chloride (5.8 g, 19.17 mmol) and triethylamine (1.94 g, 38.34 mmol) were added to dichloromethane, and the product of the above step (4 g, 19.17 mmol) in dichloride was added under room temperature. The mixture was stirred at room temperature, and TLC monitored that the reaction was completed. The product 2-(3,5-dimethylbenzofuran-2-yl)-2-(triphenylphosphoranylidene)acetonitrile was isolated by flash preparation (3.5 g, yield: 50%). MS (ESI, m/z): 368$(M +H)^+$

**Step 3**

The product of the above step (2 g, 4.49 mmol) was placed in a microwave tube, and the reaction was stirred at 110 °C to obtain the product 3-(3,5-dimethylbenzofuran-2-yl)propionitrile 0.8 g, yield 95%. MS (ESI, m/z): 195$(M +H)^+$

Reaction formula 67.2

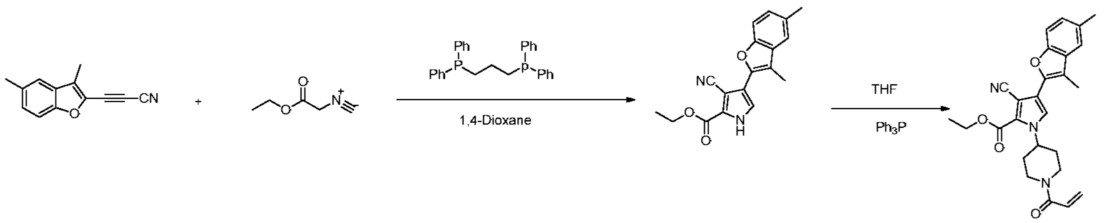


**Step 4**

The above product (2g, 10.25mmol) and ethyl isocyanate (1.16g, 10.25mmol) were dissolved in 1,4-dioxane, and a catalytic amount of 1,3-bis(diphenylphosphino)propane (0.39 g, 1.02 mmol) was added under argon protection. The reaction was stirred at 110 °C to give the product. After TLC monitored that the reaction was completed, the reaction mixture was cooled to room temperature, 50 mL of water was added, and ethyl acetate (3×50 mL) was added for extraction. The combined organic layer was then washed with water (3×50 mL), saturated NaCl solution 50 mL, dried over anhydrous $Na_2SO_4$, filtered, evaporated under reduced pressure, and purified by flash column chromatography (petroleum ether / ethyl acetate = 15/1, v/v) to provide ethyl 3-cyano-4-(3,5-dimethylbenzofuran-2-yl)-1H-pyrrole-2-carboxylate 1.2 g, yield 45.50%. MS (ESI, m/z): 308$(M +H)^+$

**Step 5**

The above product (2 g, 6.49 mmol) and 1-(4-hydroxypiperidine)prop-2-en-1-one (1.01 g, 6.49 mmol) were dissolved in THF and triphenylphosphine (0.17 g, 0.64 mmol) was added, diethyl azodicarboxylate in toluene (2.1 mL, 6.49 mmol) was added dropwise under nitrogen atmosphere, and the mixture was stirred at room temperature. After TLC monitored that the reaction was completed, 50 mL of water was added and ethyl acetate (3×50 mL) was added for extraction. The combined organic layer was then washed with water (3×50 mL), saturated NaCl solution 50 mL, dried over anhydrous $Na_2SO_4$, filtered, evaporated under reduced pressure, and purified by flash column chromatography (petroleum ether / ethyl acetate = 15/1, v/v) to provide ethyl 1 -(1 -acryloylpiperidin-4-yl)-3-cyano-4-(3,5-dimethylbenzofuran-2-yl)-1H-pyrrole-2-carboxy late 1.2 g, yield 65.50%. MS (ESI, m/z): 445$(M +H)^+$

Reaction formula 67.3

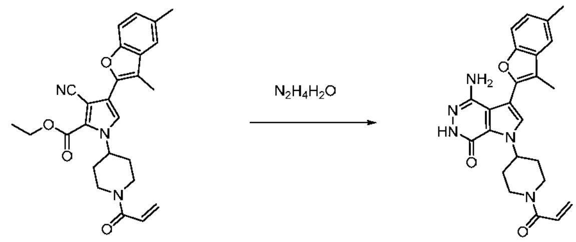


**Step 6**

The product from the previous step (1 g, 2.24 mmol) was dissolved in ethanol, and hydrazine hydrate solution (0.47 mL, 8.98 mmol) was added and heated at 60°C in an oil bath. Yield TLC monitored that the reaction was completed, and then the reaction solution was cooled to room temperature, spin-dried and directly separated by flash preparation column to gave compound A67 0.5 g, yield 50%. MS (ESI, m/z): 432(M +H)$^+$.

**Example 68 Preparation of 1-[(1-acryloylpiperidin-4-yl)methyl]-4-amino-3-(3,5-dimethyl-1-benzofuran-2-yl)-1,6-dih ydro-7H-pyrrole[2,3-d]pyridazin-7-one (A68)**

The compound was prepared by the same method as in Example 67, except that 1-(4-hydroxypiperidine)prop-2-en-1-one was replaced byl-[4-(hydroxymethyl)piperidin-1-yl]prop-2-en-1-one. The yield of the last step was 34.5%. MS (ESI, m/z): 446(M +H)$^+$.

**Example 69 Preparation of 1-[(3S)-1-acryloylpyrrolidin-3-yl]-4-amino-3-(3,5-dimethyl-1-benzofuran-2-yl)-1,6-dihyd ro-7H-pyrrolo[2,3-d]pyridazin-7-one (A69)**

The compound was prepared by the same method as in Example 67, except that 1-(4-hydroxypiperidine)prop-2-en-1-one was replaced by 1-[(3S)-3-hydroxypyrrolidin-1-yl]prop-2-en-1-one. The yield of the last step was 56.5%. MS (ESI, m/z): 418(M +H)$^+$.

**Example 70 Preparation of (2E)-N-{4-[4-amino-3-(1-methyl-1H-benzoimidazol-2-yl)-7-carbonyl-6,7-dihydro-2H-pyr azolo[3,4-d]pyridazin-2-yl]phenyl}-4-(dimethylamino)but-2-enamide (A70)**

Compound was prepared by the same method as in Example 25, except that ethyl 3,5-dimethylbenzofuran-2-carboxylate was replaced by ethyl 1-methyl-1H-benzo[d]imidazole-2-carboxylate and acryloyl chloride was replaced by (2E)-4-(dimethylamino)but-2-enoyl chloride. The yield of the last step was 56.5%. MS (ESI, m/z): 484(M +H)$^+$.

**Example 71 Preparation of (2E)-N-{4-[4-Amino-3-(1,3-dimethyl-1H-indol-2-yl)-7-carbonyl-6,7-dihydro-2H-pyrazolo [3,4-d]pyridazin-2-yl]phenyl}-4-(dimethylamino)but-2-enamide (A71)**

Compound was prepared by the same method as in Example 71, except that ethyl 1-methyl-1H-benzo[d]imidazole-2-carboxylate was replaced by ethyl 1,3-dimethyl-1H-indole-2-carboxylate. The yield of the last step was 23.5%. MS (ESI, m/z): 497(M +H)$^+$.

**Example 72 Preparation of (2E)-N-{4-[4-Amino-3-(1,3-benzothiazol-2-yl)-7-carbonyl-6,7-dihydro-2H-pyrazolo[3,4-d ]pyridazin-2-yl]phenyl}-4-(dimethylamino)but-2-enamide** (A72)

Compound was prepared by the same method as in Example 71, except that ethyl 1-methyl-1H-benzo[d]imidazole-2-carboxylate was replaced with ethylbenzo[d]thiazole-2-carboxylate. The yield of the last step was 42.5%. MS (ESI, m/z): 487(M +H)$^+$.

**Example 73 Preparation of (2E)-N-{4-[4-Amino-3-(3,5-dimethyl-1-benzofuran-2-yl)-7-carbonyl-6,7-dihydro-1H-pyr rolo[2,3-d]pyridazin-1-yl]cyclohexyl}-4-(azetidin-1-yl)but-2-enamide** (A73)

The compound was prepared by the same method as in Example 67, except that 1-(4-hydroxypiperidine)prop-2-ene-1 with (E)-4-(azetidin-1-yl)-N-(4-hydroxycyclohexyl)but-2-enamide. The yield of the last step was 51.5%. MS (ESI, m/z): 515(M +H)$^+$.

**Example 74 Preparation of 2-(1-acryloylpiperidin-4-yl)-4-amino-3-[(3,5-dimethoxyphenyl)ethynyl]-2,6-dihydro-7H-pyrazolo[3,4-d]pyridazin-7-one (A74)**

Compound was prepared by the same method as in Example 29, except that ethyl 3,5-dimethyl-1-benzofuran-2-carboxylate with ethyl 3-(3,5-dimethoxyphenyl)prop-2-ynylate. The yield of the last step was 57.5%. MS (ESI, m/z): 449(M +H)$^+$.

**Example 75 Preparation of 4-amino-2-[1-(cyclopropylcarbonyl)piperidin-4-yl]-3-(3,5-dimethyl-1-benzofuran-2-yl)-2 ,6-dihydrogen-7H-pyrazolo[3,4-d]pyridazin-7-one (A75)**

The compound was prepared in the same manner as in Example 29 except that acryloyl chloride was replaced with cyclopropionyl chloride. The yield of the next step was 56.5%. MS (ESI, m/z): 447(M +H)$^+$.

**Example 76 Preparation of N-{4-[4-amino-3-(3,5-dimethyl-1-benzofuran-2-yl)-7-carbonyl-6,7-dihydro-2H-pyrazolo[ 3,4-d]pyridazin-2-ylcyclohexyl}-2-cyanoacetamide (A76)**

The compound was prepared by the same method as in Example 33, except that acryloyl chloride was replaced by cyanoacetyl chloride. The yield of the last step was 45.5%. MS (ESI, m/z): 460(M +H)$^+$.

**Example 77 Preparation of 2-(1-acryloylpiperidin-4-yl)-4-amino-3- [(3,5-dimethoxyphenyl)ethynyl]-2,6-dihydro-7H-pyrazolo[3,4-d]pyridazin-7-one (A77)**

Compound was prepared by the same method as in Example 29, except that ethyl 3,5-dimethyl-1-benzofuran-2-carboxylate was replaced by ethyl 3-(3,5-diethoxyphenyl)prop-2-ynylate. The yield of the last step was 81.5%. MS (ESI, m/z): 473(M +H)$^+$.

**Example 78 Preparation of N-{4-[4-Amino-3-[(3,5-dimethoxyphenyl)ethynyl]-7-carbonyl-6,7-dihydro-2H-pyrazolo[ 3,4-d]pyridazin-2-yl]phenyl}prop-2-enamide (A78)**

Compound was prepared by the same method as in Example 25, except that ethyl 5-chloro-3-methyl-1-benzofuran-2-carboxylate was replaced by ethyl 3-(3,5-dimethoxyphenyl)prop-2-ynylate. The yield of the last step was 45%. MS (ESI, m/z): 457(M +H)$^+$.

**Example 79 Preparation of 2-(1-acryloylpiperidin-3-yl)-4-amino-3-(5-methoxy-3-methylbenzofuran-2-yl)-2,6-dihydr o-7H-pyrazolo[3,4-d]pyridazin-7-one (A40)**

Compound was prepared by the same method as in Example 36, except that 1-BOC-4-chloropiperidine was replaced by 1-BOC-3-chloropiperidine, and 2-chloro-N,N-dimethylethylamine was replaced by acryloyl chloride, and ethyl 3,5-dimethyl-1-benzofuran-2-carboxylate was replaced with ethyl 3-methyl-5-methoxy-1-benzofuran-2-carboxylate. MS (ESI, m/z): 449(M +H)$^+$.

**Example 80 Preparation of 2-(1-acryloylpiperidin-4-yl)-4-amino-3-(5-fluoro-3-methylbenzofuran-2-yl)-2,6-dihydro-7H-pyrazole[3,4-d]pyridazine-7-one (A80)**

Compound was prepared by the same method as in Example 36, except that 2-chloro-N,N-dimethylethylamine was replaced by acryloyl chloride, and ethyl 3,5-dimethyl-1-benzothiophene-2-carboxylate was replaced by ethyl 3-methyl-5-fluoro-1-benzofuran-2-carboxylate. MS (ESI, m/z): 437(M +H)$^+$.

**Example 81 Preparation of 2-(1-Propylpiperidin-4-yl)-4-amino-3-(3-methyl-5-(trifluoromethyl)benzofuran-2-yl)-2,6 -dihydro-7H-pyrazolo[3,4-d]pyridazin-7-one (81)**

Compound was prepared by the same method as in Example 36, except that 2-chloro-N,N-dimethylethylamine was replaced by acryloyl chloride, and ethyl 3,5-dimethyl-1-benzothiophene-2-carboxylate was replaced by ethyl 3-methyl-5-trifluoromethyl-1-benzofuran-2-carboxylate. MS (ESI, m/z): 487(M +H)$^+$.

**Example 82 Preparation of 2-(1-acryloylpiperidin-4-yl)-4-amino-3-(3,6-dimethylbenzofuran-2-yl)-2,6-dihydro-7H-p yrazole[3,4-d]pyridazine-7-one (A82)**

Compound was prepared by the same method as in Example 36, except that 2-chloro-N,N-dimethylethylamine was replaced by acryloyl chloride, and ethyl 3,5-dimethyl-1-benzofuran-2-carboxylate was replaced by ethyl 3-methyl -6-methyl-1-benzofuran-2-carboxylate. MS (ESI, m/z): 433(M +H)$^+$.

**Example 83 Preparation of 2-(1-acryloylpiperidin-4-yl)-4-amino-3-(6-fluoro-3-methylbenzofuran-2-yl)-2,6-dihydro-7H-pyrazole[3,4-d]pyridazine-7-one (A83)**

Compound was prepared by the same method as in Example 36, except that 2-chloro-N,N-dimethylethylamine was replaced by acryloyl chloride, and ethyl 3,5-dimethyl-1-benzothiophene-2-carboxylate was replaced by ethyl 3-methyl-6-fluoro -1-benzofuran-2-carboxylate. MS (ESI, m/z): 437(M +H)$^+$.

**Example 84 Preparation of 2-(1-acryloylpiperidin-4-yl)-4-amino-3-(6-chloro -3-methylbenzofuran-2-yl)-2,6-dihydro-7H-pyrazole[3,4-d]pyridazine-7-one (A84)**

Compound was prepared by the same method as in Example 36, except that 2-chloro-N,N-dimethylethylamine was replaced by acryloyl chloride, and ethyl 3,5-dimethyl-1-benzofuran-2-carboxylate was replaced by ethyl 3-methyl-6-chloro -1-benzofuran-2-carboxylate. MS (ESI, m/z): 453(M +H)$^+$.

**Example 85 Preparation of 2-(1-acryloylpiperidin-4-yl)-4-amino-3-ethyl-5-methylbenzofuran-2-yl)-2,6-dihydro-7H-pyrazole[3,4-d]pyridazine-7-one (A85)**

Compound was prepared by the same method as in Example 36, except that 2-chloro-N,N-dimethylethylamine was replaced by acryloyl chloride, and ethyl 3,5-dimethyl-1-benzothiophene-2-carboxylate was replaced by ethyl 3-ethyl-5-methyl-1-benzofuran-2-carboxylate. MS (ESI, m/z): 447(M +H)$^+$.

**Example 86 Preparation of 2-(1-acryloylpiperidin-4-yl)-4-amino-3-(5-chloro -3-methylbenzofuran-2-yl)-2,6-dihydro-7H-pyrazole[3,4-d]pyridazine-7-one (A86)**

Compound was prepared by the same method as in Example 36, except that 2-chloro-N,N-dimethylethylamine was replaced by acryloyl chloride, and ethyl 3,5-dimethyl-1-benzothiophene-2-carboxylate was replaced by ethyl 3-methyl-5-chloro -1-benzofuran-2-carboxylate. MS (ESI, m/z): 453(M +H)$^+$.

**Example 87 Preparation of 2-(1-acryloylpiperidin-3-yl)-4-amino-3-(5-chloro -3-methylbenzofuran-2-yl)-2,6-dihydro-7H-pyrazole[3,4-d]pyridazine-7-one (A87)**

Compound was prepared by the same method as in Example 36, except that 1-BOC-4-chloropiperidine was replaced by 1-BOC-3-chloropiperidine, 2-chloro-N,N-dimethylethylamine was replaced by acryloyl chloride, and ethyl 3,5-dimethyl-1-benzofuran-2-carboxylate was replaced by ethyl 3-methyl-5-chloro-1-benzofuran-2-carboxylate. MS (ESI, m/z): 453(M +H)$^+$.

**Example 88 Preparation of N-(4-(4-amino-3-(3,5-dimethylbenzofuran-2-yl)-7-carbonyl-6,7-dihydro-2H-pyrazolo[3,4 -d]pyridazin-2-yl]cyclohexyl)acrylamide (A88)**

Compound was prepared by the same method as in Example 36, except that 1-BOC-4-chloropiperidine was replaced by N-BOC-4-chlorocyclohexylamine and 2-chloro-N,N-dimethylethylamine was replaced by acryloyl chloride. MS (ESI, m/z): 447(M +H)$^+$.

**Example 89 Preparation of (E)-2-(1-but-2-enoylpiperidin-3-yl)-4-amino-3-(5-methyl-3-methylbenzofuran-2-yl)-2,6-dihydro-7H-pyrazole[3,4-d]pyridazine-7-one (A89)**

Compound was prepared by the same method as in example 40, except that acryloyl chloride was replaced by but-2-enoyl chloride. MS (ESI, m/z): 447.0(M +H)$^+$.

**Example 90 Preparation of (*E*)-2-(1-pent-2-enoylpiperidin-3-yl)-4-amino-3-(5-methyl-3-methylbenzofuran-2-yl)-2,6-dihydro-7H-pyrazolo[3,4-d]pyridazin-7-one (A90)**

Compound was prepared by the same method as in example 40, except that acryloyl chloride was replaced by pent-2-enoyl chloride. MS (ESI, m/z): 461.1$(M + H)^+$.

**Example 91 Preparation of 4-amino-3-(5-methyl-3-methyl-1-benzofuran-2-yl)-2-{1-[(2E)-4-(N,N-dimethyl)butyl-2-e noyl]piperidin-3-yl}-2,6-dihydro-7H-pyrazolo[3,4-d]pyridazin-7-one (A91)**

Compound was prepared by the same method as in example 40, except that acryloyl chloride was replaced by (E)-4-(dimethylamino)but-2-enoyl chloride. MS (ESI, m/z): 490.0$(M + H)^+$.

**Example 92 Preparation of 4-amino-3-(5-methyl-3-methyl-1-benzofuran-2-yl)-2-{1-[(2E)-4-(pyrrolidin-1-yl)butyl-2-enoyl]piperidin-3-yl]-2,6-dihydro-7H-pyrazolo[3,4-d]pyridazin-7-one (A92)**

Compound was prepared by the same method as in example 40, except that acryloyl chloride was replaced by (E)-4-(pyrrolidin-1-yl)but-2-enoyl. MS (ESI, m/z): 516.0$(M + H)^+$.

**Pharmacological experiment**

The present invention also conducted pharmacological experiments on the inhibitory activity of five-membered heterocyclic [3,4-d]pyridazinone compounds against FGFR kinase, FGFR-dependent tumor cell proliferation, and the growth inhibition of FGFR-dependent subcutaneous transplantation tumors in nude mice. The experimental materials required for pharmacological experiments are commercially available unless otherwise stated.

**I. FGFR kinase inhibitory activity experiment**

1. experimental material

Enzyme reaction substrate poly-glutamic acid-tyrosine peptide Poly$(Glu, Tyr)_{4:1}$ (Sigma, Cat. No. P7244), anti-phosphotyrosine monoclonal antibody PY99 (Santa Cruz, Cat. No. SC-7020), kinase recombinant protein FGFR1 (Millipore, Cat. No. 14-482), horseradish peroxidase-labeled Goat Anti-Mouse IgG Antibody, H&L Chain Specific Peroxidase Conjugate (Calbiochem, Cat. No. 401215). Precision electronic balance (Sartotius, Cat. No. BP210D), adjustable wavelength microplate reader (Molecular Device, Cat. No. SpectraMax 190).

2. Experimental method

Enzyme reaction substrate μPoly$(Glu,Tyr)_{4:1}$ was diluted with PBS without potassium ion (10 mM sodium phosphate buffer, 150 mM NaCl, pH7.2-7.4) to 20μg/mL, used to coat enzyme plate at 125 μL / well, and incubated at 37°C for 12-16 hours. After the liquid was removed from the wells, the plate was washed for three times with 200 μL / well of T-PBS (PBS containing 0.1% Tween-20) for 5 minutes each time. The enzyme plate was dried at 37 °C in a dryer for 1-2 hours.

50μL ATP solution diluted with reaction buffer (50 mM HEPES pH 7.4, 50 mM $MgCl_2$, 0.5 mM $MnCl_2$, 0.2 mM $Na_3VO_4$, 1 mM DTT) was added into each well at a 5μM final concentration. The compounds were diluted to appropriate concentration in DMSO, 1 μL / well or containing the corresponding concentrations of DMSO (negative control wells). The reaction was initiated by addition of FGFR kinase domain recombinant protein diluted with 49 μL of reaction buffer. Two control wells without enzyme were set in each experiment. The plate was placed on a shaker (100rpm) at 37 °C for 1 hour. The plate was washed with T-PBS for three times. 100 μL / well of primary antibody PY99 dilution was added, and incubated at 37°C for 0.5 hour on a shaker (100rpm). The plate was washed with T-PBS for three times. 100 μL / well of horseradish peroxidase-labeled secondary anti-goat anti-mouse IgG dilution was added and incubated at 37°C for 0.5 hour on a shaker (100rpm). The plate was washed with T-PBS for three times. 100 μL / well of 2 mg / mL OPD coloration solution (diluted with 0.1 M citric acid-sodium citrate buffer containing 0.03% $H_2O_2$ (pH = 5.4)) was added, and incubated in darkness for 1-10 minutes at 25°C. The reaction was quenched with 50 μL / well 2M $H_2SO_4$, and read at 490 nm using a tunable microplate microplate reader SPECTRA MAX 190.

The inhibition ratio of the sample was determined by the following formula:

$$\text{The inhibition ratio of the sample (\%)} = \left(1 - \frac{\text{the OD value of compound-the OD value of control well without enzyme}}{\text{the OD value of negative control well - the OD value of control well without enzyme}}\right) \times 100$$

The $IC_{50}$ values were obtained by four-parameter regression analysis using the software supplied with the microplate reader.

**II. FGFR-dependent tumor cell proliferation inhibition activity test**

1. experimental material

Human acute myeloid leukemia cell line KG1 (FGFR1 fusion protein expressed in the cytoplasm, FGFR1-dependent tumor cell line) (ATCC, Cat. No. CCL-246), cell culture medium RPMI-1640 (Corning Cellgro, No. 10-040-CVR), fetal bovine serum (Life Technologies, Cat. No. 10099-141) was purchased from the company; CCK-8 cell counting kit (DOJINDO, Cat. No. FK808). Adjustable wavelength microplate reader (Molecular Device, Cat. No. SpectraMax 190).

2. Experimental method

The inhibitory effect of the compound on KG1 proliferation was detected by CCK-8 Cell Counting Kit (Dojindo): Logarithmic growth phase cells were seeded in 96-well culture plate in appropriate density, 90ul for each well. After cultured overnight, different concentrations of drugs were added and incubated for 72h, while solvent control well was set (negative control). After the cells were treated by the compound for 72h, 10μL of CCK-8 reagent was added to each well. After incubated for 2-4 hours in a 37 □ incubator, the plate was read with SpectraMax 190 microplate reader at wavelength 450 nm.

The inhibition rate of compounds against cell proliferation is calculated by the following formula:

Inhibition rate (%) = (OD negative control well-OD administration well) / OD negative control well×100%.

The $IC_{50}$ values were obtained by four-parameter regression analysis using the software supplied with the microplate reader.

Table 1: Results of proliferation inhibition to FGFR kinase and FGFR-dependent tumor cell lines of some compounds (A ≤ 100 nM; 100 nM < B ≤ 1000 nM)

| Example No. | FGFR1 $IC_{50}$ | KG1 $IC_{50}$ |
|---|---|---|
| A4 | A | B |
| A7 | A | B |
| A8 | A | B |
| A9 | A | B |
| A10 | A | B |
| A11 | A | B |
| A13 | A | B |
| A23 | A | A |
| A24 | A | A |
| A25 | A | A |
| A26 | A | A |

(continued)

| Example No. | FGFR1 $IC_{50}$ | KG1 $IC_{50}$ |
|---|---|---|
| A27 | A | A |
| A28 | A | A |
| A29 | A | A |
| A30 | A | A |
| A31 | A | N/A |
| A32 | A | N/A |
| A33 | A | A |
| A34 | A | N/A |
| A35 | A | B |
| A36 | A | N/A |
| A38 | A | N/A |
| A39 | A | N/A |
| A40 | A | A |
| A41 | A | B |
| A42 | A | N/A |
| A43 | A | N/A |
| A45 | A | N/A |
| A51 | A | A |
| A52 | A | A |
| A54 | A | N/A |
| A55 | A | A |
| A56 | A | N/A |
| A62 | A | A |
| A79 | A | A |
| A80 | B | B |
| A81 | B | N/A |
| A82 | A | A |
| A83 | A | A |
| A84 | A | A |
| A85 | A | A |
| A86 | A | A |
| A87 | A | A |
| A88 | A | A |

It can be seen from the above that the tested compound has a good inhibitory activity against FGFR kinase, and also has good proliferation inhibitory activity against its dependent tumor cell line.

**III. Tumor growth inhibition experiments in tumor-bearing mice**

The example has testified the inhibition and efficacy of five-membered heterocyclic [3,4-d]pyridazinone on the

growth of subcutaneously transplanted tumor in human lung cancer NCI-H1581 nude mice.

1. experimental material

Test sample: Example A26, white powder, and stored at -20 °C in the dark. Before using, the compound was diluted with 5% Tween80 in normal saline to the desired concentration (light yellow suspension), and was prepared once a week.

The positive control drug: AZD4547 (batch number: Lot: S0902A) was a white powder, and stored at -20 °C in the dark. Before using, the compound was diluted with 1% Tween 80 in normal saline to the desired concentration (white suspension), and was prepared once a week.

Dosage settings: A26 dosages were set at 50 mg/kg and 10 mg/kg; positive control drug AZD4547 dosage was set at 12.5 mg/kg.

Animals: BALB/cA nude mice, male, 3-4 weeks old, produced by Shanghai Institute of Materia Medica, Chinese Academy of Sciences, quality certificate number No. 311613700000120, production license number: SCXK (Shanghai) 2013-0017. Shanghai Drug Administration License No.: SYXK (Shanghai) 2013-0049, the number of animals in each group: 12 in the solvent control group and 6 in the drug-administration group.

Cell line

Human lung cancer NCI-H1581 cell line (ATCC, Cat. No. CRL-5878). The cell strain was inoculated in the right axilla of the nude mice, and the amount of cells inoculated was $5\times10^6$/animal to form transplanted tumor, and passaged in vivo in nude mice by 1 generation before use.

2. Experimental method

The tumor tissue in the vigorous growth period was cut into 1.5 $mm^3$, and inoculated subcutaneously in the right axilla of the nude mice under aseptic conditions. The diameter of the subcutaneously transplanted in a nude mice was measured with a vernier caliper, and the animals were randomly grouped when the average volume of tumors was about 220 $mm^3$. A26 50 mg/kg and 10 mg/kg group were administered via intraperitoneal injection once a day for 13 days; positive control AZD454712.5 mg/kg group was administered orally once a day for 13 days. The solvent control group was given an equal amount of normal saline. The diameter of the transplanted tumor was measured twice a week during the entire experiment, and the body weights of the mice were weighed. The calculation formula of tumor volume (TV) is: TV=1/2×a×b2, where a and b respectively represent the length and width of the tumor. The relative tumor volume (RTV) was calculated based on the measured results and the formula was: RTV = Vt/Vo. Where Vo is the tumor volume measured before grouping and dosing (do), and Vt is the tumor volume at each measurement. The evaluation index of anti-tumor activity is relative tumor proliferation rate T/C (%), and the calculation formula is as follows: T/C (%) = $(T_{RTV} / C_{RTV}) \times 100\%$, $T_{RTV}$: treatment group RTV; $C_{RTV}$: negative control group RTV.

3. Test results

The experimental results are shown in Table 2, Figures 1, 2, and photos in Figure 3. 12.5 mg/kg of positive control AZD4547 groups were orally administered once a day for 13 days, which partially inhibited the growth of subcutaneously transplanted tumors in human lung cancer NCI-H1581 nude mice. On the 13th day, the T/C percentages obtained was 16.39%. 50 mg/kg, and 10 mg/kg A26 groups were intraperitoneally injected once a day for 13 days, which significantly inhibited the growth of subcutaneously transplanted tumors in human lung cancer NCI-H1581 nude mice. On the 13th day, the T/C percentages of each group obtained were 2.20% and 19.88%, respectively. During the experiment, the animals in each group were in good condition, with body weight gains, and there was no mice died.

Table 2. Treatment experiment of compound A26 on transplanted tumor of human lung cancer NCI-H1581 nude mice

| Group | Dosage, method of administration | | number of animal. | | body weight (g) | | TV ($mm^3$, mean±SD) | | RTV (mean±SD) | T/C (%) |
|---|---|---|---|---|---|---|---|---|---|---|
| | | | $d_0$ | $d_{13}$ | $d_0$ | $d_{13}$ | $d_0$ | $d_{13}$ | | |
| **Solvent control** | 0.2 ml/20g qd/13 | ip | 12 | 12 | 17.4 | 22.9 | 157±66 | 3133±1011 | 20.95±6.15 | |
| **AZD4547** | 12.5 mg/kg qd/13 | po | 6 | 6 | 17.3 | 19.9 | 153±50 | 517±392 | 3.43 ± 2.61 *** | 16.39 |
| **A26** | 50 mg/kg qd/13 | ip | 6 | 6 | 18.2 | 19.9 | 159±69 | 66±21 | 0.46±0.19* | 2.20 |
| | 10 mg/kg qd/13 | ip | 6 | 6 | 18.4 | 21.3 | 161±71 | 621±289 | 4.17±2.49* | 19.88 |

**t student's test vs solvent control group, *p<0.05**

It can be seen from the above that the tested compound has a good inhibitory effect on the growth of subcutaneously transplanted tumor in human lung cancer NCI-H1581 nude mice, the pharmacodynamic activity is better than that of the positive drug, and the compound has good tolerance at the tested dose.

**Industrial applicability**

The five-membered heterocyclic [3,4-d]pyrazinone compound of the present invention has low toxicity and good solubility.
The preparation method of the five-membered heterocyclic [3,4-d]pyrazinone compound of the present invention has advantages such as mild reaction conditions, abundant raw materials, easy operation and post-treatment, good corresponding selectivity, etc..
The five-membered heterocyclic [3,4-d]pyridazinone compounds and derivatives thereof of the present invention have a good inhibitory activity and excellent selectivity to FGFR kinase and proliferation of FGFR-dependent tumor cell lines.
Therefore, the compound of the present invention can be used to treat various diseases related to the FGFR enzyme activity and the abnormal expression or activity of FGFR ligands, such as cancer, tumor, etc.
All literatures mentioned in the present application are incorporated herein by reference, as though each one is individually incorporated by reference. Additionally, it should be understood that after reading the above teachings, those skilled in the art can make various changes and modifications to the present invention. These equivalents also fall within the scope defined by the appended claims.

## Claims

1. A compound of formula (I) or a pharmaceutically acceptable salt thereof:

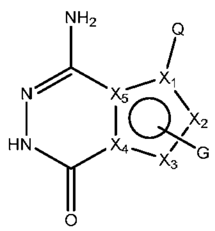

(I)

wherein,

$X_1$ is selected from CH, C, N; preferably CH or C;
$X_2$ and $X_3$ are each independently selected from CH, C, NH, N, O or S; preferably NH, N, CH or C;
$X_4$ and $X_5$ are C;
and $X_1$, $X_2$, $X_3$, $X_4$ and $X_5$ together form an aromatic five-membered ring;
Q is selected from: a substituted or unsubstituted naphthyl, substituted or unsubstituted 8-10 membered bicyclic heteroaryl;
or Q is selected from -L-A, wherein L is a substituted or unsubstituted C1-C4 alkylidene, substituted or unsubstituted C2-C4 alkenylene, C2-C4 alkynylene, C1-C4 alkylideneoxy, -(C1-C4 alkyl)-NH-, -CO-NH-, -NH-CO-; A is selected from a substituted or unsubstituted C6-C12 aryl, substituted or unsubstituted 5-8 membered cycloheteroaryl, substituted or unsubstituted 8-10 membered bicyclic heteroaryl;
G is 1-2 substituents, each of which is independently on $X_2$ and/or $X_3$ and selected from the group consisting of halogen, hydroxy, cyano, -L3-substituted or unsubstituted C6-C12 aryl, -L3-substituted or unsubstituted 5-12 membered heteroaryl, -L3-substituted or unsubstituted 3-12 membered heterocyclic group, -L3-substituted or unsubstituted C3-C8 cycloalkyl, $-(CH_2)_m$-L1 -R1, -(CH2)m-N(R2)(R3), $-(CH_2)_m-C(=O)-N(R_2)(R_4)$;
wherein, the group G may optionally have 1-3 independent $-Lx_1-Lx_2-Lx_3-Lx_4-Lx_5-M$ substituents; wherein $Lx_1$, $Lx_2$, $Lx_3$, $Lx_4$ and $Lx_5$ are each independently selected from the group consisting of: none, carbonyl (C=O), oxy (-O-), -C=S-, $-S(O)_2-$, $-CH_2-$, -CH=CH-, C3-C8 cycloalkylidene, -C≡C-, -NH-, $-N(R_5)-$; M is selected from the group consisting of H, -OH, halogen, cyano, $-N(R_2)(R_3)$, $-CH_3$, $-C(=O)CH_3$, C1-C6 alkoxy, 3-12 membered

heterocyclyl, C3-C8 cycloalkyl, 5-12 membered heteroaryl, C3-C8 cycloalkenyl;

The $-Lx_1-Lx_2-Lx_3-Lx_4-Lx_5-M$ substituent may be further substituted by one or more halogens, C1-C6 alkyl groups, C1-C6 alkoxy groups, cyano groups, hydroxyl groups, =O, $=CH_2$, vinyl ($-CH=CH_2$), $-(CH_2)_kN(R_2)(R_3)$, -(C1-C6 alkyl)-$OR_2$, 3-12 membered heterocyclic groups or C3-C8 cycloalkyl groups;

k is selected from 0, 1, 2 or 3;

m is selected from 0, 1, 2, 3 or 4;

L1 is none, O, or -C(=O)O-;

L3 is none, or a substituted or unsubstituted C1-C4 alkylidene;

$R_1$, $R_3$ and $R_4$ are each independently selected from hydrogen, halogen, substituted or unsubstituted C1-C6 alkyl, substituted or unsubstituted C3-C8 cycloalkyl, substituted or unsubstituted 3-12 membered heterocyclic group , -(C1-C6 alkyl)-N(C1-C6 alkyl)(C1-C6 alkyl), -(C1-C6 alkyl)-O-(C1-C6 alkyl), or substituted or unsubstituted phenyl;

$R_2$ is selected from hydrogen or a C1-C4 alkyl;

or $R_2$ and $R_3$ , $R_2$ and $R_4$ together with the attached nitrogen atom constitute a substituted or unsubstituted 4-7 membered heterocyclic ring containing 1 to 3 hetero atoms selected from N, O, S, and at least one hetero atom is N;

$R_5$ is selected from the group consisting of hydrogen, a C1-C4 alkyl, C3-C6 cycloalkyl, formyl, C1-C4 alkylcarbonyl, or C1-C4 alkoxycarbonyl;

the "substituted" means that one or more hydrogen atoms (preferably 1-5) on the group are substituted by groups selected from the group consisting of hydroxy, halogen, cyano, nitro, amino, carboxy, $-CH_2OH$, $-CONH_2$, a substituted or unsubstituted C1-C6 alkoxy, substituted or unsubstituted C1-C6 alkyl, substituted or unsubstituted C1-C6 alkylamino, C1-C4 alkylacyl, C1-C4 alkanesulfonyl, C1-C4 alkoxycarbonyl, C1-C4 alkanesulfonylamino, oxo (=O), $:CH_2$, C3-C6 cycloalkyl, 4-7 membered heterocyclyl, -NH(C1-C4 alkyl), -N(C1-C4 alkyl)(C1-C4 alkyl), pyrrolidinyl, piperidinyl, C3-C6 cycloalkylcarbonyl, phenyl, C2-C4 alkynyl, substituted or unsubstituted 5-10 heteroaryl; the substituents of the C1-C6 alkoxy, C1-C6 alkyl and the C1-C6 alkylamino are each independently 1 to 3 groups selected from the group consisting of: oxo, halogen, cyano, cyclopropyl, hydroxyl, amino, -N(C1-C4 alkyl)(C1-C4 alkyl); the substituents of the 5-10 membered heteroaryl group is 1-3 groups selected from the group consisting of a C1-C6 alkyl, -C1-C4 alkylene-N(C1-C4 alkyl) (C1-C4 alkyl);

the C1-C6 alkoxy, C1-C6 alkyl and C1-C6 alkylamino include a straight-chain or branched-chain group;

while the compound is not any of the following structures:

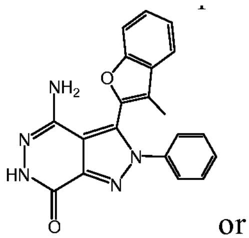

or

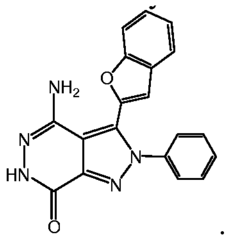

.

2. The compound of formula (I) according to claim 1, wherein Q is a substituted or unsubstituted group selected from the group consisting of: naphthyl, benzo 5-6 membered monocyclic heteroaryl, 5-6 membered monocyclic heteroaryl, and 5-6 membered monocyclic heteroaryl fused to 5-6 membered monocyclic heteroaryl.
or Q is selected from -L-A, wherein L is an unsubstituted or halogenated C1-C4 alkylidene, unsubstituted or halogenated C2-C4 alkenylene, C2-C4 alkynylene, C1-C4 alkylideneoxy, -(C1-C4 alkyl)-NH-, -CO-NH-, -NH-CO-; A is selected from a substituted or unsubstituted C6-C12 aryl, substituted or unsubstituted 5-6 membered monocyclic heteroaryl.

3. The compound of formula (I) according to claim 1, wherein G is 1-2 substituents independently selected from the group consisting of halogen, hydroxy, cyano, -L3-substituted or unsubstituted C6-C10 aryl, -L3-substituted or unsubstituted 5-10 membered heteroaryl, -L3-substituted or unsubstituted 4-7 membered heterocyclic group, -L3-substituted or unsubstituted C3-C6 cycloalkyl, $-(CH_2)_m$-L1 -R1, $-(CH_2)_m-N(R_2)(R_3)$, $-(CH_2)_m-C(=O)-N(R_2)(R_4)$;
wherein L3 is none, a substituted or unsubstituted methylidene, substituted or unsubstituted ethylidene;
and the group G may optionally have 1-3 independent $-Lx_1-Lx_2-Lx_3-Lx_4-Lx_5-M$ substituents; wherein $Lx_1$, $Lx_2$, $Lx_3$, $Lx_4$ and $Lx_5$ are each independently selected from the group consisting of: none, carbonyl (C=O), oxy (-O-), -C=S-, $-S(O)_2-$, $-CH_2-$, -CH=CH-, C3-C8 cycloalkylidene, ethynylene (-C=C-), -NH-, $-N(R_5)-$;
M is selected from the group consisting of H, -OH, halogen, cyano, $-N(R_2)(R_3)$, $-CH_3$, $-C(=O)CH_3$, C1-C4 alkoxy, 4-7 membered heterocyclyl, C3-C6 cycloalkyl, 5-10 membered heteroaryl, and C3-C6 cycloalkenyl;

the $-Lx_1-Lx_2-Lx_3-Lx_4-Lx_5-M$ substituent may be further substituted by one or more halogens, C1-C6 alkyl groups, cyano groups, hydroxy, oxygen atoms (=O), $=CH_2$, $-(CH_2)_kN(R_2)(R_3)$, $-(C1\text{-}C6\ alkyl)\text{-}OR_2$, 3-12 membered heterocyclic groups or C3-C6 cycloalkyl;

$R_1$, $R_3$ and $R_4$ are each independently selected from hydrogen, halogen, a substituted or unsubstituted C1-C4 alkyl, substituted or unsubstituted C3-C6 cycloalkyl, substituted or unsubstituted 4-7 membered heterocyclic group, or substituted or unsubstituted phenyl;

or $R_2$ and $R_3$ , $R_2$ and $R_4$ together with the attached nitrogen atom constitute a substituted or unsubstituted 4-7 saturated membered heterocyclic ring containing 1 to 3 hetero atoms selected from N, O, S, and at least one hetero atom is N;

$R_5$ is selected from the group consisting of hydrogen, C1-C4 alkyl, C3-C6 cycloalkyl, formyl, C1-C4 alkylcarbonyl, C1-C4 alkoxycarbonyl.

**4.** The compound of formula (I) according to claim 1, wherein $X_1$ is C and both of $X_2$ and $X_3$ are N; or $X_1$ is C, $X_2$ is CH and $X_3$ is N; or $X_1$ is C, $X_2$ is C and $X_3$ is N.

**5.** The compound of formula (I) according to claim 1, wherein the compound is selected from the following group:

| No. | Chemical Name | Structure |
|---|---|---|
| A1 | 4-amino-3-(3-methyl-1-benzothiophen-2-yl)-2-phenyl-2,6-dihydro-7H-pyrazolo[3,4-d]pyridazine-7-ketone | 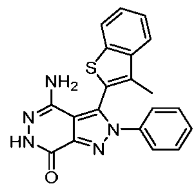 |
| A2 | 4-amino-3-(naphthalen-2-yl)-2-phenyl-2,6-dihydro-7H-pyrazolo [3,4-d]pyridazin-7-one | 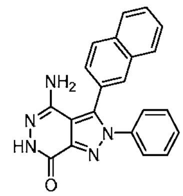 |
| A3 | 4-amino-3-(1,3-dimethyl-1H-indol-2-yl )-2-phenyl-2,6-dihydro-7H-pyrazolo[3, 4-d]pyridazine-7-one | 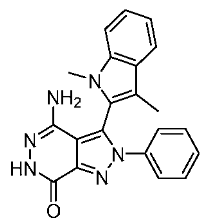 |
| A4 | 4-amino-3-(3,5-dimethyl-1-benzofuran-2-yl)-2-phenyl-2,6-dihydro-7H-pyrazolo[3,4-d]pyridazine-7-one | 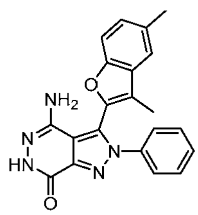 |
| A5 | 4-amino-2-phenyl-3-(quinolin-3-yl)-2,6-dihydro-7H-pyrazolo[3,4-d]pyridazin-7-one | 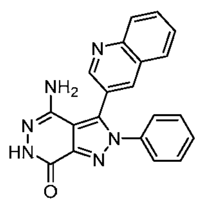 |

(continued)

| No. | Chemical Name | Structure |
|---|---|---|
| A6 | 4-amino-3-(6-chloro-3-methyl-1-benzofuran-2-yl)-2-phenyl-2,6-dihydro-7H-pyrazolo[3,4-d]pyridazin-7-one | 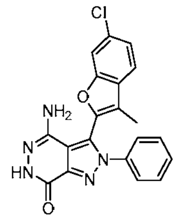 |
| A7 | 4-amino-3-(5-chloro-3-methyl-1-benzofuran-2-yl)-2-phenyl-2,6-dihydro-7H-pyrazolo[3,4-d]pyridazin-7-one | 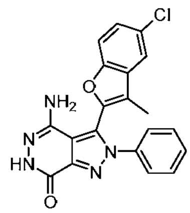 |
| A8 | 4-amino-3-(6-fluoro-3-methyl-1-benzofuran-2-yl)-2-phenyl-2,6-dihydro-7H-pyrazolo[3,4-d]pyridazin-7-one | 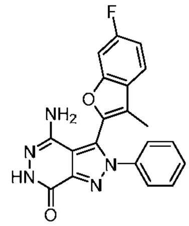 |
| A9 | 4-amino-3-(3,5-dimethyl-1-benzofuran-2-yl)-2-(4-{[(dimethylamino)methyl]amino}phenyl)-2,6-dihydro-7H-pyrazolo[3,4-d]pyridazin-7-one | 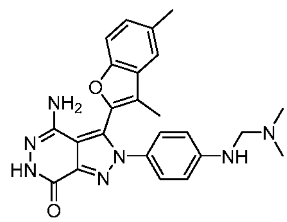 |
| A10 | 4-amino-3-(3,5-dimethyl-1-benzofuran-2-yl)-2-(1-propionylpiperidin-4-yl)-2,6-dihydro-7H-pyrazolo[3,4-d]pyridazin-7-one | 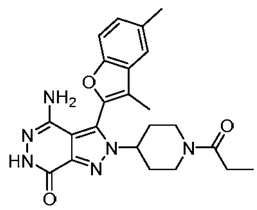 |
| A11 | 4-amino-2-(4-{[2-(dimethylamino)ethy l](methyl)amino}phenyl)-3-(3,5-dimeth yl-1-benzofuran-2-yl)-2,6-dihydro-7H-pyrazolo[3,4-d]pyridazin-7-one | 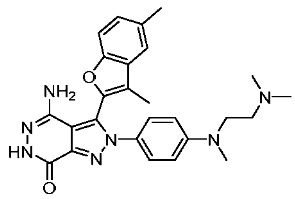 |
| A12 | 4-amino-3-(3,5-dimethyl-1-benzofuran-2-yl)-2-[4-(morpholin-4-yl)phenyl]-2,6-dihydro-7H-pyrazolo[3,4-d]pyridazin-7-one | 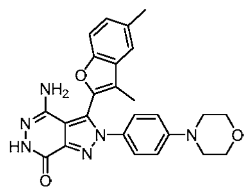 |

(continued)

| No. | Chemical Name | Structure |
|---|---|---|
| A13 | 4-amino-2-(4-chlorophenyl)-3-(3,5-dim ethyl-1-benzofuran-2-yl)-2,6-dihydro-7H-pyrazolo[3,4-d]pyridazin-7-one | 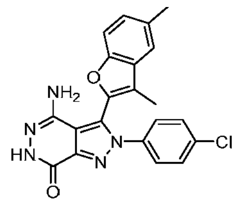 |
| A14 | 4-amino-3-(3,5-dimethyl-1-benzofuran-2-yl)-2-{4-[2-(pyrrolidin-1-yl)ethoxy]phenyl}-2,6-dihydro-7H-pyrazolo[3,4-d]pyridazin-7-one | 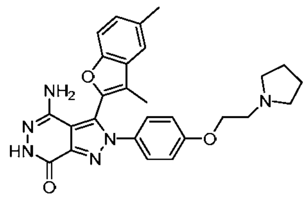 |
| A15 | 4-amino-2-{4-[2-(dimethylamino)ethoxy]phenyl}-3-(3,5-dimethyl-1-benzofuran-2-yl)-2,6-dihydro-7H-pyrazolo[3,4-d] pyridazin-7-one | 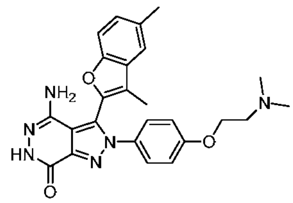 |
| A16 | 4-amino-2-[4-(4-cyclopropylpiperazin-1-yl)phenyl]-3-(3,5-dimethyl-1-benzofuran-2-yl)-2,6-dihydro-7H-pyrazolo[3, 4-d] pyridazin-7-one | 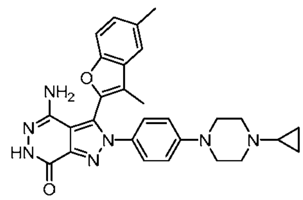 |
| A17 | 4-amino-2-{4-[3-(dimethylamino)azetidin-1-yl]phenyl}-3-(3,5-dimethyl-1-benzofuran-2-yl)-2,6-dihydro-7H-pyrazol o[3,4-d] pyridazin-7-one | 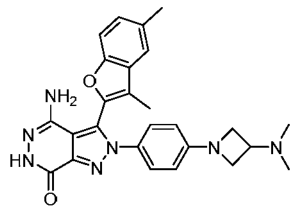 |
| A18 | 4-amino-3-(3,5-dimethyl-1-benzofuran-2-yl)-2-[4-(4-ethylpiperazin-1-yl)phenyl]-2,6-dihydro-7H-pyrazolo[3,4-d] pyridazin-7-one | 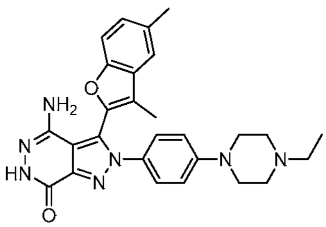 |
| A19 | 4-amino-2-(4-{[3-(dimethylamino)prop yl](methyl)amino}phenyl)-3-(3,5-dimet hyl-1-benzofuran-2-yl-2,6-dihydro-7H-pyrazolo[3,4-d]pyridazin-7-one | 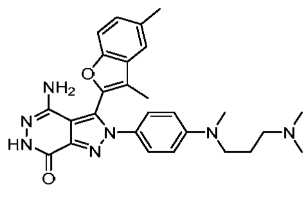 |

(continued)

| No. | Chemical Name | Structure |
|---|---|---|
| A20 | 4-amino-3-(3,5-dimethyl-1-benzofuran-2-yl)-2-(4-{[(1-methylpiperidin-4-yl)methyl]amino }phenyl)-2,6-dihydro-7H-pyrazolo[3,4-d]pyridazin-7-one | 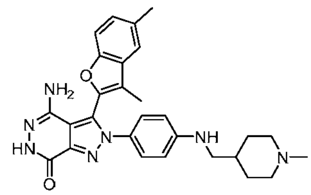 |
| A21 | 4-amino-3-(3,5-dimethyl-1-benzofuran-2-yl)-2-{4-[4-(methoxymethyl)piperidin-1-yl]phenyl}-2,6-dihydro-7H-pyrazol o[3,4-d]pyridazin-7-one | 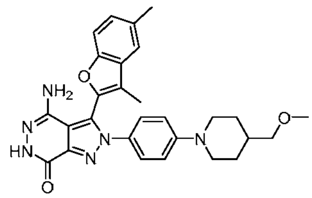 |
| A22 | 4-amino-2-{4-[3-(diethylamino)pyrroli din-1-yl]phenyl}-3-(3,5-dimethyl-1-benzofuran-2-yl)-2,6-dihydro-7H-pyrazolo[3,4-d] pyrida zin-7-one | 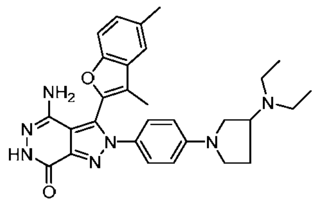 |
| A23 | 4-amino-3-(5-chloro-3-methyl-1-benzofuran-2-yl)-2-(1-ethylpiperidin-4-yl)-2, 6-dihydro-7H-pyrazolo [3,4-d]pyridazin -7-one | 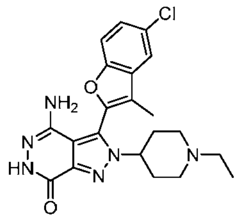 |
| A24 | 4-amino-3-(3,5-dimethyl-1-benzofuran-2-yl)-2-(1-ethylpiperidin-4-yl)-2,6-dihydro-7H-pyrazolo[3,4-d]pyridazin-7-one | 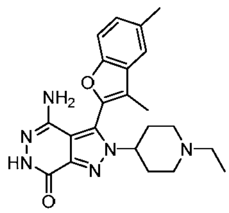 |
| A25 | N-{4-[4-amino-3-(5-chloro-3-methyl-1-benzofuran-2-yl)-7-carbonyl-6,7-dihydro-2H-pyrazolo[3,4-d]pyridazin-2-yl]p henyl} prop-2-enamide | 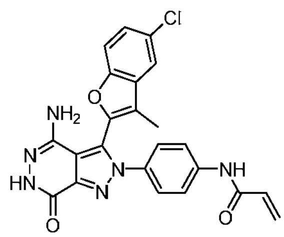 |
| A26 | (2E)-N-{4-[4-amino-3-(5-chloro-3-methyl-1-benzofuran-2-yl)-7-carbonyl-6,7-dihydro-2H-pyrazolo[3,4-d]pyridazin-2-yl]phenyl}-4-(dimethylamino)but-2-enamide | 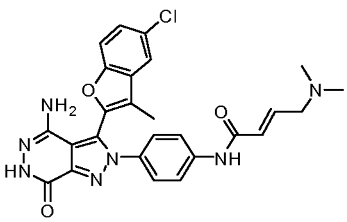 |

(continued)

| No. | Chemical Name | Structure |
|---|---|---|
| A27 | (2E)-N-{4-[4-amino-3-(3,5-dimethyl-1-benzofuran-2-yl)-7-carbonyl-6,7-dihydro-2H-pyrazolo[3,4-d]pyridazin-2-yl]phenyl}-4-(azetidin-1-yl)but-2-enamide | 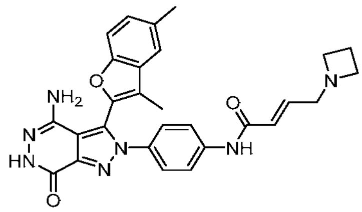 |
| A28 | (2E)-N-{4-[4-amino-3-(3,5-dimethyl-1-benzofuran-2-yl)-7-carbonyl-6,7-dihydro-2H-pyrazolo[3,4-d]pyridazin-2-yl]phenyl}-4-(morpholin-4-yl)but-2-enamide | 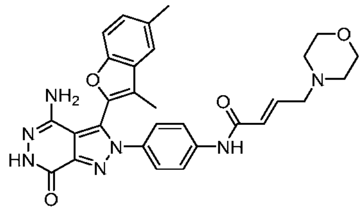 |
| A29 | 2-(1-acryloylpiperidin-4-yl)-4-amino-3-(3,5-dimethyl-1-benzofuran-2-yl)-2,6-dihydro-7H-pyrazolo[3,4-d]pyridazin-7-one | 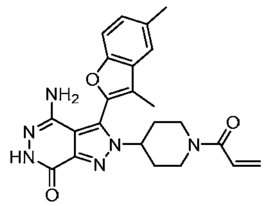 |
| A30 | 4-amino-2-{1-[(2E)-4-(dimethylamino)but-2-enoyl]piperidin-4-yl}-3-(3,5-dim ethyl-1-benzofuran-2-yl)-2,6-dihydro-7H-pyrazolo[3,4-d]pyridazin-7-one | 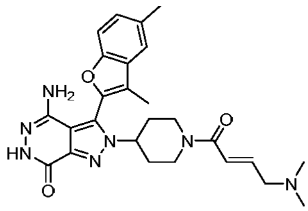 |
| A31 | 4-amino-2-{1-[(2E)-4-(azetidin-1-yl)but-2-enoyl]piperidin-4-yl}-3-(3,5-dimeth yl-1-benzofuran-2-yl)-2,6-dihydro-7H-py razolo[3,4-d]pyridazin-7-one | 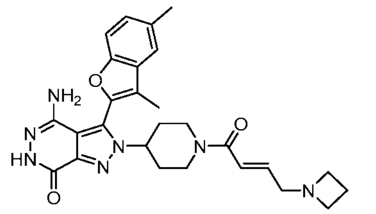 |
| A32 | N-(4-[4-amino-3-(3,5-dimethyl-1-benzofuran-2-yl)-7-carbonyl-6,7-dihydro-2H-pyrazolo[3,4-d]pyridazin-2-yl]-2-{ [(dimethylamino) ethyl](methyl)amino}phenyl)prop-2-enamide | 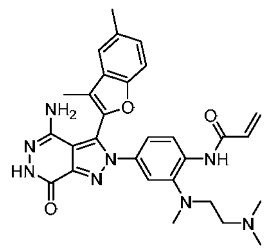 |
| A33 | N-{4-[4-amino-3-(3,5-dimethyl-1-benzofuran-2-yl)-7-carbonyl-6,7-dihydro-2H-pyrazolo[3,4-d]pyridazin-2-yl]cyclohexyl}prop-2-enamide | 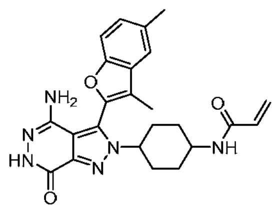 |

(continued)

| No. | Chemical Name | Structure |
|---|---|---|
| A34 | 4-amino-2-(1-{(2E)-4-[cyclopropyl(me thyl)amino]but-2-enoyl} piperidin-4-yl)-3-(3,5-dimethyl-1-benzofuran-2-yl)-2,6-dihydro-7H-pyrazolo[3,4-d]pyridazin -7-one | 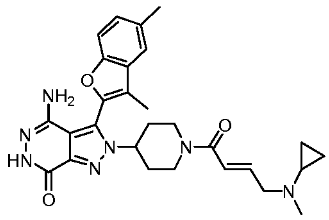 |
| A35 | N-{4-[4-amino-3-(3,5-dimethyl-1-benzofuran-2-yl)-7-carbonyl-6,7-dihydro-2H-pyrazolo[3,4-d]pyridazin-2-yl]cyclohexyl}-N,N-dimethylglycinamide | 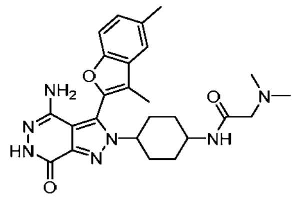 |
| A36 | 4-amino-2-{1-[2-(dimethylamino)ethyl]piperidin-4-yl}-3-(3,5-dimethylbenzofuran-2-yl)-2H-pyrazolo[3,4-d]pyridazin-7(6H)-one | 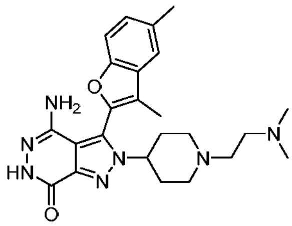 |
| A37 | 4-amino-3-(3,5-dimethyl-1-benzofuran-2-yl)-2-[(1-ethylpiperidin-4-yl)methyl]-2,6-dihydrogen-7H-pyrazolo[3,4-d]pyridazin-7-one | 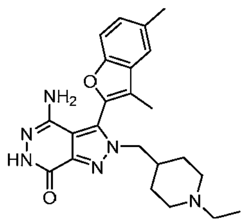 |
| A38 | 2-[(1-acryloylpiperidin-4-yl) methyl]-4-amino-3-(3,5-dimethyl-1-benzofuran-2-yl)-2,6-dihydro-7H-pyrazolo [3,4-d]pyridazin-7-one | 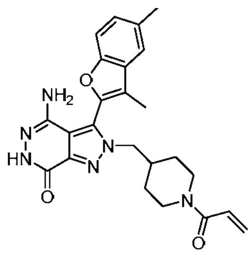 |
| A39 | 2-[(1-acryloylpiperidin-4-yl)methyl]-4-amino-3-(3,5-dimethyl-1-benzofuran-2-yl)-2,6-dihydro-7H-pyrazolo[3,4-d]pyridazin-7-one | 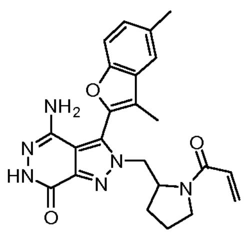 |

(continued)

| No. | Chemical Name | Structure |
|---|---|---|
| A40 | 2-(1-acryloylpiperidin-3-yl)-4-amino-3-(3,5-dimethyl-1-benzofuran-2-yl)-2,6-dihydro-7H-pyrazolo[3,4-d]pyridazin-7-one | 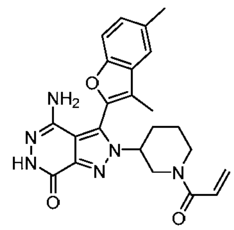 |
| A41 | 4-amino-2-{1-[2-(dimethylamino)ethyl]piperidin-4-yl}-3-(3,5-dimethylbenzofuran-2-yl)-2H-pyrazolo[3,4-d]pyridazine-7 (6H)-one | 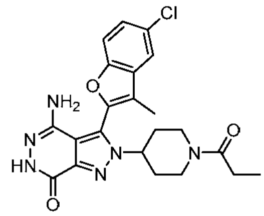 |
| A42 | 4-amino-3-(3,5-dimethyl-1-benzofuran-2-yl)-2-(tetrahydro-2H-pyran-4-yl)-2,6-dihydro-7H-pyrazolo[3,4-d]pyridazin-7-one | 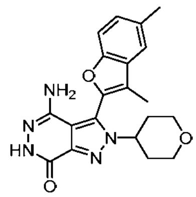 |
| A43 | 2-[(1-acryloylpiperidin-3-yl)methyl]-4-amino-3-(3,5-dimethyl-1-benzofuran-2-amino-3-(3,5-dimethyl-1-benzofuran-2-yl)-2,6-dihydro-7H-pyrazolo[3,4-d]pyridazin-7-one | 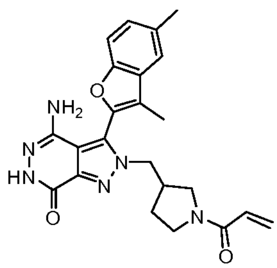 |
| A44 | 4-amino-3-(3,5-dimethyl-1-benzofuran-2-yl)-2-{4-[(5-carbonylpyrrolidin-3-yl)amino]phenyl}-2,6-dihydro-7H-pyrazolo [3,4-d]pyridazin-7-one | 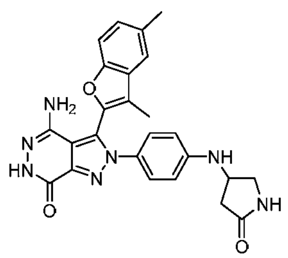 |
| A45 | 2-[(1-acryloylazetidin-3-yl)methyl]-4-amino-3-(3,5-dimethyl-1-benzofuran-2-yl)-2,6-dihydro-7H-pyrazolo[3,4-d]pyridazin-7-one | 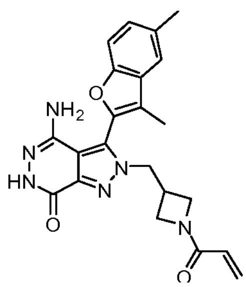 |

(continued)

| No. | Chemical Name | Structure |
| --- | --- | --- |
| A46 | 4-amino-3-(5-chloro-3-methyl-1-benzofuran-2-yl)-2-(4-{3-[2-(dimethylamino)ethyl]azetidine-1-yl}phenyl)-2,6-dihydro-7H-pyrazolo[ 3,4-d]pyridazin-7-one | 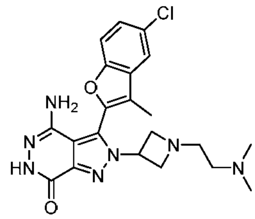 |
| A47 | 4-amino-3-(3,5-dimethyl-1-benzofuran-2-yl)-2-{1-[(4-methylpiperazin-1-yl)carbonyl]pyrrolidine-3-yl}-2,6-dihydro-7H-pyrazolo[3,4-d]pyridazin-7-one | 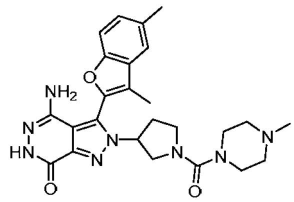 |
| A48 | 4-amino-3-(3,5-dimethyl-1-benzofuran-2-yl)-2-{1-[(1,1-dihydroxythiomorphol in-4-yl)carbonyl]pyrrolidin-3-yl}-2,6-d ihydro-7H-pyrazolo[3,4-d]pyridazin-7-one | 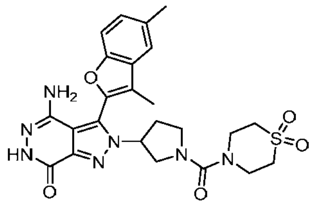 |
| A49 | 4-amino-2-{1-[(dimethylamino)acetyl] pyrrolidin-3-yl}-3-(3,5-dimethyl-1-benzofuran-2-yl)-2,6-dihydro-7H-pyrazolo[3,4-d] pyridazin-7-one | 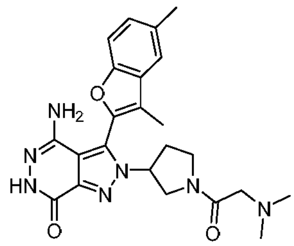 |
| A50 | 4-amino-2-{[N-cyclohexyl-(2E)-4-methoxybut-2-enamide]-4-yl}-3-(3,5-dimethyl-1-benzofuran-2-yl)-2,6-dihydro-7H-pyrazolo[3,4-d]pyridazin-7-one | 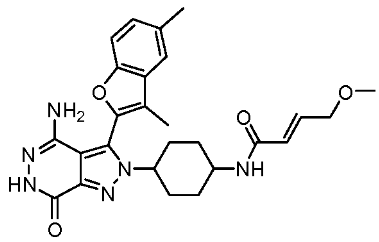 |
| A51 | 2-(1-acryloylpiperidin-3-yl)-4-amino-3-(3,5-dimethyl-1-benzofuran-2-yl)-2,6-dihydro-7H-pyrazolo[3,4-d]pyridazin-7-one | 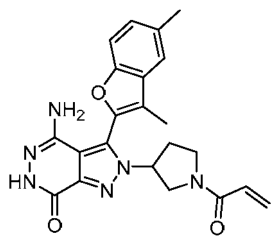 |

(continued)

| No. | Chemical Name | Structure |
|---|---|---|
| A52 | 4-amino-3-(3,5-dimethyl-1-benzofuran-2-yl)-2-[1-(2-fluoroacryloyl)pyrrolidin-3-yl]-2,6-dihydro-7H-pyrazolo[3,4-d] pyridazin-7-one | 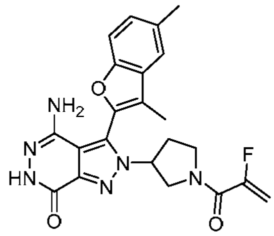 |
| A53 | 4-amino-3-(3,5-dimethyl-1-benzofuran-2-yl)-2-{1-[2-(pyrrolidin-1-ylmethyl)acryloyl]pyrrolidine-3-yl}-2,6-dihydro-7H-pyrazolo[3,4-d] pyridazin-7-one | 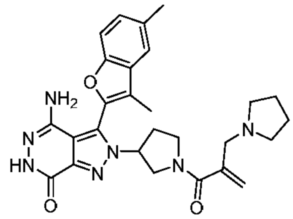 |
| A54 | 4-amino-3-(3,5-dimethyl-1-benzofuran-2-yl)-2-[1-(2-fluoroacryloyl)piperidin-4-yl]-2,6-dihydro-7H-pyrazolo[3,4-d] pyridazin-7-one | 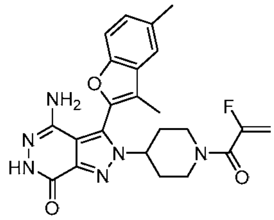 |
| A55 | 2-({4-[4-amino-3-(3,5-dimethyl-1-benzofuran-2-yl)-7-carbonyl-6,7-dihydro-2H-pyrazolo[3,4-d]pyridazin-2-yl]piperi din-1-yl} carbonyl)prop-2-enenitrile | 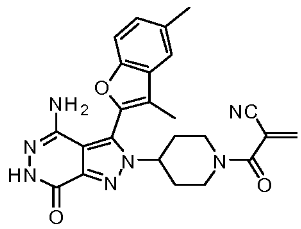 |
| A56 | 4-amino-3-(5-chloro-3-methyl-1-benzo furan-2-yl)-2-{1-[(2E)-4-(cyclopropylamino)but-2-enoyl]piperidin-4-yl}-2,6-d ihydro-7H-pyrazolo[3,4-d]pyridazin-7-one | 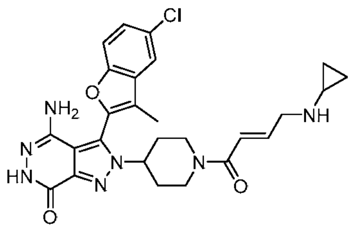 |
| A57 | 4-amino-3-(5-chloro-3-methyl-1-benzo furan-2-yl)-2-{1-[(2E)-4-(pyrrolidin-1-yl)butyl-2-enoyl]piperidin-4-yl}-2,6-di hydro-7H-pyrazolo[3,4-d]pyridazin-7-one | 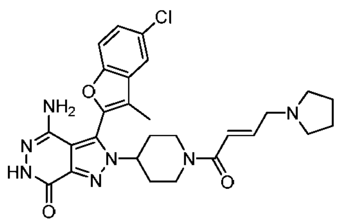 |

(continued)

| No. | Chemical Name | Structure |
| --- | --- | --- |
| A58 | N-{4-[4-Amino-3-(5-chloro-3-methyl-1-benzofuran-2-yl)-7-carbonyl-6,7-dihydro-2H-pyrazolo[3,4-d]pyridazin-2-yl]cyclohexyl}-2-fluoroprop-2-enamide | 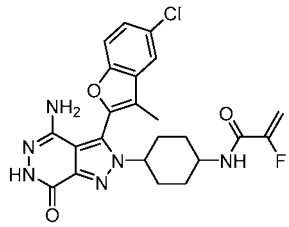 |
| A59 | N-{4-[4-amino-3-(3,5-dimethyl-1-benzofuran-2-yl)-7-carbonyl-6,7-dihydro-2H-pyrazolo[3,4-d]pyridazin-2-yl]cyclohexyl}-2-cyanoprop-2-enamide | 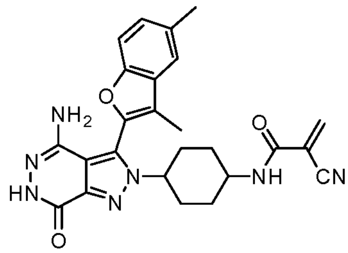 |
| A60 | (2E)-N-{4-[4-amino-3-(3,5-dimethyl-1-benzofuran-2-yl)-7-carbonyl-6,7-dihydro-2H-pyrazolo[3,4-d]pyridazin-2-yl] cyclohexyl}-4-(4-methylpiperazin-1-yl)but-2-enamide | 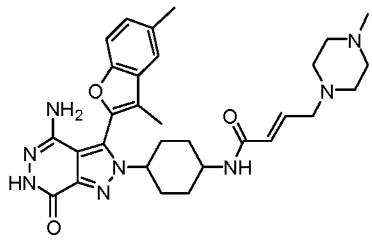 |
| A61 | (2Z)-N-{4-[4-Amino-3-(5-chloro-3-met hyl-1-benzofuran-2-yl)-7-carbonyl-6,7-dihydro-2H-pyrazolo[3,4-d]pyridazin-2-yl] cyclohexyl}-4-(dimethylamino)-2-fluorobut-2-enamide | 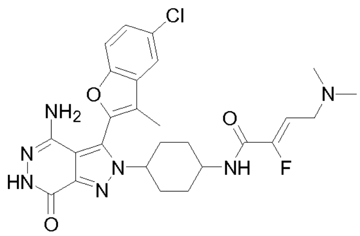 |
| A62 | 4-amino-3-(5-chloro-3-methyl-1-benzofuran-2-yl)-2-[1-(prop-2-ynyl)piperidin-4-yl]-2,6-dihydro-7H-pyrazolo[3,4-d]pyridazin-7-one | 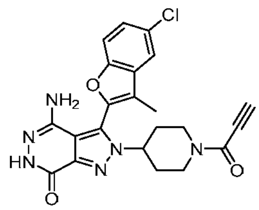 |
| A63 | 4-amino-3-(3,5-dimethyl-1-benzofuran-2-yl)-2-[1-(4-hydroxybut-2-ynyl)pyrrolidin-3-yl]-2,6-dihydro-7H-pyrazolo[3,4-d]pyridazin-7-one | 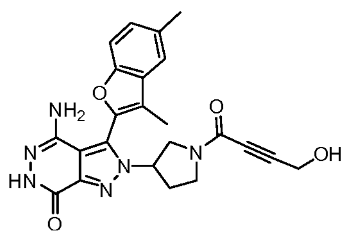 |

(continued)

| No. | Chemical Name | Structure |
|---|---|---|
| A64 | N-{4-[4-Amino-3-(5-chloro-3-methyl-1-benzofuran-2-yl)-7-carbonyl-6,7-dihydro-2H-pyrazolo[3,4-d]pyridazin-2-yl]cy clohexyl}but-2-ynylamide | 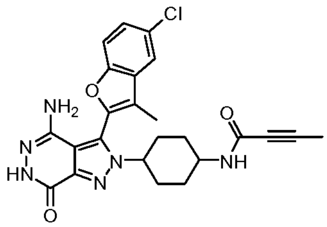 |
| A65 | 4-amino-3-(3,5-dimethyl-1-benzofuran-2-yl)-2-(1-{(2E)-4-[(3R)-3-fluoropyrrolidine-1-yl]but-2-enoyl}piperidin-4-yl)-2,6-dihydro-7H-pyrazolo[3,4-d]pyridaz in-7-one | 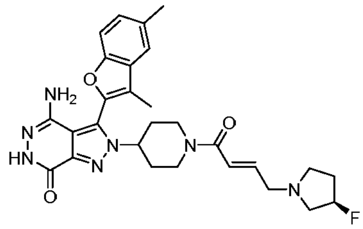 |
| A66 | 4-amino-3-(3,5-dimethyl-1-benzofuran-2-yl)-2-{1-[(2E)-4-(4-hydroxypiperidin-1-yl)-but-2-enoyl]piperidin-4-yl}-2,6-dihydro-7H-pyrazolo[3,4-d]pyridazin-7-one | 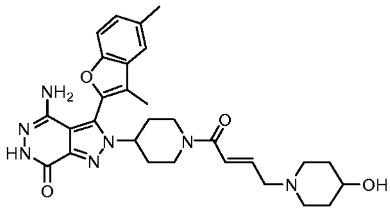 |
| A67 | 1-(1-acryloylpiperidin-4-yl)-4-amino-3-(3,5-dimethyl-1-benzofuran-2-yl)-1,6-dihydro-7H-pyrrolo[2,3-d]pyridazin-7-one | 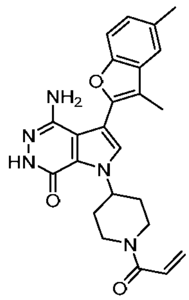 |
| A68 | 1-[(1-acryloylpiperidin-4-yl)methyl]-4-amino-3-(3,5-dimethyl-1-benzofuran-2-yl)-1,6-dihydrogen-7H-pyrrolo[2,3-d]pyridazin-7-one | 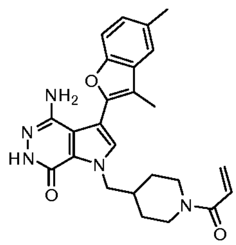 |
| A69 | 1-[(3S)-1-acryloylpyrrolidin-3-yl]-4-amino-3-(3,5-dimethyl-1-benzofuran-2-yl)-1,6-dihydrogen-7H-pyrrolo[2,3-d]pyridazin-7-one | 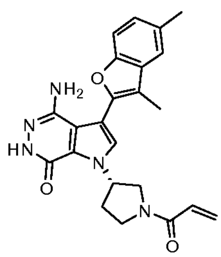 |

(continued)

| No. | Chemical Name | Structure |
|---|---|---|
| A70 | (2E)-N-{4-[4-Amino-3-(1-methyl-1H-benzimidazol-2-yl)-7-carbonyl-6,7-dihydro-2H-pyrazolo[3,4-d]pyridazin-2-yl] phenyl}-4-(dimethylamino)but-2-enamide | 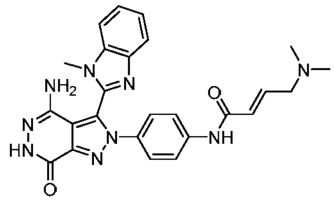 |
| A71 | (2E)-N-{4-[4-Amino-3-(1,3-dimethyl-1H-indol-2-yl)-7-carbonyl-6,7-dihydro-2H-pyrazolo[3,4-d]pyridazin-2-yl]pheny l}-4-(dimethylamino)but-2-enamide | 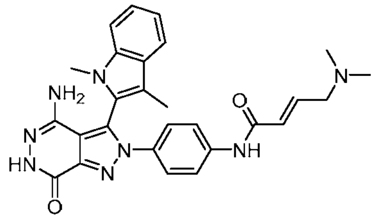 |
| A72 | (2E)-N-{4-[4-Amino-3-(1,3-benzothiaz ol-2-yl)-7-carbonyl-6,7-dihydro-2H-pyr azolo[3,4-d]pyridazin-2-yl]phenyl}-4-( dimethylamino)but-2-enamide | 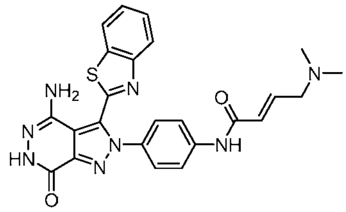 |
| A73 | (2E)-N-{4-[4-Amino-3-(3,5-dimethyl-1-benzofuran-2-yl)-7-carbonyl-6,7-dihydro-1H-pyrrolo[2,3-d]pyridazin-1-yl]cyclohexyl}-4-(azetidin-1-yl)but-2-enamide | 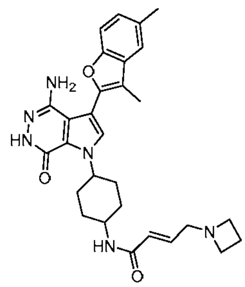 |
| A74 | 2-(1-acryloylpiperidin-4-yl)-4-amino-3-[(3,5-dimethoxyphenyl) ethynyl]-2,6-dihydro-7H-pyrazolo[3,4-d]pyridazin-7-o ne | 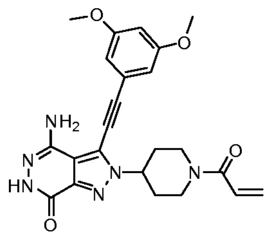 |
| A75 | 4-amino-2-[1-(cyclopropylcarbonyl)piperidin-4-yl]-3-(3,5-dimethyl-1-benzofuran-2-yl)-2,6-dihydrogen-7H-pyrazolo[ 3,4-d]pyridazin-7-one | 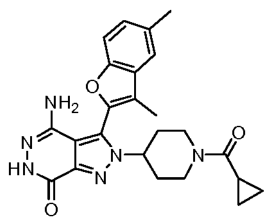 |

(continued)

| No. | Chemical Name | Structure |
|---|---|---|
| A76 | N-{4-[4-Amino-3-(3,5-dimethyl-1-benzofuran-2-yl)-7-carbonyl-6,7-dihydro-2H-pyrazolo[3,4-d]pyridazin-2-yl]cyclo hexyl}-2-cyanoacetamide | 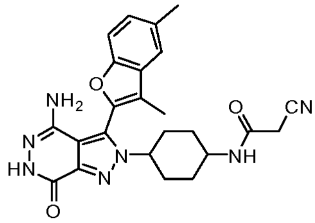 |
| A77 | 2-(1-acryloylpiperidin-4-yl)-4-amino-3-[(3,5-diethoxyphenyl) ethynyl]-2,6-dih ydro-7H-pyrazolo [3,4-d]pyridazin-7-one | 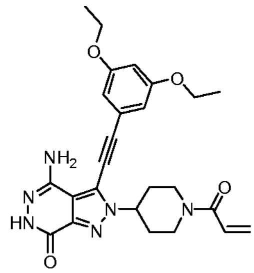 |
| A78 | N-{4-[4-Amino-3-[(3,5-dimethoxyphen yl)ethynyl]-7-carbonyl-6,7-dihydro-2H-pyrazolo[3,4-d]pyridazin-2-yl]phenyl} prop-2-enamide | 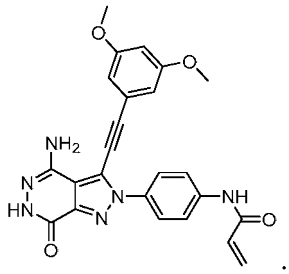 |
| A79 | 2-(1-acryloylpiperidin-3-yl)-4-amino-3-(5-methoxy-3-methylbenzofuran-2-yl)-2,6-dihydro-7H-pyrazolo[3,4-d]pyrida zin-7-one | 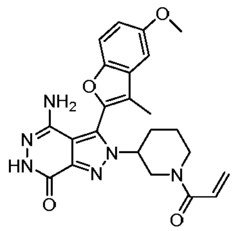 |
| A80 | 2-(1-acryloylpiperidin-4-yl)-4-amino-3-(5-fluoro-3-methylbenzofuran-2-yl)-2,6-dihydro-7H-pyrazolo [3,4-d]pyridazin -7-one | 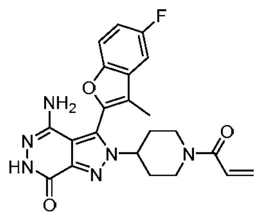 |
| A81 | 2-(1-propylpiperidin-4-yl)-4-amino-3-(3-methyl-5-(trifluoromethyl) benzofuran-2-yl)-2,6-dihydro-7H-pyrazolo[3,4-d] pyridazin-7-one | 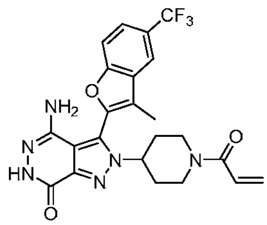 |

(continued)

| No. | Chemical Name | Structure |
|---|---|---|
| A82 | 2-(1-acryloylpiperidin-4-yl)-4-amino-3-(3,6-dimethylbenzofuran-2-yl)-2,6-dihydro-7H-pyrazole[3,4-d]pyridazin-7-on e | 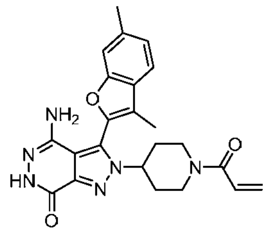 |
| A83 | 2-(1-acryloylpiperidin-4-yl)-4-amino-3-(6-fluoro-3-methylbenzofuran-2-yl)-2, 6-dihydro-7H-pyrazolo[3,4-d]pyridazin -7-one | 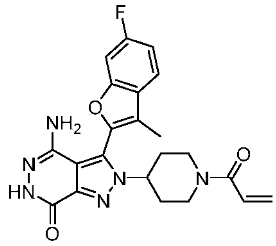 |
| A84 | 2-(1-acryloylpiperidin-4-yl)-4-amino-3-(6-chloro-3-methylbenzofuran-2-yl)-2,6-dihydro-7H-pyrazolo[3,4-d]pyridazin-7-one | 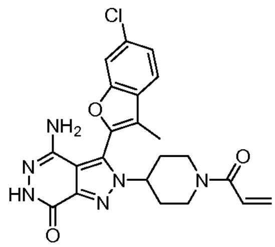 |
| A85 | 2-(1-acryloylpiperidin-4-yl)-4-amino-3-(3-ethyl-5-methylbenzofuran-2-yl)-2,6-dihydro-7H-pyrazolo[3,4-d]pyridazin-7-one | 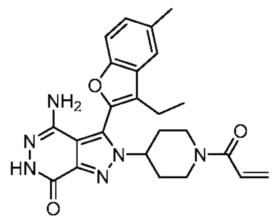 |
| A86 | 2-(1-acryloylpiperidin-4-yl)-4-amino-3-(5-chloro-3-methylbenzofuran-2-yl)-2,6-dihydro-7H-pyrazolo[3,4-d]pyridazin-7-one | 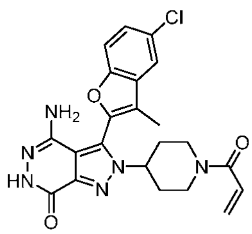 |
| A87 | 2-(1-acryloylpiperidin-3-yl)-4-amino-3-(5-chloro-3-methylbenzofuran-2-yl)-2,6-dihydro-7H-pyrazolo[3,4-d]pyridazin e-7-one | 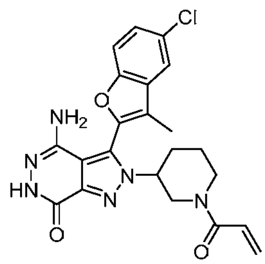 |

(continued)

| No. | Chemical Name | Structure |
|---|---|---|
| A88 | N-(4-(4-amino-3-(3,5-dimethylbenzofuran-2-yl)-7-carbonyl-6,7-dihydro-2H-pyrazolo[3,4-d]pyridazin-2-yl)cyclohexy l) acryloylamide | 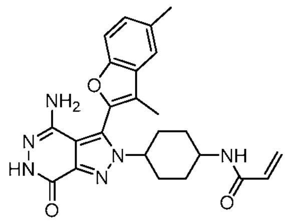 |
| A89 | (E)-2-(1-but-2-enoylpiperidin-3-yl)-4-amino-3-(5-methyl-3-methylbenzofuran-2-yl)-2,6-dihydro-7H-pyrazolo[3,4-d]pyridazin-7-one | 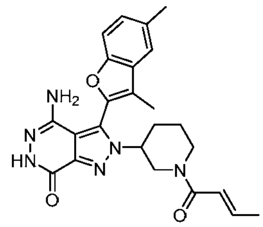 |
| A90 | (E)-2-(1-pent-2-enoylpiperidin-3-yl)-4-amino-3-(5-methyl-3-methylbenzofuran-2-yl)-2,6-dihydro-7H-pyrazolo[3,4-d]pyridazin-7-one | 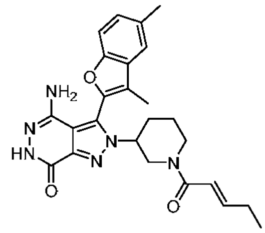 |
| A91 | 4-amino-3-(5-methyl-3-methyl-1-benzofuran-2-yl)-2-{1-[(2E)-4-(N,N-dimethyl)butyl-2-enoyl]piperidin-3-yl}-2,6-dih ydro-7H-pyrazolo[3,4-d]pyridazin-7-one | 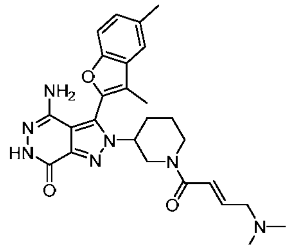 |
| A92 | 4-amino-3-(5-methyl-3-methyl-1-benzo furan-2-yl)-2-{1-[(2E)-4-(pyrrolidin-1-yl)butyl-2-enoyl]piperidin-3-yl}-2,6-di hydro-7H-pyrazolo[3,4-d]pyridazin-7-one | 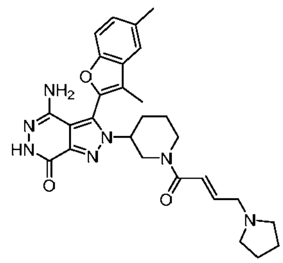 |

**6.** A preparation method for formula I compound of claim 1, wherein the method comprises the following steps:

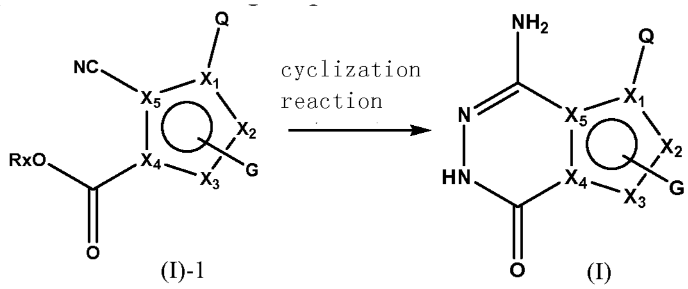


(I)-1 (I)

wherein $X_1$, $X_2$, $X_3$, $X_4$, $X_5$, Q and G have the same definitions as the corresponding claims; Rx is selected from C1-C6 alkyl;
the compound of formula (I)-1 is cyclized with hydrazine hydrate to give a compound of formula (I).

**7.** Use of compound I of claim 1 or a pharmaceutically acceptable salt thereof for

(a) preparation of a medicine for treating diseases associated with FGFR kinase activity or expression amount;
(b) preparation of FGFR kinase targeting inhibitor;
(c) in vitro non-therapeutic inhibition of FGFR kinase activity;
(d) in vitro non-therapeutic inhibition of tumor cell proliferation; and / or
(e) treatment of disease associated with FGFR kinase activity or expression amount.

**8.** The use of claim 7, wherein the disease associated with FGFR activity or expression amount is selected from the group consisting of carcinoma, hematopoietic malignant disease, other neoplasms, bone and chondrocyte disorders, hypophosphatemia, fibrotic diseases, psoriasis, keloid, bullous skin disease, atherosclerosis, restenosis, glomerular membrane cell proliferative disorder, glomerular lesion, diabetic nephropathy, nephropathy and benign prostatic hyperplasia, eye disease, and craniosynostosis; preferably is a carcinoma selected from the group consisting of: bladder cancer, breast cancer, cervical cancer, colorectal cancer, endometrial cancer, stomach cancer, head and neck cancer, kidney cancer, liver cancer, lung cancer, ovarian cancer, prostate cancer, esophageal cancer, gallbladder cancer, pancreatic cancer, thyroid cancer, skin cancer, leukemia, multiple myeloma, chronic lymphocytic lymphoma, adult T-cell leukemia, B-cell lymphoma, acute myeloid leukemia, Hodgkin's lymphoma or non-Hodgkin's lymphoma, Waldenstrom's macroglobulinemia, hairy cell lymphoma, Bojit's lymphoma, glioblastoma, melanoma, and rhabdomyosarcoma.

**9.** A pharmaceutical composition, wherein the pharmaceutical composition comprises: (i) effective amount of formula I compound, or a pharmaceutically acceptable salt thereof, and (ii) a pharmaceutically acceptable carrier.

**10.** A method for inhibiting FGFR kinase activity, wherein the method comprises steps: administering an inhibitory effective amount of formula I compound of claim 1 or a pharmaceutically acceptable salt thereof to a subject in need thereof, or administering an inhibitory effective amount of pharmaceutical composition of claim 9 to a subject in need thereof.

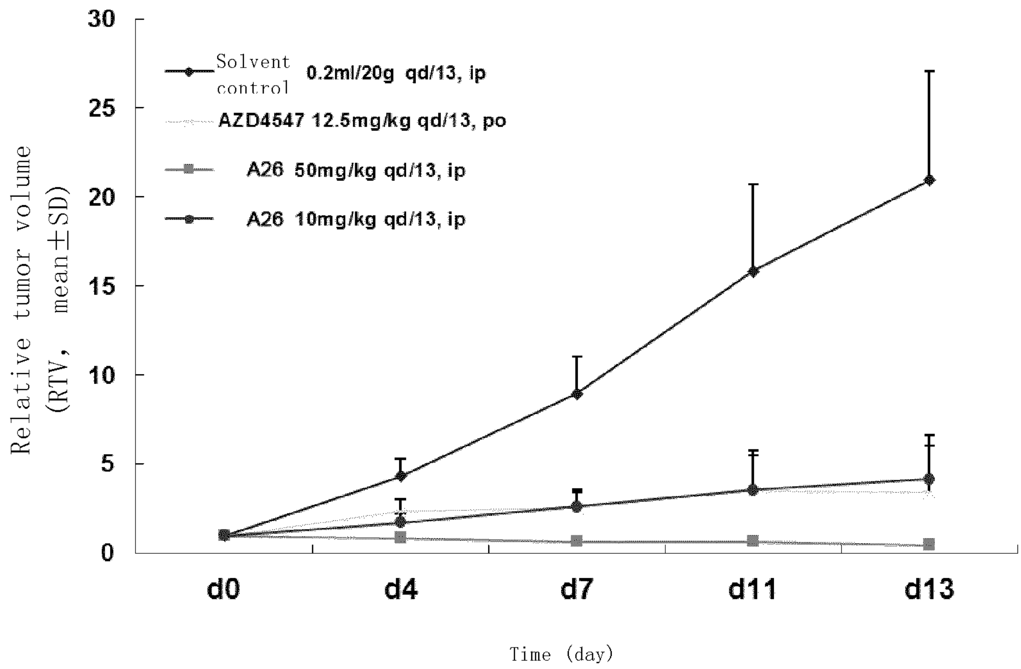
Relative tumor volume (RTV, mean±SD)
Solvent control 0.2ml/20g qd/13, ip
AZD4547 12.5mg/kg qd/13, po
A26 50mg/kg qd/13, ip
A26 10mg/kg qd/13, ip
30
25
20
15
10
5
0
d0
d4
d7
d11
d13
Time (day)

Fig. 1

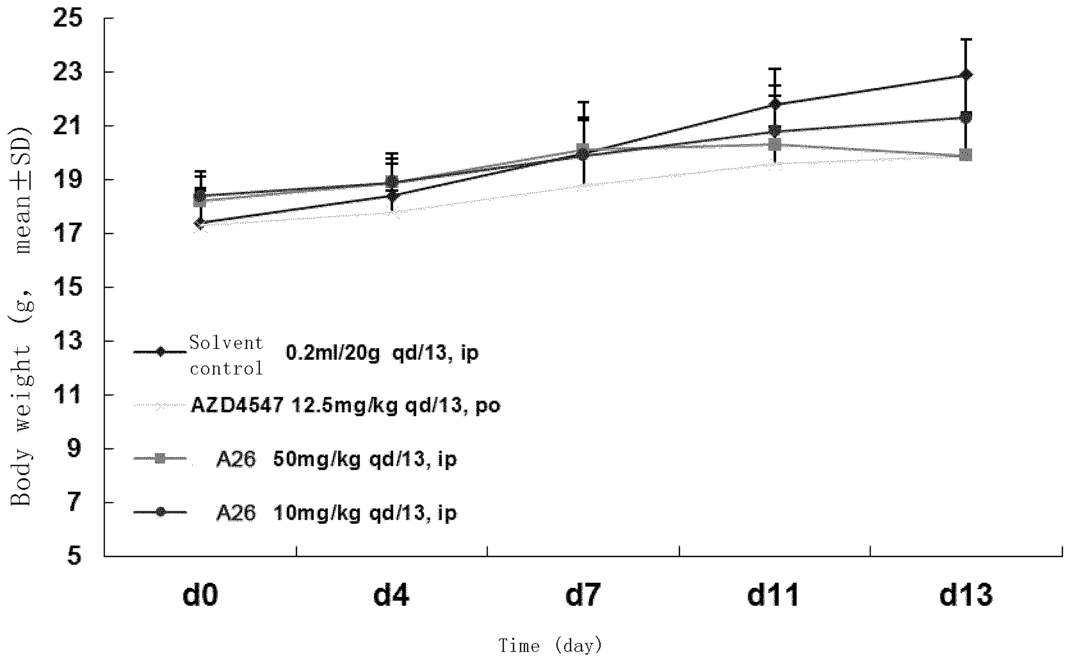
Body weight (g, mean±SD)
Solvent control 0.2ml/20g qd/13, ip
AZD4547 12.5mg/kg qd/13, po
A26 50mg/kg qd/13, ip
A26 10mg/kg qd/13, ip
25
23
21
19
17
15
13
11
9
7
5
d0
d4
d7
d11
d13
Time (day)

Fig. 2

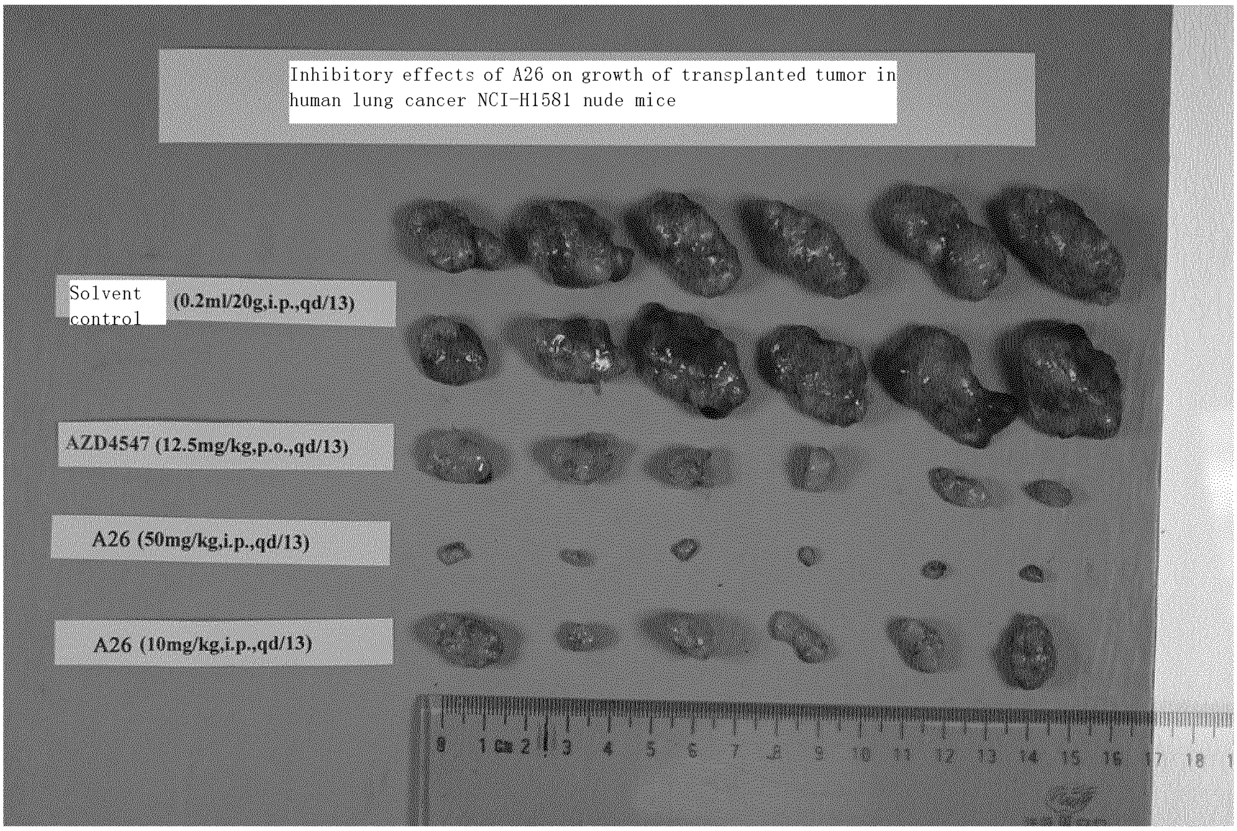
Inhibitory effects of A26 on growth of transplanted tumor in human lung cancer NCI-H1581 nude mice
Solvent control (0.2ml/20g,i.p.,qd/13)
AZD4547 (12.5mg/kg,p.o.,qd/13)
A26 (50mg/kg,i.p.,qd/13)
A26 (10mg/kg,i.p.,qd/13)

Fig. 3

# INTERNATIONAL SEARCH REPORT

International application No.
**PCT/CN2017/085765**

**A. CLASSIFICATION OF SUBJECT MATTER**

C07D 487/04 (2006.01) i; A61K 31/5025 (2006.01) i; A61K 31/5377 (2006.01) i; A61K 31/541 (2006.01) i
According to International Patent Classification (IPC) or to both national classification and IPC

**B. FIELDS SEARCHED**

Minimum documentation searched (classification system followed by classification symbols)

C07D 487/04; C07D 487/+; A61K 31/5025; A61K 31/5377; A61K 31/541

Documentation searched other than minimum documentation to the extent that such documents are included in the fields searched

Electronic data base consulted during the international search (name of data base and, where practicable, search terms used)
Data Bases: DWPI, SIPOABS, CNABS, CNKI, REGISTRY, CAPLUS
Search Terms: pyridazinone, pyrazole, kinase, pyridazin, one, FGFR, Pyrazolo, structure search carried out according to the structure of formula (I)

**C. DOCUMENTS CONSIDERED TO BE RELEVANT**

| Category* | Citation of document, with indication, where appropriate, of the relevant passages | Relevant to claim No. |
|---|---|---|
| PX | WO 2016112637 A1 (HUBEI BIO PHARMACEUTICAL IND TECHNOLOGICAL INST INC et al.), 21 July 2016 (21.07.2016), see the whole document, particularly description, pages 118 and 207 | 1-4, 6, 9 |
| PX | YUE, Y., et al., “Facile Synthesis of Cyanofurans via Michaeladdition/Cyclization of Ene-Yne-Ketones with Trimethylsilyl Cyanide”, CHEMICAL COMMUNICATIONS, vol. 53, no. 3, 09 December 2016 (09.12.2016), pages 640-643 | 1-3, 6 |
| A | WO 2016007185 A1 (ETERNITY BIOSCIENCE INC.), 14 January 2016 (14.01.2016), see the whole document, particularly claim 1 | 1-10 |

☐ Further documents are listed in the continuation of Box C. ☒ See patent family annex.

* Special categories of cited documents:
“A” document defining the general state of the art which is not considered to be of particular relevance
“E” earlier application or patent but published on or after the international filing date
“L” document which may throw doubts on priority claim(s) or which is cited to establish the publication date of another citation or other special reason (as specified)
“O” document referring to an oral disclosure, use, exhibition or other means
“P” document published prior to the international filing date but later than the priority date claimed
“T” later document published after the international filing date or priority date and not in conflict with the application but cited to understand the principle or theory underlying the invention
“X” document of particular relevance; the claimed invention cannot be considered novel or cannot be considered to involve an inventive step when the document is taken alone
“Y” document of particular relevance; the claimed invention cannot be considered to involve an inventive step when the document is combined with one or more other such documents, such combination being obvious to a person skilled in the art
“&” document member of the same patent family

| Date of the actual completion of the international search | Date of mailing of the international search report |
|---|---|
| 23 August 2017 (23.08.2017) | **01 September 2017 (01.09.2017)** |

| Name and mailing address of the ISA/CN: | Authorized officer |
|---|---|
| State Intellectual Property Office of the P. R. China<br>No. 6, Xitucheng Road, Jimenqiao<br>Haidian District, Beijing 100088, China<br>Facsimile No.: (86-10) 62019451 | **FU, Wei**<br>Telephone No.: (86-10) **62086335** |

Form PCT/ISA/210 (second sheet) (July 2009)

## INTERNATIONAL SEARCH REPORT

International application No.
**PCT/CN2017/085765**

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 7-8 and 10
because they relate to subject matter not required to be searched by this Authority, namely:
[1] although claims 7 and 8 relate to the technical solution of (e), and claim 10 relates to a method of treatment of a human or animal body by therapy (PCT Rule 39.1(iv)), a search is still carried out based on the claimed effects of the compound or composition.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet (2)) (July 2009)

**INTERNATIONAL SEARCH REPORT**
Information on patent family members

International application No.
**PCT/CN2017/085765**

| Patent Documents referred in the Report | Publication Date | Patent Family | Publication Date |
|---|---|---|---|
| WO 2016112637 A1 | 21 July 2016 | CN 105837576 A | 10 August 2016 |
| WO 2016007185 A1 | 14 January 2016 | US 2017152264 A1 | 01 June 2017 |
| | | TW 201625626 A | 16 July 2016 |
| | | EP 3166608 A1 | 17 May 2017 |
| | | KR 20170023156 A | 02 March 2017 |
| | | CA 2953798 A1 | 14 January 2016 |
| | | AU 2014400628 A1 | 19 January 2017 |
| | | CN 106573001 A | 19 April 2017 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *Nature Reviews Clinical Oncology,* 2015
- **CYTOKINE ; GROWTH.** *Factor Reviews,* 2005, vol. 16, 233-247
- *Cancer Discovery,* 2013, vol. 3 (3), 264-279
- *Annals of Oncology,* 2014, vol. 25, 552-563